# EUROPEAN PATENT APPLICATION

(11) **EP 2 527 451 A1**
(43) Date of publication of application: **28.11.2012**
(21) Application number: 12176682.8
(22) Date of filing: 10.12.2009
(51) Int. Cl.: C12N 15/82, A01H 5/00

(54) **Screening method for identifying genes involved in plant cell cycle**

(30) Priority: 10.12.2008 EP 08171187
(62) Divisional of application: 09765131.9
(71) Applicant: VIB VZM, 9052 Gent (BE); Universiteit Gent, 9000 Gent (BE); Universiteit Antwerpen, 2000 Antwerpen (BE)
(72) Inventor: de Jaeger, Geert, 9940 Evergem (BE); Gonzalez, Nathalie, 9820 Merelbeke (BE); Inzé, Dirk Gustaaf, 9310 Moorsel - Aalst (BE); van Leene, Jelle, 9000 Gent (BE); van Onckelen, Henri, 2100 Antwerpen (BE); Witters, Erwin, 2610 Wilrijk (BE)

(57) **Abstract**

The present invention relates to a novel method for screening proteins related to and/or involved in plant cell cycle. It further relates to proteins isolated with the method, and the use of those proteins, and/or the genes encoding those proteins for modulating plant yield and plant growth.

## Description

The present invention relates to a novel method for screening proteins related to and/or involved in plant cell cycle. It further relates to proteins isolated with the method, and the use of those proteins, and/or the genes encoding those proteins for modulating plant yield and plant growth.

Knowledge of the basic cell cycle machinery is a prerequisite to grasp how signaling pathways impinge on it and regulate cell proliferation during plant growth and development in a changing environment. The fundamental underlying mechanisms of cell division are conserved among all eukaryotes, however, due to their sessile lifestyle, plants have evolved unique features. Plant genome sequence analysis revealed the existence of an unexpected high number of genes involved in cell proliferation (Capron et al., 2003; Vandepoele et al., 2002; Menges et al., 2005; Schultz et al., 2007), compared to other organisms. Microarray analysis showed that a lot of these follow a cell cycle-dependent expression profile (Menges et al., 2005), sustaining their role in cell cycle regulation. To elucidate which molecular machines are involved in plant cell division, we isolated cellular complexes by TAP from *Arabidopsis thaliana* cell suspension cultures (Van Leene et al., 2007; Van Leene et al., 2008) using 'core' cell cycle proteins as baits. The dataset was corrected for non-specific interactions and divided in a 'core' dataset of interactions that were biologically confirmed in at least two independent repeats, and a 'non-core' dataset. Surprisingly we found that the datasets, apart from known cell cycle proteins, were also comprising proteins of which the role in cell cycle never has been illustrated before. The robustness of both the core and non-core datasets is demonstrated through different computational analysis and through the biological interpretation of the network. The interactome serves as an excellent hypothesis-generating tool, and the power of it is reached in particular when integrated with other data. Combining our interactome data with cell cycle related expression profiles for example gives insight in which CDK/cyclin complexes are active during cell division and when. The data proof that the high numbers of cell cycle regulators in plant are not the sole consequence of redundancy, but that different cell cycle regulators are combined in complexes providing functional diversity leading to increased complexity and flexibility of the plant cell cycle.

A first aspect of the invention is a method to isolate novel cell-cycle related proteins, comprising (1) performing a tap analysis using a known cell cycle protein as bait (2) correcting the results for non-specific interactions and (3) reconfirming the corrected results. Reconfirming the result, as used here, can be done either by repeating the tap-tag experiment, or by carrying out a reversed tap-tag, wherein the original prey is now used as bait. Another aspect of the invention is the use of a cell-cycle related protein isolated with the method of the invention, for the modulation of plant growth and/or yield. In a preferred embodiment, said plant is a crop plant, preferably a monocot or a cereal, even more preferably it is a cereal selected from the group consisting of rice, maize, wheat, barley, millet, rye, sorghum and oats. The use, as indicated here, is the use of the protein, and/or the use of a nucleic acid encoding this protein, or the complement thereof. It is including, but not limited to genomic DNA, cDNA, messenger RNA (including the 5' and 3' untranslated regions) and RNAi; said use can result, as a non-limiting example, in overexpression, or repression of the expression of the gene. Overexpression or repression of expression of a target gene can be obtained by transfer of a genetic construct, intended for said overexpression or said repression of expression into a plant. The transfer of foreign genes into the genome of a plant is called transformation. Transformation of plant species is a fairly routine technique known to the person skilled in the art. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include, but are not limited to agrobacterium mediated transformation, the use of liposomes, electroporation, chemicals that increase free DNA uptake, injection of the DNA directly into the plant, particle gun bombardment, transformation using viruses or pollen and microprojection.

Preferably, said cell cycle related proteins is selected from the list consisting of At1g56110, At3g17020, At3g21140, At5g25460, At5g60790, At4g38900, At3g49240, At5g24690, At1g06070, At4g34150, At1g20480, At5g20920, At3g15970, At5g13030, At1g01880, At5g07310, At2g46610, At1g10690, At3g04710, At3g24690, At4g16130, At2g05830, At1g29220, At1g55890, At1g60650, At1g70830, At2g43140, At1g77180, At5g18620, At5g02530, At5g14170, At1g52730, At2g33340, At1g03060, At3g62240, At4g38740, At5g61220, At3g53880, At3g56860, At1g01970, At1g19520, At1g14620, At2g03820, At3g01280, At3g56690, At5g41190, At5g03740, At1g42440, At2g28450, At1g09760, At1g10840, At3g11830, At5g54900, At1g31760, At1g61870, At3g11760, At1g05805, At1g29200, At4g13850, At4g38780, At1g71380, At3g13640, At5g25060, At1g43700, At2g46020, At3g55760 and At5g21160 or a variant thereof. Variants, as used here, are including, but not limited to homologues, orthologues and paralogues of said cell cycle related proteins. "Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.. Preferably, said homologue, orthologue or paralogue has a sequence identity at protein level of at least 50%, 51%, 52%, 53%, 54% or 55%, 56%, 57%, 58%, 59%, preferably 60%, 61%, 62%, 63%, 64%, 65%, 66%, 67%, 68%, 69%, more preferably 70%, 71%, 72%, 73%, 74%, 75%, 76%, 77%, 78%, 79%, even more preferably 80%, 81%, 82%, 83%, 84%, 85%, 86%, 87%, 88%, 89% most preferably 90%, 91%, 92%, 93%, 94%, 95%, 96%, 97%, 98%, 99% or more as measured in a BLASTp (Altschul et al., 1997; Altschul et al., 2005).

Preferably said use is overexpression of the gene encoding the cell-cycle related protein, even more preferably it is overexpression of a cell-cycle protein according to the invention, selected from the group consisting of At1g56110, At3g17020, At3g21140, At5g25460, At5g60790 (SEQ ID N° 1-5), or a variant thereof. Preferably, said overexpression results in an increase of plant growth and/or yield. Increase of plant growth and/or yield is measured by comparing the test plant, comprising a gene used according to the invention, with the parental, non-transformed plant, grown under the same conditions as control. Preferably, increase of growth is measured as an increase of biomass production. "Yield" refers to a situation where only a part of the plant, preferably an economical important part of the plant, such as the leaves, roots or seeds, is increased in biomass. The term "increase" as used here means least a 5%, 6%, 7%, 8%, 9% or 10%, preferably at least 15% or 20%, more preferably 25%, 30%, 35% or 40% more yield and/or growth in comparison to control plants as defined herein. Increase of plant growth, as used here, is preferably measured as increase of any one or more of total plant biomass, leaf biomass, root biomass and seed biomass. In one preferred embodiment, said increase is an increase in total plant biomass. In a preferred embodiment, said plant is a crop plant, preferably a monocot or a cereal, even more preferably it is a cereal selected from the group consisting of rice, maize, wheat, barley, millet, rye, sorghum and oats.

Still another aspect of the invention is a novel cell cycle related protein, isolated with the method according to the invention.

### Brief description of the figures

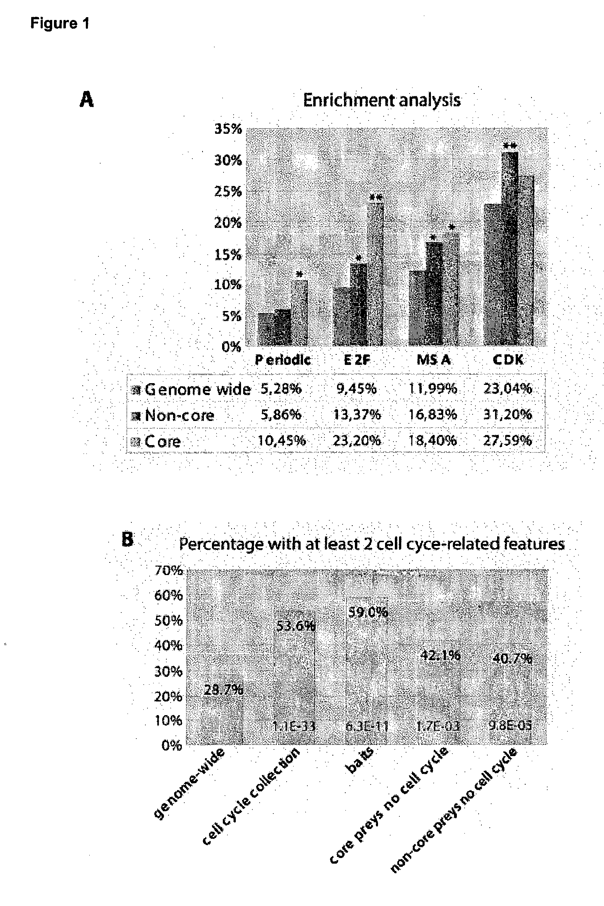

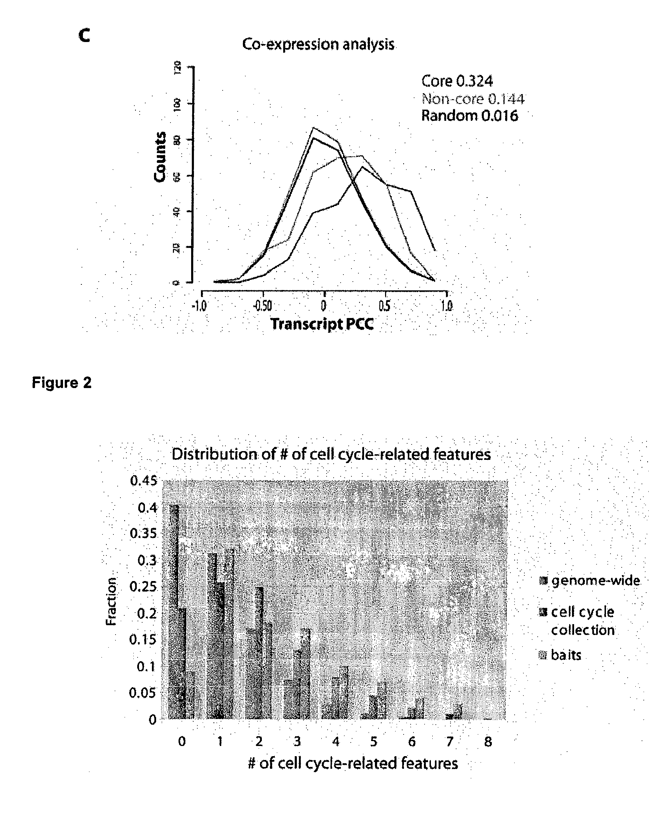

**Figure 1****:** (**A**) Enrichment analysis on the core and non-core datasets. Statistically significant enrichments are indicated with * (p-value < 0.05) or with ** (p-value < 0.01). An enrichment analysis was performed for genes showing cell cycle-regulated expression (Periodic), for genes containing an E2F or MSA consensus motif in their promoter and for proteins containing a CDK consensus phosphorylation site. (**B**) Percentage of the whole gene pool (genome-wide), a cell cycle collection of 518 genes, bait proteins (without reverse baits), and core and non-core preys (without the cell cycle collection) having at least two features. P-values are shown in red. (**C**) Distribution of the transcript PCC of the core (red curve) and non-core dataset (green curve), compared to the average distribution (black and grey curves) of 100 random networks containing an equal number of interactions and genes.
**Figure 2****:** Distribution of the number of cell cycle-related features among the whole gene pool (genome-wide), a collection of 518 cell cycle genes, and bait proteins (without reverse baits).
**Figure 3****:** Overview of the subnetworks.
**Figure 4****:** Fresh weight of control lines transformed with empty vector. About 100 plants were analyzed for each independent line (C1 and C2).
**Figure 5****:** Distribution of plants in function of their fresh weight. The graphs represent the frequency of plant belonging to a certain category of weight.
**Figure 6****:** Distribution of plants in function of their fresh weight. The graphs represent the frequency of plant belonging to a certain category of weight (% of plants versus fresh weight category, as in figure 5). The lines analyzed are independent lines of the ones presented in figure 5.

### Examples

### Materials and methods to the examples

### Cloning and TAP purification

Cloning of transgenes encoding tag fusions under control of the constitutive Cauliflower tobacco mosaic virus 35S promoter, transformation of *Arabidopsis* cell suspension cultures, protein extract preparation, TAP purification, protein precipitation and separation were done as previously described (Van Leene et al., 2007). The adapted protocol used for purification of protein complexes incorporating GS-tagged bait is described elsewhere (Van Leen et al., 2008). For identification by mass spectrometry, minor adjustments were implemented compared to previous described protocols (Van Leene et al., 2007), as described below.

### Proteolysis and peptide isolation

After destaining, gel slabs were washed for 1 hour in H2O, polypeptide disulfide bridges were reduced for 40 min in 25 mL of 6,66 mM DTT in 50 mM NH₄HCO₃ and sequentially the thiol groups were alkylated for 30 min in 25 mL 55 mM IAM in 50 mM NH₄HCO₃. After washing the gel slabs 3 times with water, complete lanes from the protein gels were cut into slices, collected in microtiter plates and treated essentially as described before with minor modifications (Van Leene et al., 2007). Per microtiter plate well, dehydrated gel particles were rehydrated in 20 µL digest buffer containing 250 ng trypsin (MS Gold; Promega, Madison, WI), 50 mM NH4HCO3 and 10% CH3CN (v/v) for 30 min at 4° C. After adding 10 µL of a buffer containing 50 mM NH4HCO3 and 10% CH3CN (v/v), proteins were digested at 37° C for 3 hours. The resulting peptides were concentrated and desalted with microcolumn solid phase tips (PerfectPureTM C18 tip, 200 nL bed volume; Eppendorf, Hamburg, Germany) and eluted directly onto a MALDI target plate (Opti-TOFTM384 Well Insert; Applied Biosystems, Foster City, CA) using 1.2 µL of 50% CH3CN: 0.1 % CF3COOH solution saturated with α-cyano-4-hydroxycinnamic acid and spiked with 20 fmole/µL Glu1 Fibrinopeptide B (Sigma Aldrich), 20 fmole/µL des-Pro2-Bradykinin (Sigma Aldrich), and 20 fmole/µL Adrenocorticotropic Hormone Fragment 18-39 human (Sigma Aldrich).

### Acquisition of mass spectra

A MALDI tandem MS instrument (4700 and 4800 Proteomics Analyzer; Applied Biosystems) was used to acquire peptide mass fingerprints and subsequent 1 kV CID fragmentation spectra of selected peptides. Peptide mass spectra and peptide sequence spectra were obtained using the settings essentially as previously presented (Van Leene et al., 2007). Each MALDI plate was calibrated according to the manufacturers' specifications. All peptide mass fingerprinting (PMF) spectra were internally calibrated with three internal standards at m/z 963.516 (des-Pro2-Bradykinin), m/z 1570.677 (Glul-Fibrinopeptide B), and m/z 2465,198 (Adrenocorticotropic Hormone Fragment 18-39) resulting in an average mass accuracy of 5 ppm ± 10 ppm for each analyzed peptide spot on the analyzed MALDI targets. Using the individual PMF spectra, up to sixteen peptides, exceeding a signal-to-noise ratio of 20 that passed through a mass exclusion filter were submitted to fragmentation analysis.

### MS based protein homology identification

PMF spectra and the peptide sequence spectra of each sample were processed using the accompanied software suite (GPS Explorer 3.6, Applied Biosystems) with parameter settings essentially as previously described (Van Leene et al, 2007). Data search files were generated and submitted for protein homology identification by using a local database search engine (Mascot 2.1, Matrix Science). An in house non-redundant *Arabidopsis* protein database called SNAPS *Arabidopsis thaliana* version 0.4 (SNAPS = Simple Non-redundant Assembly of Protein Sequences, 77488 sequence entries, 30468560 residues; available at http://www.ptools.ua.ac.be/snaps) was compiled from nine public databases. Protein homology identifications of the top hit (first rank) with a relative score exceeding 95% probability were retained. Additional positive identifications (second rank and more) were retained when the score exceeded the 98% probability threshold. Because identifications were done with different versions of the SNAPS -database (Van Leen et al., 2007), and with the goal to obtain more uniformity between the identifications, all identifications from the core and the non-core dataset were resubmitted to Mascot and identified with the protein sequence repertoire from the latest TAIR database (TAIR8.0). Furthermore, an additional restriction was implemented to reduce the number of false positive identifications, and as so, identifications, for which more than 50 % of the corresponding peptides had a trypsin miss-cleavage, were discarded.

### GO enrichment analysis

Analysis of over- and underrepresentation of GO terms was done with the BiNGO tool (Maere et al., 2005) in Cytoscape (Shannon et al., 2003). The hypergeometric test was chosen at a significance value of 0.05 with the Benjamini and Hochberg False Discovery Rate Correction for multiple testing. The *Arabidopsis* gene annotation file used in the analysis was downloaded from the gene ontology website on the 4th of October 2008.

### Cell cycle enrichment analysis

For the periodic gene enrichment analysis, a list of 1258 genes showing cell cycle-regulated and cell cycle-associated expression was compiled from two datasets (Menges et al., 2003; Jensen et al., 2006). Genome wide corresponds to all 23834 genes present on the Affymtetrix ATH1 microarray. Genes containing E2F or M-specific activator (MSA) motifs in their promoter sequence were *in silico* determined by combining transcript expression data and comparative genomics (Vandepoele et al, 2006). Here, genome wide corresponds to 19173 genes for which a unique probe set is available on the ATH1 microarray. Proteins containing the CDK consensus phosphorylation site [ST]PX[KR], a known hallmark of CDK substrates (De Veylder et al., 1997), were considered as potential CDK substrates. The presence of the consensus motif was screened with the patmatch tool available at TAIR, and hence, genome wide corresponds here to all 27235 proteins present in the TAIR8.0 release. For all enrichment analysis, p-values were calculated with the hypergeometric cumulative distribution function of the Matlab 7.5 software. Proteins that could not be assigned to a specific gene locus were discarded from all enrichment analysis.

### Overlap with protein-protein databases

To assess the novelty of the cell cycle interactome, we screened for the overlap of our datasets with the following databases containing protein-protein interactions: TAIR (Huala et al., 2001), InTact (Kerrien et al., 2007), *Arabidopsis* Reactome (Tsesmetzis et al., 2008), AtPID (Cui et al., 2008), Reactome (Vastrik et al., 2007) and The Bio-Array Resource (BAR) for *Arabidopsis* Functional Genomics (Geisler-Lee et al., 2007).

### Co-expression analysis

Transcript Pearson Correlation Coefficients (PCC), representing the degree of co-expression of gene pairs, were calculated based on an *Arabidopsis* ATH1 micro-array compendium of 518 experiments focused towards cell cycle or plant growth and development (table 7). We compared the PCC distribution of both datasets with the PCC distribution of 100 randomized datasets with an equal number of randomly chosen proteins and interactions.

### Constructs and plant transformation

The overexpressing constructs were produced by using gateway cloning technology. The cDNA of the genes of interest (table, sheet OE produced and LOF requested) were amplified by PCR from reverse transcribed RNA extracted from tissues of *Arabidopsis thaliana* ecotype Columbia. The PCR reactions were performed using the Phusion High fidelity DNA polymerase (Finnzymes) according to the manufacturer's instructions. The PCR fragments, corresponding to complete cDNA of the genes of interest were introduced into pDONr 201 using the Gateway system (Invitrogen) by attBXattP recombination sites and subsequently recombined into the pK7WG2 expression vector by *att*L X*att*R sites recombination. The sequence was confirmed by sequencing. The constructs containing the genes of interest under the control of the CaMV 35S promoter were used to transform *Arabidopsis thaliana* by the flowerdip method (Clough and Bent, 1998).

### Plant material and growth condition

Transgenic lines were identified by selection on MS medium (half-strength Murashige and Skoog medium (Duchefa, Haarlem, The Netherlands), Sucrose 1%) supplemented with 50 mg/l kanamycin and later transferred to soil for seed production. A second selection on MS plus kanamycine allowed the selection of lines containing one site of insertion of the transgene. Plants were grown under a 16-h day and 8-h night regime at 21 °C.

### Biomass test

For the biomass measurement, the vegetative part of a 20 days old plant grown on MS medium was harvested and the fresh weight was measured by weighing about 60 plants of each line.

### Example 1: isolation of the TAP complexes

As baits we used 73 'core' cell cycle regulatory proteins (Vandepoele et al., 2002; Menges et al., 2006; Perez et al., 2007), 4 mitotic checkpoint proteins (Menges et al., 2005), 8 anaphase promoting complex (APC) subunits and 6 APC activators (Capron et al., 2003), one 26S proteasome subunit (Brukhin et al., 2005), 10 proteins involved in DNA replication or repair (Schultz et al., 2007), and as proof of concept, 6 proteins for reverse TAP experiments (table 1). Of the 108 TAP fusions, 102 were expressed successfully. In total, 303 purifications were performed with at least two independent purifications per bait.

### Example 2: identification of the prey proteins

Purified proteins were identified via MALDI-TOFTOF. Non-specific proteins, determined by control purifications, were subtracted from the hit lists (table 2), generating a non-redundant dataset of 857 interactions among 393 proteins. This dataset was divided in a 'core' dataset of 371 interactions among 196 proteins, containing interactions that were biologically confirmed in at least 2 independent repeats or in the reciprocal experiment, and a 'non-core' dataset with the remainder 486 interactions among 320 proteins.

### Example 3: enrichment analysis

To assess the quality of the interactome, we performed different enrichment analysis on the core and non-core preys. In both datasets, the GO term 'cell cycle' was highly enriched (table 3). Additional GO enrichments demonstrate that cell cycle is linked to a myriad of biological processes including growth and development, response to stress and hormone stimuli, energy production, chromatin remodeling and others. Next, we observed an enrichment in the core dataset for genes periodically expressed during cell cycle (Fig. 1A). Furthermore, both datasets were enriched for genes with E2F or M-specific activator (MSA) motifs in their promoter. The less pronounced enrichments for the non-core dataset indicate that it is more biased for interactions linking the core cell cycle machinery with other pathways. This is supported by the fact that the non-core dataset is more enriched for potential CDK substrates, as assessed by the presence of CDK phosphorylation sites. The fact that these interactions were often not confirmed is likely due to the transient nature of e.g. CDK/substrate interactions.

### Example 4: isolation of novel cell cycle genes

In a quest for new cell cycle-related proteins, we integrated different cell cycle related features (table 4). The distribution of the number of features per gene shows that a collection of known cell cycle genes (table 5) is enriched for these features compared to the whole gene pool (Fig. 2). A clear shift between the whole gene pool and the cell cycle collection was visible at 2 features, as it was the case for the original bait list, validating the choice of our baits. In search for new cell cycle proteins, we started from the core and noncore prey list and subtracted the collection of known cell cycle genes. The percentage meeting the criterion of having at least 2 features is shown (Fig. 1 B), and all classes are significantly enriched compared to the whole gene pool. By filtering for genes containing at least 2 features, we generated a list of 123 potential new cell cycle genes from the core and non-core dataset (table 6).

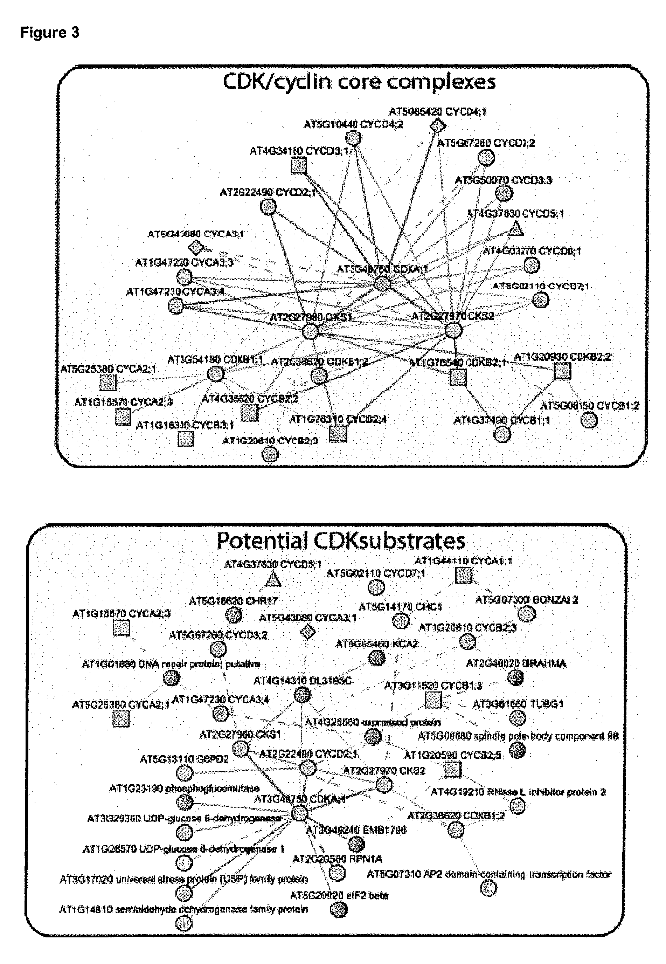

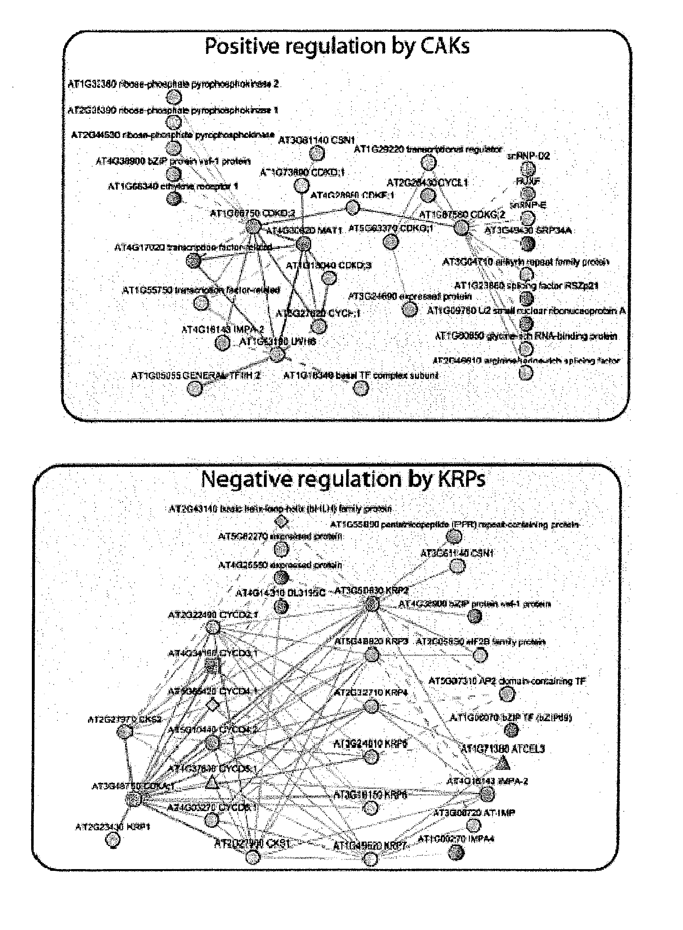

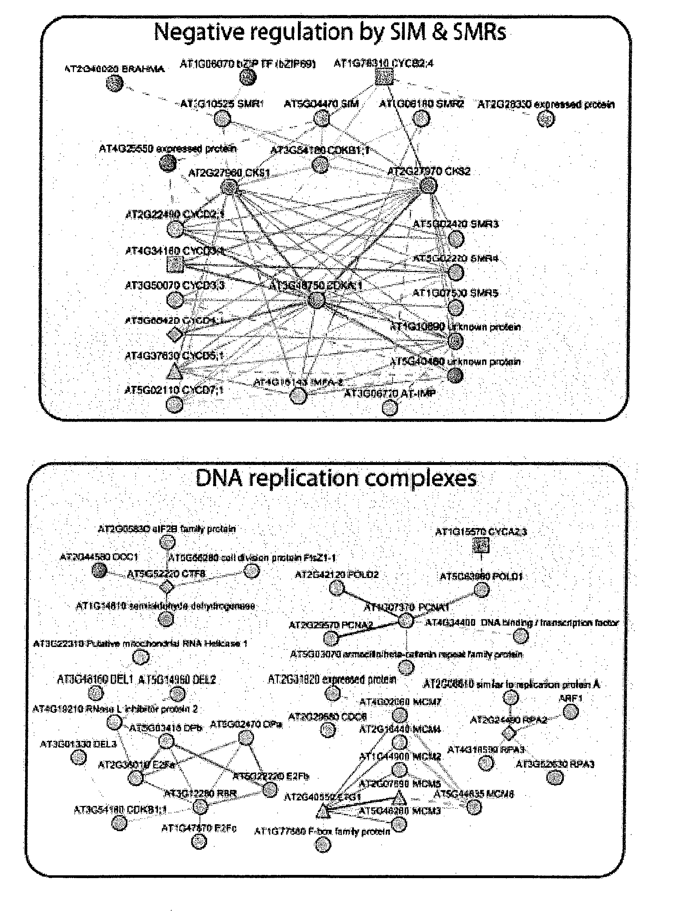

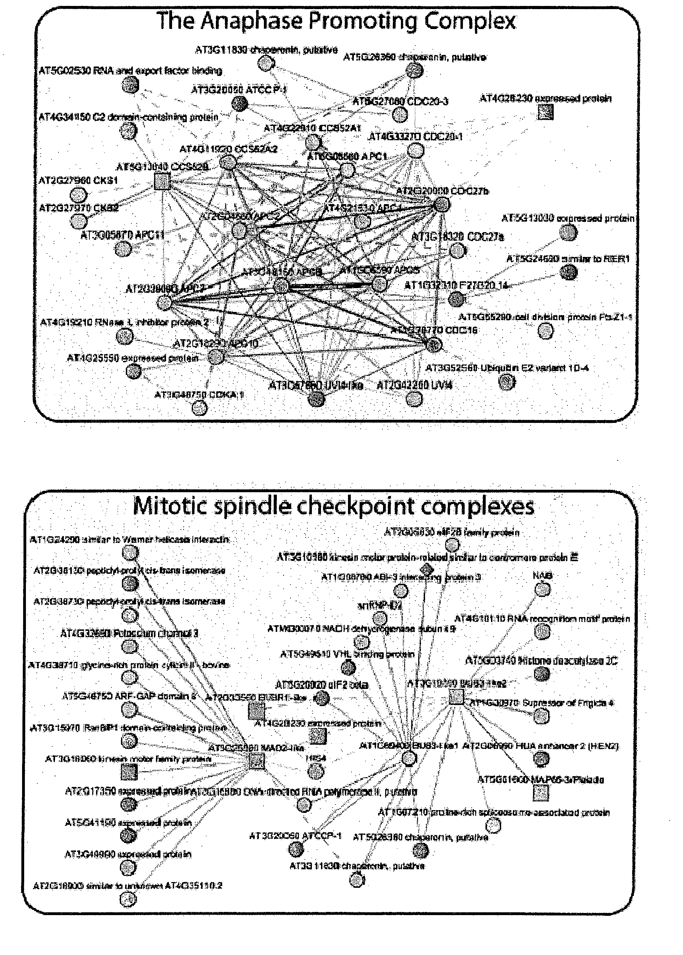

The robustness of the data is further exemplified by the observation that 46% of the core and 8% of the non-core dataset interactions are between baits, as our baits are supposed to act in common pathways. Screening our data for overlap with existing protein-protein interaction databases learned that 66% of the core and 95% of the non-core dataset interactions are new. On the other hand, this implicates that one third of the core dataset is validated by other means. Finally, interactions from both datasets tend to be more co-expressed compared to interactions from randomized datasets as assessed by calculation of the transcript Pearson Correlation Coefficient (PCC) (Fig. 1 C). The integrated cell cycle interactome can be accessed through a Cytoscape webstart and is provided as a matrix pivot table that allows easy querying of the interactome. To demonstrate the biological importance of the interactome, a selection of subnetworks is discussed below, covering interactions from both the core and non-core dataset (Fig. 3).

### Example 5: isolated genes of the CDK complexes

Key players in cell cycle progression are cyclin-dependent kinase (CDK) complexes. CDKA;1, the *Arabidopsis* ortholog of yeast cdc2/cdc28, co-purified with all tested D-type cyclins and with A3-type cyclins, but not with the mitotic A1-, A2- or B-type cyclins. Combining our interactome data with expression data (Menges et al. 2005) we speculate that at cell cycle reentry and early in G1-phase, CDKA;1 binds CYCD3;3 and CYCD5;1. Further on in G1-phase and at the G1/S checkpoint, CDKA;1 binds a variety of D-type cyclins, such as CYCD4;1, CYCD4;2, CYCD3;1, CYCD6;1 and CYCD7;1. In addition, CDKA;1 interacts with S-phase specific A3-type cyclins. The other A- and B-type cyclins, of which most possess a peak of expression at the G2/M-boundary, bind the plant-specific mitotic B-type CDKs. B1-type cyclins associate exclusively with B2-type CDKs, while the remainder A- and B-type cyclins preferentially bind B1-type CDKs. Although transient interactions are more difficult to screen with TAP, our interactome contains different potential CDK substrates. As predicted (Geisler-Lee et al., 2007), CDKA;1 is present as a highly connected hub in the core network. It co-purified the unknown protein AT4G14310 which was further present in complexes with CKS1, CKS2, CYCA3;1, CyCA3;4 and KRP2 and the reverse purification confirmed interaction with CDKA;1 and CKS2 and revealed interaction with the plant-specific kinesin motor protein KCA2 involved in division plane determination. Next, CDKA;1 was pulled down with the 26S proteasome complex, purified through RPN1a, possibly reflecting cell cycle regulation of the 26S proteasome. CDKA;1 further interacted with 3 proteins from the UDP-xylose biosynthesis pathway, coupling cell cycle regulation with cell wall synthesis (Siefert, 2004). With 3 A-type cyclins, we picked up a DNA repair protein and with CYCB1;3 we found γ-tubulin and a spindle pole body component, 2 proteins involved in microtubule nucleation during e.g. assembly of the preprophase band, a plant specific structure required for polarity determination during cell cycle (Erhardt et al., 2002). Furthermore, some interesting chromatin remodeling proteins were identified with different cyclins: CHR17, an E2F-upregulated ISWI protein (Huanca-Mamani et al., 2005) interacted with CYCD3;2 and CYCD5;1. CHC1 associated with CYCA1;1, CYCD7;1, CYCB2;3 and CKS2, and BRAHMA, a SWI/SNF chromatin remodeling ATPase implicated in the formation and/or maintenance of cotyledon boundary cells during embryogenesis (Kwon et al., 2006), was identified with CYCB1;3 and CYCB2;3.

### Example 6: isolation of positive regulators of the cell cycle

For full activity CDKs require, next to cyclin binding, phosphorylation of a threonine residue within the T-loop by CDK activating kinases (CAK). The *Arabidopsis* genome encodes 4 CAKs, namely 3 D-type CDKs, homologous to human CDK7, and 1 cyclin-independent CAK-activating kinase (CAKAK) CDKF;1. Here we show that both CDKD;2 and CDKD;3 form a trimeric complex with CYCH;1 and the CAK assembly factor MAT1. Like in rice (Rohila et al., 2006), CDKD;2 is also part of the basal TFIIH complex involved in transcription and DNA repair, as 3 members co-purified (UVH6/XPD, AT1 G55750 and AT4G17020). In this complex, CDKD;2 activates transcription through phosphorylation of the C-Terminal Domain (CTD) of RNA polymerase II. With UVH6 and MAT1 as baits, we confirmed interaction with CDKD;2 and purified two more proteins of the TFIIH complex. CDKD;2 further co-purified proteins involved in nucleotide biosynthesis, namely 3 ribose-phosphate pyrophosphokinases. More upstream, the monomeric CAKAK CDKF;1 activates CDKD;2 in a cyclin-independent manner. On the other hand, CDKF;1 also binds CDKG;2. The G-type CDK class has 2 members in *Arabidopsis,* and is homologous to the human cytokinesis-associated p58 galactosyltransferase protein. Here we discovered CYCL1, a cyclin with a SR-like splicing domain (Forment et al., 2002), as the regulatory cyclin partner of both G-type CDKs, validating the clustering of CYCL1 with CDKG;2 in a tissue-specific gene expression analysis (Menges et al., 2005). Both core and non-core interacting proteins hint for a function of CDKG/CYCL complexes in regulation of transcription and splicing, so activation of CDKF;1 could lead to altered splicing events during cell proliferation.

### Example 7: isolation of negative regulators of the cell cycle

Negative regulation of cell cycle progression is achieved by docking of small proteins to the CDK/cyclin complexes. *Arabidopsis* encodes 7 proteins related to the mammalian Kip/Cip inhibitors, known as Kip-related proteins (KRPs). Here we show that all KRPs, except KRP1, interact with both CDKA;1 and D-type cyclins. With 3 KRPs, CDKB1;2 and 2 APC activators we found an ethylene responsive AP2 transcription factor (TF), and with KRP2 we picked up a bZIP TF also found with B-type cyclins and CDKB1;2. In plants, a second family of cell cycle inhibitor proteins exist that are upregulated by abiotic and biotic stress, comprising SIAMESE (SIM) and SIAMESE-Related (SMR) proteins (Peres et al., 2007; Churchman et al., 2006 ). SIM is a nuclear protein promoting endoreduplication in trichomes by suppression of mitosis. It was proposed that it inhibits mitosis through inhibition of CDKA;1/CYCD complexes (Churchman et al., 2006). In our dataset however, SIM co-purifies CDKB1;1 and not CDKA;1, so endoreduplication may be triggered directly by inhibiting mitotic CDKB/cyclin complexes. Next to SIM, also SMR1 and SMR2 associate with CDKB1;1, and the CDKB1;1 interactor CYCB2;4 binds AT2G28330, an additional member of the SMR family. In contrast, SMR3-5 bind CDKA;1 and D-type cyclins. Besides, with CDKA;1 and different D-type cyclins as bait, we picked up 2 new members of the SMR clan, AT5G40460 and AT1 G10690, and reverse purifications confirmed these interactions. As AT5G40460 was almost 20-fold induced in plants overexpressing E2Fa and DPa (Vandepoele et al., 2005), it may inhibit CDKA;1/CYCD complexes during S-phase preventing re-initiation of DNA replication. SMR1 further co-purified bZIP69, a TF also found with KRP3 and KRP5. Importins often co-purified both with KRPs and SMRs, supporting the importance of the regulation of their subcellular localization for their activity.

### Example 8: isolation of genes involved in nucleotide synthesis, DNA replication and DNA repair

At the G1/S boundary, CDKs activate the E2F/DP pathway by phosphorylation of the repressor RBR, inducing transcription of genes mainly involved in nucleotide synthesis, DNA replication and DNA repair. We demonstrate that E2Fa and E2Fb can associate both with DPa and DPb, and that all E2F and DP proteins co-purify RBR. Since CDKB1;1 interacted with DEL3, an atypical E2F protein lacking the trans-activation domain, we propose that DEL3 is regulated by CDKB1;1 activity, consistent with a second expression peak of DEL3 at G2/M (Menges et al., 2005). Interestingly, the mitotic CDKB1;1, and not CDKA;1, co-purified with RBR, providing further evidence that the E2F/DP/RBR network is not only active at G1/S but also at G2/M transitions, as was previously suggested in plants (Magyar et al., 2005) and mammalian cells (Ishida et al.,2001), or that mitotic CDK/cyclin complexes are active during S-phase as in yeast (Wuarin et al., 2002). We further identified some complexes involved in DNA replication, like the MCM complex, possessing helicase activity for unwinding of double stranded DNA during DNA replication. This complex was isolated with MCM6 as bait, together with the recently published (Takahashi et al., 2008) and highly co-expressed E2F-target gene 1 (ETG1). The co-purified fraction of proliferating cell nuclear antigen 1 (PCNA1), a sliding clamp for DNA polymerase and thus a key actor in DNA replication, contained PCNA2, two DNA polymerase delta subunits (POLD1-2), of which one also interacted with CYCA2;3, an armadillo/beta-catenin repeat family of unknown function and a DNA binding protein. Furthermore, we proof the existence of the alternative Ctf18 replication factor C complex in plants, required for sister chromatid cohesion in yeast (Mayer et al., 2001) and a protein complex involved in stabilization of single stranded DNA during replication, repair and transcription, including RPA2, 2 RPA3 proteins and a putative replication protein (Schultz et al., 2007).

### Example 9: Analysis of overexpression lines

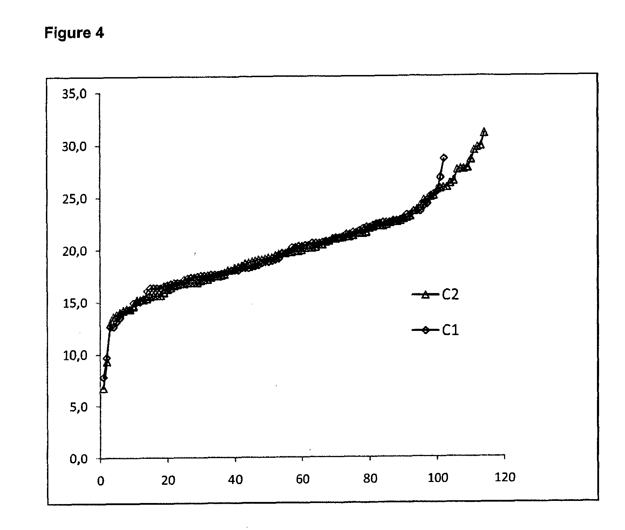

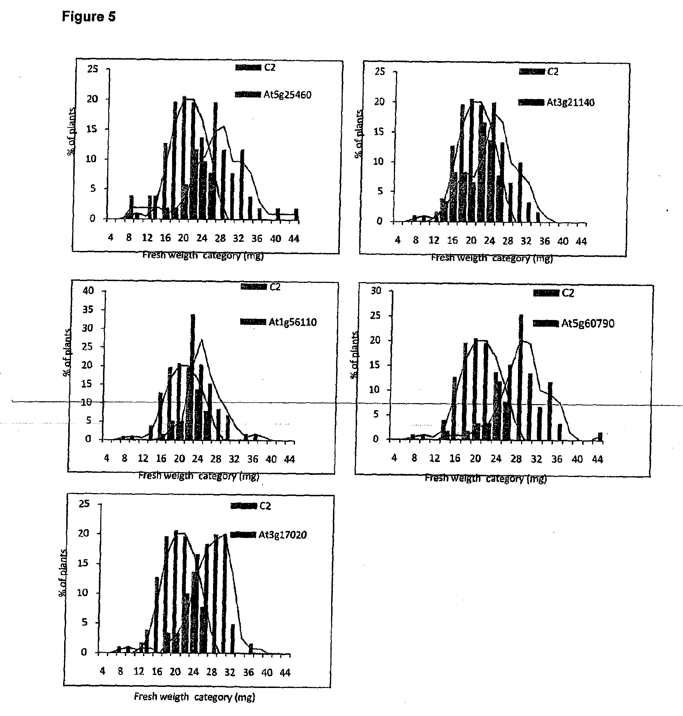

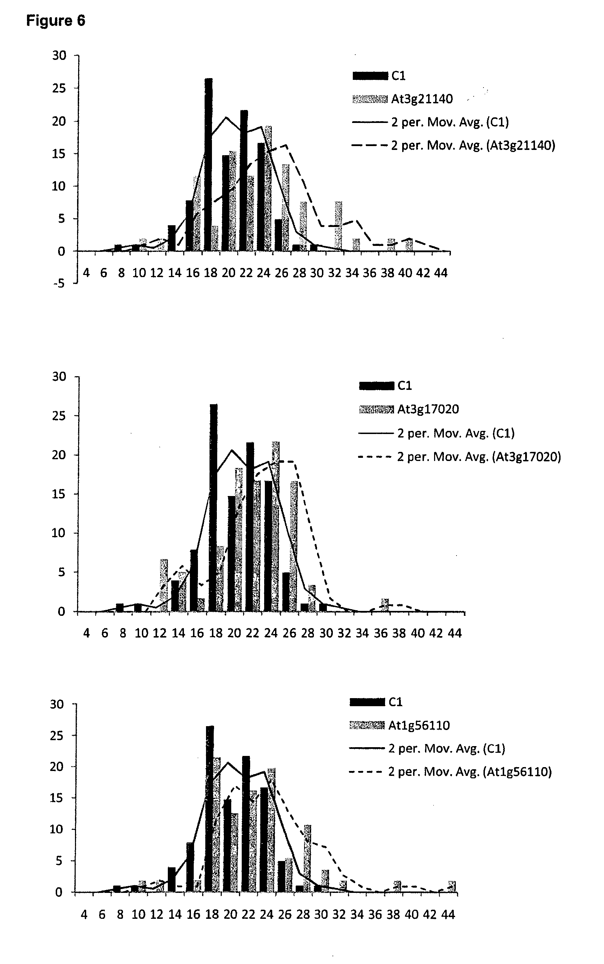

Thirty two genes were cloned into expression vectors using Gateway cloning to produce plants overexpressing the genes of interest under the control of the CaMV35S promoter (table, sheet OE produced and LOF requested). These constructs were used for *Arabidopsis thaliana* transformation using the flower dip method. Primo-transformants were selected for all the constructs and grown for seed production. The seeds from 11 constructs (At5g25460, At3g01280, At1g31760, At3g21140, At3g17020, At1g05805, At1g10690, At1g56110, At5g24690, At5g60790, At1g09760) were harvested and used to select lines having one site of insertion of the transgene. A biomass test has been carried on these segregating populations containing one insertion site. In this population, it is expected to find ¼ of wt, 2/4 of hemizygous and ¼ of homozygous plants. When the overexpression of a transgene leads to the production of larger plants, a fraction (¼ or ¾) of the plants analyzed will show an increased biomass. This method allows a fast screening of genes of which the overexpression gives a positive effect on plant growth. Plants were then grown under in vitro condition and the rosette fresh weight of approximately 60 plants was measured 20 days after stratification (table, sheet OE data+average). The control plants used in this experiment correspond to segregating plants transformed with an empty vector (C1 and C2). As shown in figure 4, for these two independent control lines, the plant fresh weight varies from 8 mg to 28 mg with an average of 20 mg. Among the 11 overexpressing lines analysed, we found that for 5 (At1g56110, At3g17020, At3g21140, At5g25460, At5g60790) this distribution of weight is shifted towards larger plants than in the controls (figure 5). This shift in biomass range was found for 3 of these lines in two independent transformants (figure 6). The presence of larger plants in this segregating population proves that these 5 genes are involved in the control of growth.

**Table 1: Overview of bait proteins used to elucidate the core cell cycle interactome of Arabidopsis thaliana.**

| | | | | | | |
|---|---|---|---|---|---|---|
| Topology = C- or N-terminal tag fusions. Tag refers to the applied tag being either the traditional TAP tag developed for *Saccharomyces cerevisiae (15)*, or an optimized dual affinity tag (GS) (2). Expression was indicated as (+) if the TAP fusion protein could be detected by western blot analysis. The total number of purifications performed per bait is shown, with a minimum of 2 experiments. | | | | | | |

| Bait | Locus | Category | Topology | Tag | Expressed | Purifications |
|---|---|---|---|---|---|---|
| CDKA;1 | AT3G48750 | core cell cycle | C + N | TAP | + | 11 |
| CDKB1;1 | AT3G54180 | core cell cycle | C + N | TAP | + | 4 |
| CDKB1;2 | AT2G38620 | core cell cycle | C | TAP | + | 2 |
| CDKB2;1 | AT1G76540 | core cell cycle | N | TAP | + | 2 |
| CDKB2;2 | AT1G20930 | core cell cycle | C | TAP | + | 4 |
| CDKC;1 | AT5G10270 | core cell cycle | C | TAP | + | 2 |
| CDKC;2 | AT5G64960 | core cell cycle | C | TAP | + | 4 |
| CDKD;1 | AT1G73690 | core cell cycle | C | TAP | + | 2 |
| CDKD;2 | AT1G66750 | core cell cycle | C + N | TAP + GS | + | 10 |
| CDKD;3 | AT1G18040 | core cell cycle | C | TAP | + | 2 |
| CDKE;1 | AT5G63610 | core cell cycle | N | TAP | + | 2 |
| CDKF;1 | AT4G28980 | core cell cycle | C | TAP | + | 3 |
| CDKG;1 | AT5G63370 | core cell cycle | C | GS | + | 2 |
| CDKG;2 | AT1G67580 | core cell cycle | C | TAP | + | 2 |
| CKS1 | AT2G27960 | core cell cycle | C | TAP + GS | + | 10 |
| CKS2 | AT2G27970 | core cell cycle | C | TAP | + | 2 |
| CYCA1;1 | AT1G44110 | core cell cycle | C | TAP | + | 2 |
| CYCA1;2 | AT1G77390 | core cell cycle | C | TAP | - | |
| CYCA2;1 | AT5G25380 | core cell cycle | N | TAP | + | 2 |
| CYCA2;2 | AT5G11300 | core cell cycle | C | TAP | + | 4 |
| CYCA2;3 | AT1G15570 | core cell cycle | N | TAP | + | 4 |
| CYCA2;4 | AT1G80370 | core cell cycle | C | TAP | - | |
| CYCA3;1 1 | AT5G43080 | core cell cycle | C | TAP | + | 4 |
| CYCA3;2 | AT1G47210 | core cell cycle | N | TAP | - | |
| CYCA3;3 | AT1G47220 | core cell cycle | N | GS | + | 2 |
| CYCA3;4 | AT1G47230 | core cell cycle | C | TAP | + | 4 |
| CYCB1;1 | AT4G37490 | core cell cycle | C | TAP + GS | + | 4 |
| CYCB1;2 | AT5G06150 | core cell cycle | C | TAP | + | 4 |
| CYCB1;3 | AT3G11520 | core cell cycle | C | TAP | + | 2 |
| CYCB1;4 | AT2G26760 | core cell cycle | C | TAP | + | 2 |
| CYCB2;1 | AT2G 17620 | core cell cycle | N | TAP | + | 2 |
| CYCB2;2 | AT4G35620 | core cell cycle | C | TAP | + | 5 |
| CYCB2;3 | AT1G20610 | core cell cycle | C | TAP | + | 4 |
| CYCB2;4 | AT1G76310 | core cell cycle | C | GS | + | 2 |
| CYCB2;5 | AT1G20590 | core cell cycle | C | TAP | + | 2 |
| CYCB3;1 | AT1G16330 | core cell cycle | N | TAP | + | 4 |
| CYCD1;1 | AT1G70210 | core cell cycle | C | TAP | - | |
| CYCD2;1 | AT2G22490 | core cell cycle | C | TAP | + | 6 |
| CYCD3;1 | AT4G34160 | core cell cycle | N | TAP | + | 2 |
| CYCD3;2 | AT5G67260 | core cell cycle | C | TAP | + | 3 |
| CYCD3;3 | AT3G50070 | core cell cycle | C | TAP | + | 4 |
| CYCD4;1 | AT5G65420 | core cell cycle | C | TAP | + | 2 |
| CYCD4;2 | AT5G10440 | core cell cycle | C | TAP | + | 2 |
| CYCDS;1 | AT4G37630 | core cell cycle | C | TAP | + | 2 |
| CYCD6;1 | AT4G03270 | core cell cycle | C | TAP | + | 2 |
| CYCD7;1 | AT5G02110 | core cell cycle | C | GS | + | 2 |
| CYCH;1 | AT5G27620 | core cell cycle | N | TAP | + | 4 |
| CYCT1;3 | AT1G27630 | core cell cycle | C | TAP | + | 2 |
| DEL1 | AT3G48160 | core cell cycle | N | TAP | + | 2 |
| DEL2 | AT5G14960 | core cell cycle | N | TAP | + | 2 |
| DEL3 | AT3G01330 | core cell cycle | N | TAP | + | 2 |
| DPa | AT5G02470 | core cell cycle | C | TAP | + | 2 |
| DPb | AT5G03415 | core cell cycle | N | TAP | + | 2 |
| E2Fa | AT2G36010 | core cell cycle | C | TAP + GS | + | 5 |
| E2Fb | AT5G22220 | core cell cycle | N | TAP | + | 2 |
| E2Fc | AT1G47870 | core cell cycle | C | TAP | + | 5 |
| KRP1 | AT2G23430 | core cell cycle | N | TAP | + | 2 |
| KRP2 | AT3G50630 | core cell cycle | N | TAP | + | 6 |
| KRP3 | AT5G48820 | core cell cycle | N | TAP | + | 4 |
| KRP4 | AT2G32710 | core cell cycle | N | TAP | + | 6 |
| KRP5 | AT3G24810 | core cell cycle | N | TAP | + | 2 |
| KRP6 | AT3G 19150 | core cell cycle | N | TAP | + | 4 |
| KRP7 | AT1G49620 | core cell cycle | N | TAP | + | 2 |
| RBR | AT3G12280 | core cell cycle | N | TAP | + | 2 |
| WEE1 | AT1G02970 | core cell cycle | N | TAP | + | 4 |
| Cdc25-like | AT5G03455 | core cell cycle | C | TAP | + | 2 |
| MAT1 | AT4G30820 | core cell cycle | N | TAP | + | 2 |
| SIM | AT5G04470 | core cell cycle | C | TAP | + | 4 |
| SMR1 | AT3G10525 | core cell cycle | C | TAP | + | 4 |
| SMR2 | AT1G08180 | core cell cycle | C | TAP | + | 2 |
| SMR3 | AT5G02420 | core cell cycle | C | TAP | + | 2 |
| SMR4 | AT5G02220 | core cell cycle | C + N | TAP | + | 4 |
| SMR5 | AT1G07500 | core cell cycle | N | GS | + | 2 |
| APC2 | AT2G04660 | APC core | N | TAP | + | 2 |
| APC4 | AT4G21530 | APC core | C | TAP | - | |
| APC7 | AT2G39090 | APC core | N | TAP | + | 2 |
| APC8 | AT3G48150 | APC core | N | TAP | + | 2 |
| APC10 | AT2G18290 | APC core | N | TAP | + | 4 |
| APC11 | AT3G05870 | APC core | C | TAP | + | 2 |
| CDC16 | AT1G78770 | APC core | C | TAP | + | 2 |
| CDC27B | AT2G20000 | APC core | C | TAP | + | 2 |
| CCS52A1 | AT4G22910 | APC activator | N | TAP | + | 4 |
| CCS52A2 | AT4G11920 | APC activator | N | TAP | + | 6 |
| CCS52B | AT5G13840 | APC activator | N | TAP | + | 4 |
| CDC20.1 | AT4G33270 | APC activator | N | TAP | + | 2 |
| CDC20.3 | AT5G27080 | APC activator | N | TAP | + | 2 |
| CDC20.6 | AT5G27945 | APC activator | C | TAP | - | |
| RPN1a | AT2G20580 | 26S proteasome | N | TAP | + | 2 |
| CDC6 | AT2G29680 | DNA replication | N | TAP | + | 2 |
| CDC6b | AT1G07270 | DNA replication | C | TAP | + | 2 |
| CTF8 | AT5G52220 | DNA replication | N | GS | + | 2 |
| ETG1 | AT2G40550 | DNA replication | C + N | TAP | + | 4 |
| MCM6 | AT5G44635 | DNA replication | C | TAP | + | 2 |
| MCM7 | AT4G02060 | DNA replication | C | TAP | + | 2 |
| ORC1a | AT4G14700 | DNA replication | N | TAP | + | 2 |
| PCNA1 | AT1G07370 | DNA replication | N | TAP | + | 2 |
| RPA2 | AT2G24490 | DNA replication | N | TAP | + | 2 |
| UVH6 | AT1G03190 | DNA repair | C | TAP | + | 2 |
| BUB3 | AT1G69400 | mitotic checkpoint | N | TAP | + | 2 |
| BUB3-like | AT3G19590 | mitotic checkpoint | N | TAP | + | 2 |
| BUBR1-like | AT2G33560 | mitotic checkpoint | N | TAP | + | 2 |
| MAD2-like | AT3G25980 | mitotic checkpoint | N | TAP | + | 3 |
| DL3195C | AT4G14310 | reverse | N | TAP | + | 2 |
| F27G20.14 | AT1G32310 | reverse | C | GS | + | 2 |
| expressed | AT5G40460 | reverse | C | TAP | + | 2 |
| expressed | AT1G10690 | reverse | N | TAP | + | 2 |
| UVI4 | AT2G42260 | reverse | N | TAP | + | 2 |
| UVI4-like | AT3G57860 | reverse | N | TAP | + | 2 |

**Table 2: List of control identifications.**

| | |
|---|---|
| Control identifications determined by mock TAP (7) or GS (3) purifications, and by purifications with extracts from cultures expressing TAP fusions of heterologous GFP (7), RFP (2) or β-glucuronidase (5), or GS fusions of heterologous GFP (8), or β-glucuronidase (4). Ribosomal proteins, actins and tubulins identified in these control experiments were not included. The number of control experiments is shown between brackets. | |

| Accession number | Protein name |
|---|---|
| AT1G01460 | phosphatidylinositol-4-phosphate 5-kinase family protein |
| AT1G02400 | gibberellin 2-oxidase, putative /GA2-oxidase, putative |
| AT1G02500 | S-adenosylmethionine synthetase 1 (SAM1) |
| AT1G02930 | glutathione S-transferase, putative |
| AT1G04190 | tetratricopeptide repeat (TPR)-containing protein |
| AT1G04600 | myosin, putative |
| AT1G04690 | potassium channel protein, putative |
| AT1G05320 | myosin-related |
| AT1G05450 | protease inhibitor/seed storage/lipid transfer protein (LTP)-related |
| AT1G05970 | expressed protein |
| AT1G06220 | elongation factor Tu family protein |
| AT1G06780 | glycosyl transferase family 8 protein |
| AT1G07920 | elongation factor 1-alpha |
| AT1 G07930 | elongation factor 1-alpha |
| AT1G08520 | magnesium-chelatase subunit chID, chloroplast, putative |
| AT1G09080 | luminal binding protein 3 (BiP-3) (BP3) |
| AT1G09450 | haspin-related |
| AT1G09780 | 2,3-biphosphoglycerate-independent phosphoglycerate mutase, putative |
| AT1G09980 | expressed protein |
| AT1G10270 | pentatricopeptide (PPR) repeat-containing protein |
| AT1G10390 | nucleoporin family protein |
| AT1G10590 | DNA-binding protein-related |
| AT1 G11480 | eukaryotic translation initiation factor-related |
| AT1G12410 | ATP-dependent Clp protease proteolytic subunit (CIpP2) |
| AT1G13020 | eukaryotic translation initiation factor, putative |
| AT1G13440 | glyceraldehyde 3-phosphate dehydrogenase, cytosolic, putative |
| AT1G13610 | expressed protein |
| AT1G13910 | F16A14.12 (leucine-rich repeat family protein) |
| AT1G14315 | Putative F-box protein |
| AT1G14850 | non-repetitive/WGA-negative nucleoporin family protein |
| AT1G14980 | 10 kDa chaperonin (CPN10) |
| AT1G15730 | PRLI-interacting factor L, putative (Fragment) |
| AT1G16030 | heat shock protein 70, putative |
| AT1G16410 | cytochrome P450, putative |
| AT1G16750 | expressed protein |
| AT1G18370 | kinesin motor family protein (NACK1) |
| AT1G19290 | pentatricopeptide (PPR) repeat-containing protein |
| AT1G19920 | sulfate adenylyltransferase 2 / ATP-sulfurylase 2 |
| AT1G21480 | exostosin family protein |
| AT1G23410 | ubiquitin extension protein, putative / 40S ribosomal protein S27A |
| AT1G24300 | GYF domain-containing protein |
| AT1G24510 | T-complex protein 1 epsilon subunit, putative |
| AT1G24938 | hypothetical protein |
| AT1G26750 | expressed protein |
| AT1G27430 | GYF domain-containing protein |
| AT1G27970 | nuclear transport factor 2 (NTF2), putative |
| AT1G29350 | kinase-related |
| AT1G29370 | kinase-related |
| AT1G31230 | bifunctional aspartate kinase/homoserine dehydrogenase |
| AT1G31280 | PAZ domain-containing protein / piwi domain-containing protein (Fragment) |
| AT1G31770 | ABC transporter family protein |
| AT1G34610 | UIp1 protease family protein |
| AT1G35380 | hypothetical protein |
| AT1G36120 | putative reverse transcriptase |
| AT1 G36280 | adenylosuccinate lyase, putative / adenylosuccinase, putative |
| AT1G36580 | 2,4-dienoyl-CoA reductase-related |
| AT1G37200 | gypsy-like retrotransposon family protein |
| AT1G44900 | DNA replication licensing factor, putative, similar to DNA replication licensing factor MCM2 |
| AT1G48400 | F-box family protein |
| AT1G48630 | guanine nucleotide-binding family protein / activated protein kinase |
| AT1G49040 | stomatal cytokinesis defective / SCD1 protein (SCD1) |
| AT1G51380 | eukaryotic translation initiation factor 4A, putative |
| AT1G51710 | ubiquitin-specific protease 6, putative (UBP6) |
| AT1G52450 | ubiquitin carboxyl-terminal hydrolase-related |
| AT1G52740 | histone H2A, putative |
| AT1G53240 | malate dehydrogenase [NAD], mitochondrial, putative |
| AT1G53550 | F-box family protein |
| AT1G54270 | eukaryotic translation initiation factor 4A-2 |
| AT1G55490 | RuBisCO subunit binding-protein beta subunit, chloroplast |
| AT1G56070 | elongation factor 2, putative |
| AT1G56070 | elongation factor 2, putative |
| AT1G56410 | heat shock cognate 70 kDa protein, putative |
| AT1G58265 | cytochrome P450-related |
| AT1G59610 | dynamin-like protein, putative (ADL3) |
| AT1G61210 | WD-40 repeat family protein / katanin p80 subunit, putative |
| AT1G61300 | disease resistance protein (NBS-LRR class), putative |
| AT1G61570 | mitochondrial import inner membrane translocase (TIM13) |
| AT1G62020 | coatomer protein complex, subunit alpha, putative |
| AT1G62610 | short-chain dehydrogenase/reductase (SDR) family protein |
| AT1G62630 | disease resistance protein (CC-NBS-LRR class), putative |
| AT1G64520 | 26S proteasome regulatory subunit, putative (RPN12) |
| AT1G64550 | ABC transporter family protein |
| AT1G65130 | ubiquitin carboxyl-terminal hydrolase-related |
| AT1G65290 | acyl carrier family protein / ACP family protein |
| AT1G65540 | calcium-binding EF hand family protein |
| AT1G65870 | disease resistance-responsive family protein |
| AT1G66410 | calmodulin |
| AT1G66510 | AAR2 protein family |
| AT1G67090 | ribulose bisphosphate carboxylase small chain 1A |
| AT1G68750 | phosphoenolpyruvate carboxylase family protein |
| AT1G68910 | expressed protein similar to Myosin heavy chain, nonmuscle type B |
| AT1G70100 | expressed protein |
| AT1G71220 | UDP-glucose:glycoprotein glucosyltransferase, putative |
| AT1G71270 | Vps52/Sac2 family protein |
| AT1G72000 | beta-fructofuranosidase, putative / invertase, putative / saccharase, putative |
| AT1G72730 | eukaryotic translation initiation factor 4A, putative |
| AT1G73720 | transducin family protein / WD-40 repeat family protein |
| AT1G75010 | MORN (Membrane Occupation and Recognition Nexus) repeat-containing protein |
| AT1G76520 | auxin efflux carrier family protein |
| AT1G76530 | auxin efflux carrier family protein |
| AT1G77120 | alcohol dehydrogenase (ADH) |
| AT1G78130 | transporter-related low similarity to spinster type III |
| AT1G78390 | 9-cis-epoxycarotenoid dioxygenase, putative |
| AT1G78900 | vacuolar ATP synthase catalytic subunit A |
| AT1G79280 | expressed protein |
| AT1G79550 | phosphoglycerate kinase, putative |
| AT1G79920 | heat shock protein 70, putative |
| AT1G79930 | heat shock protein, putative |
| AT1G80070 | splicing factor, putative |
| AT1G80270 | DNA-binding protein, putative |
| AT1G80670 | transducin family protein / WD-40 repeat family protein |
| AT2G01210 | leucine-rich repeat transmembrane protein kinase, putative |
| AT2G01350 | quinolinate phosphoribosyl transferase family protein |
| AT2G02160 | zinc finger (CCCH-type) family protein |
| AT2G03430 | ankyrin repeat family protein |
| AT2G04030 | heat shock protein, putative |
| AT2G05990 | enoyl-[acyl-carrier protein] reductase [NADH], chloroplast, putative |
| AT2G06850 | xyloglucan:xyloglucosyl transferase |
| AT2G07620 | putative helicase |
| AT2G07714 | transcription factor-related similar to male sterility 1 proteins |
| AT2G13680 | callose synthase 5 (1,3-beta-glucan synthase) |
| AT2G14120 | dynamin-like protein 2b |
| AT2G14880 | SWIB complex BAF60b domain-containing protein |
| AT2G16600 | peptidyl-prolyl cis-trans isomerase, cytosolic / cyclophilin |
| AT2G17130 | isocitrate dehydrogenase subunit 2 / NAD+ isocitrate dehydrogenase subunit 2 |
| AT2G18700 | glycosyl transferase family 20 protein |
| AT2G 19070 | transferase family protein |
| AT2G19210 | leucine-rich repeat protein kinase, putative |
| AT2G20330 | transducin family protein / WD-40 repeat family protein |
| AT2G20420 | succinyl-CoA ligase [GDP-forming] beta-chain, mitochondrial, putative |
| AT2G20580 | 26S proteasome regulatory subunit S2 (RPN1) |
| AT2G20805 | hypothetical protein |
| AT2G21170 | triosephosphate isomerase, chloroplast, putative |
| AT2G21390 | coatomer protein complex, subunit alpha, putative |
| AT2G21410 | vacuolar proton ATPase, putative |
| AT2G21920 | hypothetical protein (Fragment) |
| AT2G22230 | beta-hydroxyacyl-ACP dehydratase, putative |
| AT2G23930 | small nuclear ribonucleoprotein G, putative |
| AT2G24420 | DNA repair ATPase-related contains 2 transmembrane domains |
| AT2G26570 | expressed protein |
| AT2G26890 | DNAJ heat shock N-terminal domain-containing protein |
| AT2G27030 | calmodulin |
| AT2G27610 | pentatricopeptide (PPR) repeat-containing protein |
| AT2G27870 | hypothetical protein |
| AT2G28000 | RuBisCO subunit binding-protein alpha subunit, chloroplast |
| AT2G28620 | kinesin motor protein-related |
| AT2G29540 | DNA-directed RNA polymerase I(A) and III(C) 14 kDa subunit (RPAC14) |
| AT2G31680 | Ras-related GTP-binding protein, putative |
| AT2G32240 | putative myosin heavy chain |
| AT2G33210 | chaperonin, putative |
| AT2G33730 | DEAD box RNA helicase, putative |
| AT2G34170 | expressed protein |
| AT2G35605 | SWIB complex BAF60b domain-containing protein |
| AT2G36060 | ubiquitin-conjugating enzyme family protein |
| AT2G36260 | iron-sulfur cluster assembly complex protein, putative |
| AT2G36460 | fructose-bisphosphate aldolase, putative |
| AT2G36810 | expressed protein |
| AT2G37230 | pentatricopeptide (PPR) repeat-containing protein |
| AT2G37420 | kinesin motor protein-related |
| AT2G38560 | transcription factor S-II (TFIIS) domain-containing protein |
| AT2G38810 | histone H2A, putative |
| AT2G39730 | ribulose bisphosphate carboxylase/oxygenase activase |
| AT2G39990 | eukaryotic translation initiation factor 3 subunit 5 |
| AT2G40070 | expressed protein |
| AT2G40270 | protein kinase family protein |
| AT2G41450 | GCN5-related N-acetyltransferase (GNAT) family protein |
| AT2G41800 | expressed protein |
| AT2G42230 | tubulin-specific chaperone C-related |
| AT2G42450 | lipase class 3 family protein |
| AT2G42520 | DEAD box RNA helicase, putative |
| AT2G43160 | epsin N-terminal homology (ENTH) domain-containing protein |
| AT2G43750 | cysteine synthase, chloroplast / O-acetylserine (thiol)-lyase |
| AT2G44060 | late embryogenesis abundant family protein / LEA family protein |
| AT2G45030 | mitochondrial elongation factor, putative |
| AT2G45620 | nucleotidyltransferase family protein |
| AT2G46520 | cellular apoptosis susceptibility protein, putative / importin-alpha |
| AT2G47110 | ubiquitin extension protein 6 (UBQ6) /40S ribosomal protein S27A |
| AT3G01040 | glycosyl transferase family 8 protein |
| AT3G01720 | expressed protein |
| AT3G01740 | expressed protein |
| AT3G02200 | proteasome family protein |
| AT3G02230 | reversibly glycosylated polypeptide-1 |
| AT3G02500 | unknown protein |
| AT3G02530 | chaperonin, putative |
| AT3G02650 | pentatricopeptide (PPR) repeat-containing protein |
| AT3G03300 | DEAD/DEAH box helicase carpel factory-related |
| AT3G03780 | AtMS2 \|AtMS2 (Arabidopsis thaliana methionine synthase 2) |
| AT3G03960 | chaperonin, putative |
| AT3G04120 | glyceraldehyde-3-phosphate dehydrogenase, cytosolic |
| AT3G05810 | expressed protein |
| AT3G07160 | glycosyl transferase family 48 protein |
| AT3G07630 | prephenate dehydratase family protein |
| AT3G07810 | heterogeneous nuclear ribonucleoprotein, putative |
| AT3G08030 | expressed protein |
| AT3G08530 | clathrin heavy chain, putative |
| AT3G08590 | 2,3-biphosphoglycerate-independent phosphoglycerate mutase, putative |
| AT3G09170 | UIp1 protease family protein |
| AT3G09350 | armadillo/beta-catenin repeat family protein |
| AT3G09440 | heat shock cognate 70 kDa protein 3 |
| AT3G10310 | kinesin motor protein-related |
| AT3G10860 | ubiquinol-cytochrome C reductase complex ubiquinone-binding protein |
| AT3G11130 | clathrin heavy chain, putative |
| AT3G11400 | eukaryotic translation initiation factor 3G |
| AT3G11770 | expressed protein |
| AT3G11950 | ATHST; prenyltransferase |
| AT3G12580 | heat shock protein 70, putative |
| AT3G12800 | short-chain dehydrogenase/reductase (SDR) family protein |
| AT3G13160 | pentatricopeptide (PPR) repeat-containing protein |
| AT3G13460 | expressed protein |
| AT3G13470 | chaperonin, putative |
| AT3G13920 | eukaryotic translation initiation factor 4A-1 |
| AT3G14990 | 4-methyl-5(b-hydroxyethyl)-thiazole monophosphate biosynthesis protein |
| AT3G15020 | malate dehydrogenase [NAD], mitochondrial, putative |
| AT3G16230 | expressed protein similar to ASC-1 complex subunit P50 |
| AT3G16270 | expressed protein |
| AT3G16540 | DegP protease, putative |
| AT3G16640 | translationally controlled tumor family protein |
| AT3G17300 | expressed protein |
| AT3G17360 | kinesin motor protein-related similar to KLP2 protein |
| AT3G17390 | S-adenosylmethionine synthetase, putative |
| AT3G18000 | phosphoethanolamine N-methyltransferase 1 |
| AT3G18190 | chaperonin, putative |
| AT3G18530 | expressed protein |
| AT3G19050 | kinesin motor protein-related |
| AT3G20670 | histone H2A, putative |
| AT3G20780 | topoisomerase 6 subunit B (TOP6B) |
| AT3G21070 | ATP-NAD kinase family protein |
| AT3G22330 | DEAD box RNA helicase, putative |
| AT3G22890 | sulfate adenylyltransferase 1 /ATP-sulfurylase 1 |
| AT3G23990 | chaperonin (CPN60) (HSP60) |
| AT3G24890 | synaptobrevin-related |
| AT3G26020 | serine/threonine protein phosphatase 2A (PP2A) regulatory subunit B', putative |
| AT3G26380 | glycosyl hydrolase family protein 27 |
| AT3G27400 | pectate lyase family protein |
| AT3G28925 | hypothetical protein |
| AT3G42100 | AT hook motif-containing protein-related |
| AT3G42270 | putative protein |
| AT3G43300 | guanine nucleotide exchange family protein |
| AT3G43810 | calmodulin |
| AT3G45770 | oxidoreductase, zinc-binding dehydrogenase family protein |
| AT3G46560 | mitochondrial import inner membrane translocase (TIM9) |
| AT3G46990 | hypothetical protein |
| AT3G47850 | expressed protein |
| AT3G48250 | pentatricopeptide (PPR) repeat-containing protein |
| AT3G48670 | XH/XS domain-containing protein / XS zinc finger domain-containing protein |
| AT3G48870 | ATP-dependent Clp protease ATP-binding subunit (CIpC) |
| AT3G49640 | nitrogen regulation family protein |
| AT3G50280 | transferase family protein |
| AT3G50370 | expressed protein |
| AT3G51150 | kinesin motor family protein |
| AT3G51570 | disease resistance protein (TIR-NBS-LRR class), putative |
| AT3G51950 | zinc finger (CCCH-type) family protein / RNA recognition motif (RRM) |
| AT3G52140 | tetratricopeptide repeat (TPR)-containing protein |
| AT3G52930 | fructose-bisphosphate aldolase, putative |
| AT3G54360 | expressed protein DNA-binding Mel-18 protein |
| AT3G54610 | histone acetyltransferase (GCN5) |
| AT3G54940 | cysteine proteinase, putative |
| AT3G55000 | Tonneau 1a |
| AT3G55005 | Tonneau 1b |
| AT3G55290 | short-chain dehydrogenase/reductase (SDR) family protein |
| AT3G55310 | short-chain dehydrogenase/reductase (SDR) family protein |
| AT3G55460 | SC35-like splicing factor, 30 kD (SCL30) |
| AT3G55770 | LIM domain-containing protein |
| AT3G57290 | eukaryotic translation initiation factor 3E |
| AT3G58510 | DEAD box RNA helicase, putative (RH11) |
| AT3G58610 | ketol-acid reductoisomerase |
| AT3G59760 | cysteine synthase, mitochondrial, putative |
| AT3G62010 | expressed protein |
| AT3G62250 | ubiquitin extension protein 5 (UBQ5) /40S ribosomal protein S27A |
| AT4G00020 | BRCA2 repeat-containing protein |
| AT4G01290 | expressed protein |
| AT4G01800 | preprotein translocase secA subunit, putative |
| AT4G02930 | elongation factor Tu, putative |
| AT4G03550 | glycosyl transferase family 48 protein |
| AT4G11150 | vacuolar ATP synthase subunit E |
| AT4G11420 | eukaryotic translation initiation factor 3 subunit 10 |
| AT4G12130 | glycine cleavage T family protein |
| AT4G12770 | auxilin-related |
| AT4G12780 | auxilin-like protein |
| AT4G14060 | major latex protein-related |
| AT4G14130 | xyloglucan:xyloglucosyl transferase, putative |
| AT4G14140 | DNA (cytosine-5-)-methyltransferase (METII) |
| AT4G14260 | hypothetical protein |
| AT4G15640 | expressed protein |
| AT4G15760 | monooxygenase, putative (MO1) |
| AT4G17150 | unknown protein |
| AT4G17440 | expressed protein |
| AT4G17720 | RNA recognition motif (RRM)-containing protein |
| AT4G18040 | eukaryotic translation initiation factor 4E 1 |
| AT4G18080 | hypothetical protein |
| AT4G19530 | disease resistance protein (TIR-NBS-LRR class), putative |
| AT4G20160 | expressed protein |
| AT4G20360 | elongation factor Tu |
| AT4G20980 | eukaryotic translation initiation factor 3 subunit 7, putative |
| AT4G23670 | major latex protein-related |
| AT4G23850 | long-chain-fatty-acid--CoA ligase / long-chain acyl-CoA synthetase |
| AT4G24100 | protein kinase family protein |
| AT4G24280 | heat shock protein 70, putative |
| AT4G24760 | expressed protein |
| AT4G25580 | stress-responsive protein-related |
| AT4G26900 | imidazole glycerol phosphate synthase hisHF, chloroplast |
| AT4G27440 | protochlorophyllide reductase B, chloroplast |
| AT4G28400 | protein phosphatase 2C, putative |
| AT4G29800 | patatin-related low similarity to patatin precursor |
| AT4G31805 | WRKY family transcription factor |
| AT4G31820 | phototropic-responsive NPH3 family protein |
| AT4G31990 | aspartate aminotransferase, chloroplast / transaminase A (ASP5) (AAT1) |
| AT4G32500 | potassium channel protein, putative |
| AT4G32551 | WD-40 repeat family protein (LEUNIG) |
| AT4G33200 | myosin, putative |
| AT4G34290 | SWIB complex BAF60b domain-containing protein |
| AT4G34350 | LytB family protein |
| AT4G35260 | isocitrate dehydrogenase subunit 1 /NAD+ isocitrate dehydrogenase subunit 1 |
| AT4G35650 | isocitrate dehydrogenase, putative / NAD+ isocitrate dehydrogenase, putative |
| AT4G35890 | La domain-containing protein |
| AT4G35970 | L-ascorbate peroxidase, putative |
| AT4G36960 | RNA recognition motif (RRM)-containing protein |
| AT4G37420 | hypothetical protein |
| AT4G37580 | N-acetyltransferase, putative / hookless1 (HLS1) |
| AT4G37910 | heat shock protein 70, mitochondrial, putative |
| AT4G38030 | hypothetical protein |
| AT4G38070 | bHLH family protein |
| AT4G39580 | kelch repeat-containing F-box family protein |
| AT5G01010 | expressed protein |
| AT5G02490 | heat shock cognate 70 kDa protein 2 |
| AT5G02500 | heat shock cognate 70 kDa protein 1 |
| AT5G02590 | chloroplast lumen common family protein |
| AT5G03340 | cell division cycle protein 48, putative |
| AT5G03650 | 1,4-alpha-glucan branching enzyme |
| AT5G03690 | fructose-bisphosphate aldolase, putative |
| AT5G04590 | sulfite reductase / ferredoxin |
| AT5G05370 | ubiquinol-cytochrome C reductase complex ubiquinone-binding protein |
| AT5G05640 | nucleoprotein-related |
| AT5G05780 | 26S proteasome non-ATPase regulatory subunit 7, putative |
| AT5G06140 | phox (PX) domain-containing protein |
| AT5G06400 | pentatricopeptide (PPR) repeat-containing protein |
| AT5G06450 | expressed protein |
| AT5G06850 | anthranilate phosphoribosyltransferase-like protein |
| AT5G08415 | lipoic acid synthase family protein |
| AT5G08670 | ATP synthase beta chain, mitochondrial |
| AT5G08680 | ATP synthase beta chain, mitochondrial |
| AT5G08690 | ATP synthase beta chain, mitochondrial |
| AT5G09590 | heat shock protein 70 |
| AT5G09900 | 26S proteasome regulatory subunit, putative (RPN5) |
| AT5G10160 | beta-hydroxyacyl-ACP dehydratase, putative |
| AT5G11110 | sucrose-phosphate synthase -like protein |
| AT5G11340 | GCN5-related N-acetyltransferase (GNAT) family protein |
| AT5G12010 | expressed protein |
| AT5G12240 | expressed protein |
| AT5G12310 | zinc finger (C3HC4-type RING finger) family protein |
| AT5G13130 | hypothetical protein low similarity to microrchidia |
| AT5G14040 | mitochondrial phosphate transporter |
| AT5G15580 | expressed protein |
| AT5G15610 | proteasome family protein |
| AT5G15650 | reversibly glycosylated polypeptide-2 |
| AT5G15920 | structural maintenance of chromosomes (SMC) family protein |
| AT5G16070 | chaperonin, putative |
| AT5G17680 | disease resistance protein (TIR-NBS-LRR class), putative |
| AT5G17890 | LIM domain-containing protein / disease resistance protein-related |
| AT5G17920 | 5-methyltetrahydropteroyltriglutamate--homocysteine methyltransferase |
| AT5G18110 | novel cap-binding protein (nCBP) |
| AT5G18730 | hypothetical protein |
| AT5G19310 | homeotic gene regulator, putative |
| AT5G19550 | aspartate aminotransferase, cytoplasmic isozyme 1 / transaminase A |
| AT5G20010 | Ras-related GTP-binding nuclear protein |
| AT5G20020 | Ras-related GTP-binding nuclear protein |
| AT5G20360 | octicosapeptide/Phox/Bem1p (PB1) domain-containing protein (Fragment) |
| AT5G20720 | 20 kDa chaperonin, chloroplast |
| AT5G20890 | chaperonin, putative |
| AT5G21274 | calmodulin |
| AT5G22650 | expressed protein |
| AT5G23540 | 26S proteasome regulatory subunit, putative |
| AT5G23890 | expressed protein |
| AT5G23930 | mitochondrial transcription termination factor-related |
| AT5G24710 | WD-40 repeat family protein |
| AT5G25230 | elongation factor Tu family protein |
| AT5G25940 | early nodulin-related |
| AT5G26200 | mitochondrial substrate carrier family protein |
| AT5G26710 | glutamate-tRNA ligase, putative |
| AT5G26742 | DEAD box RNA helicase (RH3) |
| AT5G26800 | expressed protein |
| AT5G27390 | expressed protein |
| AT5G27920 | F-box family protein |
| AT5G28430 | hypothetical protein |
| AT5G28540 | luminal binding protein 1 |
| AT5G28850 | calcium-binding EF hand family protein |
| AT5G35360 | acetyl-CoA carboxylase, biotin carboxylase subunit (CAC2) |
| AT5G35620 | eukaryotic translation initiation factor 4E 2 |
| AT5G36670 | putative protein |
| AT5G37150 | tRNA-splicing endonuclease positive effector-related |
| AT5G37470 | hypothetical protein |
| AT5G37510 | NADH-ubiquinone dehydrogenase, mitochondrial, putative |
| AT5G39320 | UDP-glucose 6-dehydrogenase, putative |
| AT5G40060 | disease resistance protein (TIR-NBS-LRR class), putative |
| AT5G40760 | glucose-6-phosphate 1-dehydrogenase / G6PD |
| AT5G42020 | luminal binding protein 2 |
| AT5G42080 | GTP-binding protein / phragmoplastin, putative |
| AT5G42950 | GYF domain-containing protein |
| AT5G42980 | thioredoxin H-type 3 |
| AT5G43780 | sulfate adenylyltransferase 4 / ATP-sulfurylase 4 |
| AT5G44320 | eukaryotic translation initiation factor 3 subunit 7, putative |
| AT5G44510 | disease resistance protein (TIR-NBS-LRR class), putative (TAO1) |
| AT5G45060 | disease resistance protein (TIR-NBS-LRR class), putative |
| AT5G45140 | DNA-directed RNA polymerase, putative |
| AT5G47460 | pentatricopeptide (PPR) repeat-containing protein |
| AT5G47890 | NADH-ubiquinone oxidoreductase B8 subunit, putative |
| AT5G48620 | disease resistance protein (CC-NBS-LRR class), putative (Fragment) |
| AT5G49030 | tRNA synthetase class I (I, L, M and V) family protein |
| AT5G49910 | heat shock protein 70 |
| AT5G50370 | adenylate kinase, putative |
| AT5G50810 | mitochondrial import inner membrane translocase (TIM8) |
| AT5G50920 | ATP-dependent Clp protease ATP-binding subunit (CIpC) |
| AT5G51 000 | F-box family protein |
| AT5G51670 | expressed protein |
| AT5G51795 | Kin17 DNA-binding protein-related (Fragment) |
| AT5G52040 | arginine/serine-rich splicing factor RSP41 (RSP41) |
| AT5G52400 | cytochrome P450 family protein |
| AT5G53460 | glutamate synthase [NADH], chloroplast, putative |
| AT5G54640 | histone H2A |
| AT5G54670 | kinesin-like protein C (KATC) |
| AT5G55190 | Ras-related GTP-binding nuclear protein |
| AT5G56000 | heat shock protein 81 |
| AT5G56030 | heat shock protein 81 |
| AT5G56500 | chaperonin, putative |
| AT5G57350 | ATPase 3, plasma membrane-type / proton pump 3 |
| AT5G58070 | lipocalin, putative similar to temperature stress-induced lipocalin |
| AT5G59220 | protein phosphatase 2C, putative |
| AT5G59620 | putative protein |
| AT5G59880 | actin-depolymerizing factor 3 (ADF3) |
| AT5G60390 | elongation factor 1-alpha |
| AT5G60720 | expressed protein |
| AT5G60980 | nuclear transport factor 2 (NTF2) family protein |
| AT5G61780 | tudor domain-containing protein / nuclease family protein |
| AT5G63020 | disease resistance protein (CC-NBS-LRR class), putative |
| AT5G63400 | adenylate kinase |
| AT5G63800 | glycosyl hydrolase family 35 protein |
| AT5G64760 | 26S proteasome regulatory subunit, putative (RPN5) |
| AT5G66470 | expressed protein |

### Table 3: Overrepresentation of Biological Processes among the preys of the core (A) and non-core (B) dataset as determined with the Cytoscape plugin BiNGO.

### A. Overrepresentation of Biological Processes among the preys of the core dataset.

### Search parameters:

| |
|---|
| File created with BiNGO (c) on 22-okt-2008 at 16:17:17 |
| ontology: process |
| curator: GO |
| Selected ontology file : BiNGO.jarl/GO_Biological_Process |
| Selected annotation file : gene_association_2008okt04.tair |
| Overrepresentation |
| Selected statistical test : Hypergeometric test |
| Selected correction : Benjamini & Hochberg False Discovery Rate (FDR) correction |
| Selected significance level : 0.05 |
| Testing option : Test cluster versus whole annotation |
| Number of annotated genes in selection : 137 |
| Number of annotated genes in network/whole annotation : 25557 |

### Results:

| GO-ID | p-value | corr p-value | # selected | # total | Description |
|---|---|---|---|---|---|
| 7049 | 2,30E-34 | 7,17E-32 | 28 | 164 | cell cycle |
| 51726 | 1,11E-28 | 1,74E-26 | 21 | 93 | regulation of cell cycle |
| 6260 | 2,09E-18 | 2,17E-16 | 16 | 116 | DNA replication |
| 6263 | 4,33E-18 | 3,38E-16 | 13 | 58 | DNA-dependent DNA replication |
| 74 | 8,10E-15 | 5,05E-13 | 9 | 26 | regulation of progression through cell cycle |
| 6259 | 7,00E-14 | 3,60E-12 | 21 | 463 | DNA metabolism |
| 43285 | 8,43E-14 | 3,60E-12 | 18 | 312 | biopolymer catabolism |
| 30163 | 9,23E-14 | 3,60E-12 | 17 | 268 | protein catabolism |
| 9057 | 3,38E-13 | 1,10E-11 | 19 | 390 | macromolecule catabolism |
| 6511 | 4,22E-13 | 1,10E-11 | 16 | 249 | ubiquitin-dependent protein catabolism |
| 43632 | 4,22E-13 | 1,10E-11 | 16 | 249 | modification-dependent macromolecule catabolism |
| 19941 | 4,22E-13 | 1,10E-11 | 16 | 249 | modification-dependent protein catabolism |
| 51603 | 4,77E-13 | 1,15E-11 | 16 | 251 | proteolysis during cellular protein catabolism |
| 44257 | 6,86E-13 | 1,53E-11 | 16 | 257 | cellular protein catabolism |
| 44265 | 1,05E-12 | 2,19E-11 | 18 | 362 | cellular macromolecule catabolism |
| 79 | 4,38E-12 | 8,55E-11 | 6 | 10 | regulation of cyclin-dependent protein kinase activity |
| 6270 | 1,91E-11 | 3,51E-10 | 6 | 12 | DNA replication initiation |
| 278 | 3,69E-11 | 6,40E-10 | 8 | 40 | mitotic cell cycle |
| 6469 | 2,27E-10 | 3,38E-09 | 5 | 8 | negative regulation of protein kinase activity |
| 45736 | 2,27E-10 | 3,38E-09 | 5 | 8 | negative regulation of cyclin-dependent protein kinase activity |
| 51348 | 2,27E-10 | 3,38E-09 | 5 | 8 | negative regulation of transferase activity |
| 8151 | 2,45E-10 | 3,47E-09 | 97 | 1129 | cellular physiological process |
| | | | | 3 | |
| 9056 | 3,36E-10 | 4,55E-09 | 19 | 582 | catabolism |
| 45786 | 5,09E-10 | 6,62E-09 | 5 | 9 | negative regulation of progression through cell cycle |
| 42023 | 5,44E-10 | 6,79E-09 | 6 | 19 | DNA endoreduplication |
| 43086 | 1,01E-09 | 1,22E-08 | 5 | 10 | negative regulation of enzyme activity |
| 44248 | 1,30E-09 | 1,50E-08 | 18 | 560 | cellular catabolism |
| 43283 | 1,38E-09 | 1,54E-08 | 48 | 3695 | biopolymer metabolism |
| 45859 | 1,99E-09 | 2,04E-08 | 6 | 23 | regulation of protein kinase activity |
| 43549 | 1,99E-09 | 2,04E-08 | 6 | 23 | regulation of kinase activity |
| 9987 | 2,03E-09 | 2,04E-08 | 98 | 11858 | cellular process |
| 51338 | 2,64E-09 | 2,58E-08 | 6 | 24 | regulation of transferase activity |
| 50794 | 6,88E-09 | 6,51 E-08 | 33 | 2040 | regulation of cellular process |
| 51325 | 7,92E-09 | 7,06E-08 | 5 | 14 | interphase |
| 51329 | 7,92E-09 | 7,06E-08 | 5 | 14 | interphase of mitotic cell cycle |
| 51244 | 1,26E-08 | 1,09E-07 | 32 | 1980 | regulation of cellular physiological process |
| 50791 | 3,60E-08 | 3,04E-07 | 32 | 2070 | regulation of physiological process |
| 7582 | 1,33E-07 | 1,09E-06 | 100 | 13066 | physiological process |
| 50789 | 2,21 E-07 | 1,77E-06 | 33 | 2359 | regulation of biological process |
| 50790 | 2,72E-07 | 2,12E-06 | 7 | 81 | regulation of catalytic activity |
| 6139 | 6,29E-07 | 4,79E-06 | 37 | 2974 | nucleobase, nucleoside, nucleotide and nucleic acid metabolism |
| 43170 | 1,23E-06 | 9,17E-06 | 60 | 6396 | macromolecule metabolism |
| 6508 | 1,61 E-06 | 1,17E-05 | 16 | 718 | proteolysis |
| 51302 | 1,24E-05 | 8,77E-05 | 3 | 9 | regulation of cell division |
| 44238 | 1,87E-05 | 1,30E-04 | 69 | 8433 | primary metabolism |
| 51510 | 2,85E-05 | 1,93E-04 | 2 | 2 | regulation of unidimensional cell growth |
| 44237 | 1,45E-04 | 9,61 E-04 | 67 | 8584 | cellular metabolism |
| 80 | 1,70E-04 | 1,06E-03 | 2 | 4 | G1 phase of mitotic cell cycle |
| 82 | 1,70E-04 | 1,06E-03 | 2 | 4 | G1/S transition of mitotic cell cycle |
| 51318 | 1,70E-04 | 1,06E-03 | 2 | 4 | G1 phase |
| 7346 | 2,82E-04 | 1,73E-03 | 2 | 5 | regulation of progression through mitotic cell cycle |
| 48856 | 3,80E-04 | 2,28E-03 | 17 | 1248 | anatomical structure development |
| 48519 | 4,57E-04 | 2,69E-03 | 6 | 182 | negative regulation of biological process |
| 9653 | 5,58E-04 | 3,22E-03 | 10 | 526 | morphogenesis |
| 51243 | 6,65E-04 | 3,72E-03 | 5 | 129 | negative regulation of cellular physiological process |
| 51301 | 6,67E-04 | 3,72E-03 | 3 | 32 | cell division |
| 48523 | 6,89E-04 | 3,77E-03 | 5 | 130 | negative regulation of cellular process |
| 1558 | 7,82E-04 | 4,21E-03 | 2 | 8 | regulation of cell growth |
| 43118 | 1,02E-03 | 5,42E-03 | 5 | 142 | negative regulation of physiological process |
| 6281 | 1,09E-03 | 5,67E-03 | 5 | 144 | DNA repair |
| 9793 | 1,28E-03 | 6,56E-03 | 9 | 487 | embryonic development (sensu Magnoliophyta) |
| 6974 | 1,47E-03 | 7,39E-03 | 5 | 154 | response to DNA damage stimulus |
| 9790 | 2,01 E-03 | 9,94E-03 | 9 | 520 | embryonic development |
| 48316 | 2,28E-03 | 1,11E-02 | 9 | 530 | seed development |
| 48272 | 2,32E-03 | 1,11E-02 | 3 | 49 | trichome morphogenesis (sensu Magnoliophyta) |
| 48608 | 2,37E-03 | 1,12E-02 | 9 | 533 | reproductive structure development |
| 7148 | 2,43E-03 | 1,13E-02 | 6 | 252 | cell morphogenesis |
| 6457 | 2,58E-03 | 1,18E-02 | 6 | 255 | protein folding |
| 904 | 2,60E-03 | 1,18E-02 | 3 | 51 | cellular morphogenesis during differentiation |
| 7017 | 2,66E-03 | 1,19E-02 | 4 | 107 | microtubule-based process |
| 7275 | 2,81E-03 | 1,23E-02 | 19 | 1765 | development |
| 9965 | 2,94E-03 | 1,27E-02 | 4 | 110 | leaf morphogenesis |
| 7018 | 3,23E-03 | 1,37E-02 | 3 | 55 | microtubule-based movement |
| 9934 | 3,26E-03 | 1,37E-02 | 2 | 16 | regulation of meristem organization |
| 8152 | 3,29E-03 | 1,37E-02 | 70 | 10044 | metabolism |
| 19952 | 3,72E-03 | 1,53E-02 | 11 | 794 | reproduction |
| 48827 | 3,90E-03 | 1,58E-02 | 5 | 193 | phyllome development |
| 16043 | 3,95E-03 | 1,58E-02 | 16 | 1420 | cell organization and biogenesis |
| 9117 | 4,64E-03 | 1,83E-02 | 4 | 125 | nucleotide metabolism |
| 35315 | 4,95E-03 | 1,91E-02 | 3 | 64 | hair cell differentiation |
| 10026 | 4,95E-03 | 1,91 E-02 | 3 | 64 | trichome differentiation (sensu Magnoliophyta) |
| 51446 | 5,36E-03 | 2,02E-02 | 1 | 1 | positive regulation of progression through meiotic cell cycle |
| 51445 | 5,36E-03 | 2,02E-02 | 1 | 1 | regulation of progression through meiotic cell cycle |
| 30154 | 5,56E-03 | 2,07E-02 | 5 | 210 | cell differentiation |
| 9887 | 5,67E-03 | 2,08E-02 | 5 | 211 | organ morphogenesis |
| 30705 | 6,86E-03 | 2,49E-02 | 3 | 72 | cytoskeleton-dependent intracellular transport |
| 48468 | 8,86E-03 | 3,18E-02 | 3 | 79 | cell development |
| 10016 | 8,97E-03 | 3,18E-02 | 4 | 151 | shoot morphogenesis |
| 7276 | 9,80E-03 | 3,44E-02 | 4 | 155 | gametogenesis |
| 7050 | 1,07E-02 | 3,51E-02 | 1 | 2 | cell cycle arrest |
| 42276 | 1,07E-02 | 3,51 E-02 | 1 | 2 | error-prone postreplication DNA repair |
| 86 | 1,07E-02 | 3,51E-02 | 1 | 2 | G2/M transition of mitotic cell cycle |
| 45020 | 1,07E-02 | 3,51E-02 | 1 | 2 | error-prone DNA repair |
| 184 | 1,07E-02 | 3,51 E-02 | 1 | 2 | mRNA catabolism, nonsense-mediated decay |
| 6358 | 1,07E-02 | 3,51E-0₂ | 1 | 2 | regulation of global transcription from RNA polymerase II promoter |
| 46907 | 1,10E-02 | 3,58E-02 | 7 | 451 | intracellular transport |
| 9165 | 1,15E-02 | 3,70E-02 | 3 | 87 | nucleotide biosynthesis |
| 51649 | 1,18E-02 | 3,75E-02 | 7 | 457 | establishment of cellular localization |
| 51641 | 1,27E-02 | 3,93E-02 | 7 | 464 | cellular localization |
| 9744 | 1,27E-02 | 3,93E-02 | 2 | 32 | response to sucrose stimulus |
| 40008 | 1,27E-02 | 3,93E-02 | 2 | 32 | regulation of growth |
| 48366 | 1,59E-02 | 4,76E-02 | 4 | 179 | leaf development |
| 16288 | 1,60E-02 | 4,76E-02 | 2 | 36 | cytokinesis |
| 10071 | 1,60E-02 | 4,76E-02 | 1 | 3 | root meristem specification |
| 48367 | 1,60E-02 | 4,76E-02 | 5 | 273 | shoot development |

### B. Overrepresentation of Biological Processes among the preys of the non-core dataset.

### Search parameters:

| |
|---|
| File created with BiNGO (c) on 22-okt-2008 at 16:28:37 |
| ontology: process |
| curator: GO |
| Selected ontology file : BiNGO.jar!/GO_Biological_Process |
| Selected annotation file : gene_association_2008okt04.tair |
| Overrepresentation |
| Selected statistical test : Hypergeometric test |
| Selected correction : Benjamini & Hochberg False Discovery Rate (FDR) correction |
| Selected significance level : 0.05 |
| Testing option : Test cluster versus whole annotation |
| Number of annotated genes in selection : 197 |
| Number of annotated genes in network/whole annotation : 25538 |

### Results:

| GO-ID | p-value | corr p- value | # selecte d | # total | Description |
|---|---|---|---|---|---|
| 46686 | 5,78E-13 | 3,14E-10 | 22 | 396 | response to cadmium ion |
| 10038 | 4,07E-12 | 1,11E-09 | 22 | 437 | response to metal ion |
| 10035 | 9,76E-12 | 1,77E-09 | 22 | 457 | response to inorganic substance |
| 7049 | 4,69E-11 | 6,38E-09 | 14 | 166 | cell cycle |
| 51726 | 2,75E-09 | 2,99E-07 | 10 | 93 | regulation of cell cycle |
| 42221 | 1,01E-08 | 9,16E-07 | 38 | 1789 | response to chemical stimulus |
| 6259 | 2,22E-08 | 1,73E-06 | 18 | 462 | DNA metabolism |
| 8151 | 8,57E-08 | 5,83E-06 | 124 | 11291 | cellular physiological process |
| 50896 | 1,42E-07 | 8,56E-06 | 56 | 3617 | response to stimulus |
| 9987 | 2,38E-07 | 1,29E-05 | 127 | 11856 | cellular process |
| 42023 | 2,76E-07 | 1,36E-05 | 5 | 19 | DNA endoreduplication |
| 6263 | 3,15E-07 | 1,36E-05 | 7 | 58 | DNA-dependent DNA replication |
| 6260 | 3,24E-07 | 1,36E-05 | 9 | 117 | DNA replication |
| 6950 | 8,79E-07 | 3,42E-05 | 28 | 1300 | response to stress |
| 74 | 1,50E-06 | 5,43E-05 | 5 | 26 | regulation of progression through cell cycle |
| 7582 | 2,21 E-06 | 7,53E-05 | 133 | 13063 | physiological process |
| 6970 | 2,77E-06 | 8,87E-05 | 14 | 397 | response to osmotic stress |
| 6139 | 6,40E-06 | 1,94E-04 | 45 | 2973 | nucleobase, nucleoside, nucleotide and nucleic acid metabolism |
| 9056 | 1,27E-05 | 3,64E-04 | 16 | 582 | catabolism |
| 9628 | 2,56E-05 | 6,96E-04 | 23 | 1140 | response to abiotic stimulus |
| 9651 | 3,21 E-05 | 7,96E-04 | 12 | 369 | response to salt stress |
| 44248 | 3,22E-05 | 7,96E-04 | 15 | 560 | cellular catabolism |
| 6454 | 3,40E-05 | 8,04E-04 | 6 | 79 | translational initiation |
| 79 | 5,21 E-05 | 1,18E-03 | 3 | 10 | regulation of cyclin-dependent protein kinase activity |
| 9057 | 5,47E-05 | 1,19E-03 | 12 | 390 | macromolecule catabolism |
| 51510 | 5,92E-05 | 1,24E-03 | 2 | 2 | regulation of unidimensional cell growth |
| 44237 | 8,90E-05 | 1,79E-03 | 92 | 8580 | cellular metabolism |
| 15980 | 1,10E-04 | 2,15E-03 | 7 | 140 | energy derivation by oxidation of organic com pounds |
| 44265 | 1,25E-04 | 2,35E-03 | 11 | 362 | cellular macromolecule catabolism |
| 45333 | 1,33E-04 | 2,41 E-03 | 4 | 34 | cellular respiration |
| 43283 | 1,51 E-04 | 2,66E-03 | 48 | 3692 | biopolymer metabolism |
| 9826 | 1,63E-04 | 2,77E-03 | 7 | 149 | unidimensional cell growth |
| 43044 | 1,77E-04 | 2,91 E-03 | 2 | 3 | ATP-dependent chromatin remodeling |
| 7275 | 2,23E-04 | 3,56E-03 | 28 | 1765 | development |
| 278 | 2,53E-04 | 3,93E-03 | 4 | 40 | mitotic cell cycle |
| 9266 | 3,13E-04 | 4,74E-03 | 10 | 338 | response to temperature stimulus |
| 9719 | 4,96E-04 | 7,30E-03 | 20 | 1131 | response to endogenous stimulus |
| 6084 | 5,43E-04 | 7,77E-03 | 3 | 21 | acetyl-CoA metabolism |
| 6561 | 5,83E-04 | 8,05E-03 | 2 | 5 | proline biosynthesis |
| 9737 | 5,92E-04 | 8,05E-03 | 8 | 241 | response to abscisic acid stimulus |
| 40007 | 6,59E-04 | 8,74E-03 | 8 | 245 | growth |
| 45859 | 7,14E-04 | 9,04E-03 | 3 | 23 | regulation of protein kinase activity |
| 43549 | 7,14E-04 | 9,04E-03 | 3 | 23 | regulation of kinase activity |
| 43285 | 7,46E-04 | 9,23E-03 | 9 | 312 | biopolymer catabolism |
| 7148 | 7,91E-04 | 9,46E-03 | 8 | 252 | cell morphogenesis |
| 51338 | 8,12E-04 | 9,46E-03 | 3 | 24 | regulation of transferase activity |
| 6092 | 8,18E-04 | 9,46E-03 | 5 | 94 | main pathways of carbohydrate metabolism |
| 16049 | 9,82E-04 | 1,11E-02 | 7 | 201 | cell growth |
| 43170 | 1,08E-03 | 1,20E-02 | 69 | 6392 | macromolecule metabolism |
| 8361 | 1,13E-03 | 1,23E-02 | 7 | 206 | regulation of cell size |
| 30163 | 1,17E-03 | 1,24E-02 | 8 | 268 | protein catabolism |
| 50789 | 1,18E-03 | 1,24E-02 | 32 | 2359 | regulation of biological process |
| 50791 | 1,26E-03 | 1,30E-02 | 29 | 2070 | regulation of physiological process |
| 6091 | 1,29E-03 | 1,30E-02 | 8 | 272 | generation of precursor metabolites and energy |
| 51244 | 1,34E-03 | 1,33E-02 | 28 | 1980 | regulation of cellular physiological process |
| 16043 | 1,47E-03 | 1,42E-02 | 22 | 1419 | cell organization and biogenesis |
| 6560 | 1,61 E-03 | 1,42E-02 | 2 | 8 | proline metabolism |
| 6469 | 1,61 E-03 | 1,42E-02 | 2 | 8 | negative regulation of protein kinase activity |
| 45736 | 1,61E-03 | 1,42E-02 | 2 | 8 | negative regulation of cyclin-dependent protein kinase activity |
| 51348 | 1,61 E-03 | 1,42E-02 | 2 | 8 | negative regulation of transferase activity |
| 1558 | 1,61E-03 | 1,42E-02 | 2 | 8 | regulation of cell growth |
| 48856 | 1,64E-03 | 1,42E-02 | 20 | 1248 | anatomical structure development |
| 9725 | 1,65E-03 | 1,42E-02 | 14 | 729 | response to hormone stimulus |
| 44238 | 1,81 E-03 | 1,53E-02 | 85 | 8429 | primary metabolism |
| 8152 | 1,82E-03 | 1,53E-02 | 98 | 10040 | metabolism |
| 9409 | 1,92E-03 | 1,59E-02 | 7 | 226 | response to cold |
| 45786 | 2,06E-03 | 1,64E-02 | 2 | 9 | negative regulation of progression through cell cycle |
| 51302 | 2,06E-03 | 1,64E-02 | 2 | 9 | regulation of cell division |
| 50794 | 2,08E-03 | 1,64E-02 | 28 | 2040 | regulation of cellular process |
| 51170 | 2,27E-03 | 1,76E-02 | 3 | 34 | nuclear import |
| 9790 | 2,47E-03 | 1,89E-02 | 11 | 520 | embryonic development |
| 43086 | 2,56E-03 | 1,93E-02 | 2 | 10 | negative regulation of enzyme activity |
| 6395 | 2,59E-03 | 1,93E-02 | 4 | 74 | RNA splicing |
| 6099 | 3,11E-03 | 2,26E-02 | 2 | 11 | tricarboxylic acid cycle |
| 46356 | 3, 11E-03 | 2,26E-02 | 2 | 11 | acetyl-CoA catabolism |
| 6511 | 3,30E-03 | 2,30E-02 | 7 | 249 | ubiquitin-dependent protein catabolism |
| 43632 | 3,30E-03 | 2,30E-02 | 7 | 249 | modification-dependent macromolecule catabolism |
| 19941 | 3,30E-03 | 2,30E-02 | 7 | 249 | modification-dependent protein catabolism |
| 51603 | 3,45E-03 | 2,37E-02 | 7 | 251 | proteolysis during cellular protein catabolism |
| 6270 | 3,71 E-03 | 2,53E-02 | 2 | 12 | DNA replication initiation |
| 9064 | 3,88E-03 | 2,55E-02 | 3 | 41 | glutamine family amino acid metabolism |
| 9615 | 3,88E-03 | 2,55E-02 | 3 | 41 | response to virus |
| 44257 | 3,92E-03 | 2,55E-02 | 7 | 257 | cellular protein catabolism |
| 48827 | 3,94E-03 | 2,55E-02 | 6 | 193 | phyllome development |
| 51169 | 4,16E-03 | 2,66E-02 | 3 | 42 | nuclear transport |
| 16071 | 4,40E-03 | 2,78E-02 | 5 | 138 | mRNA metabolism |
| 9793 | 4,72E-03 | 2,95E-02 | 10 | 487 | embryonic development (sensu Magnoliophyta) |
| 9109 | 5,07E-03 | 3,13E-02 | 2 | 14 | coenzyme catabolism |
| 6800 | 5,22E-03 | 3,19E-02 | 7 | 271 | oxygen and reactive oxygen species metabolism |
| 398 | 5,38E-03 | 3,25E-02 | 3 | 46 | nuclear mRNA splicing, via spliceosome |
| 48367 | 5,43E-03 | 3,25E-02 | 7 | 273 | shoot development |
| 6096 | 5,71E-03 | 3,38E-02 | 3 | 47 | glycolysis |
| 9060 | 5,82E-03 | 3,40E-02 | 2 | 15 | aerobic respiration |
| 375 | 6,06E-03 | 3,47E-02 | 3 | 48 | RNA splicing, via transesterification reactions |
| 377 | 6,06E-03 | 3,47E-02 | 3 | 48 | RNA splicing, via transesterification reactions with bulged adenosine as nucleophile |
| 9084 | 7,46E-03 | 4,23E-02 | 2 | 17 | glutamine family amino acid biosynthesis |
| 42891 | 7,71E-03 | 4,24E-02 | 1 | 1 | antibiotic transport |
| 7091 | 7,71E-03 | 4,24E-02 | 1 | 1 | mitotic metaphase/anaphase transition |
| 30071 | 7,71E-03 | 4,24E-02 | 1 | 1 | regulation of mitotic metaphase/anaphase transition |
| 48316 | 8,36E-03 | 4,55E-02 | 10 | 530 | seed development |
| 48608 | 8,68E-03 | 4,68E-02 | 10 | 533 | reproductive structure development |
| 19952 | 8,92E-03 | 4,76E-02 | 13 | 794 | reproduction |
| 6732 | 9,45E-03 | 4,99E-02 | 5 | 166 | coenzyme metabolism |

**Table 4: Overview of cell cycle-related features used in the computational analysis to search for new cell cycle proteins.**

| Cell cycle feature | Category | # of genes | References |
|---|---|---|---|
| Periodicity | Gene expression | 1258 | (5, 6) |
| MSA-like | Promotor motif | 2295 | (7) |
| E2Fa-like | Promotor motif | 1809 | (7) |
| E2F10SPCNA | Promotor motif | 2221 | (7) |
| OS_motifslandlla | Promotor motif | 2310 | (7) |
| UP1ATMSD | Promotor motif | 3738 | (7) |
| wrrmGCGn | Promotor motif | 2179 | (7) |
| CDK consensus site | Phosphorylation motif | 6321 | (8) |
| [IM]R-tail | Protein sequence motif | 116 | (16, 17) |
| PEST-sequence | Destruction motif | 2719 | (18) |
| D-box | Destruction motif | 2369 | (19) |
| KEN-box | Destruction motif | 410 | (19) |
| GxEN-box | Destruction motif | 300 | (20) |
| A-box | Destruction motif | 1779 | (21) |

**Table 5: collection of cell cycle genes.**

| | | |
|---|---|---|
| Collection of 518 genes annotated as cell cycle by gene ontology, including all genes described as core cell cycle genes or related to cell cycle (*19*, *22*-*24*). The following GO terms were used to build the collection: cell cycle (GO:0007049), cell division (GO:0051301), cell proliferation (GO:0008283), chromosome segregation (GO:0007059), DNA replication (GO:0006260), DNA replication and chromosome cycle (GO:0000067). For every gene, the number of cell cycle-related features present is shown. This collection was subtracted from the prey lists to find novel cell cycle proteins. | | |

| Accession | Name | # features |
|---|---|---|
| AT2G31270 | CDT1a | 8 |
| AT5G13840 | CCS52B | 7 |
| AT3G16320 | cdc27a | 7 |
| AT1G07270 | cdc6b | 7 |
| AT3G59550 | cohesion family protein SYN3 (SYN3) | 7 |
| AT5G25380 | CYCA2;1 | 7 |
| AT3G11450 | DNAJ HEAT SHOCK N-TERMINAL DOMAIN-CONTAINING | 7 |
| AT1G65470 | chromatin assembly factor-1 (FASCIATA1) (FAS1), identical to FAS1 | 6 |
| AT1G77390 | CYCA1;2 | 6 |
| AT5G43080 | CYCA3;1 | 6 |
| AT3G11520 | CYCB1;3 | 6 |
| AT3G10690 | DNA gyrase subunit A family protein | 6 |
| AT4G25540 | DNA mismatch repair protein MSH3; AtMsh3 | 6 |
| AT1G47870 | E2Fc | 6 |
| AT4G38600 | KAK (KAKTUS) | 6 |
| AT5G46280 | MCM3 | 6 |
| AT5G07280 | receptor-like protein kinase-like protein; receptor-like protein kinase - | 6 |
| AT5G61000 | Replication protein A1; Putative RNA helicase | 6 |
| AT5G42190 | ASK2 (ARABIDOPSIS SKP1-LIKE 2); UBIQUITIN-PROTEIN LIGASE | 5 |
| AT2G29680 | cdc6 | 5 |
| AT1G18670 | CKL3 | 5 |
| AT1G70210 | CYCD1;1 | 5 |
| AT4G37630 | CYCD5;1 | 5 |
| AT5G49160 | DNA (cytosine-5-)-methyltransferase (AtHIM), identical to SP:P34881 | 5 |
| AT1G08130 | DNA ligase / polydeoxyribonucleotide synthase [AtP] identical to | 5 |
| AT5G41880 | DNA polymerase alpha subunit IV (Primase)-like protein | 5 |
| AT3G13170 | DNA TOPOISOMERASE VIA (SPO11-1), IDENTICAL TO ATSPO11- | 5 |
| AT5G67100 | DNA-directed DNA polymerase alpha catalytic subunit, putative, | 5 |
| AT5G02470 | DPa | 5 |
| AT5G22220 | E2Fb | 5 |
| AT3G43210 | kinesin motor family protein (NACK2) | 5 |
| AT1G18800 | NRP2 (NAP1-RELATED PROTEIN 2); DNA BINDING / CHROMATIN | 5 |
| AT2G37560 | ORC2 | 5 |
| AT1G26840 | ORC6 | 5 |
| AT5G16270 | Rad21/Rec8-like family protein weak similarity to cohesion family | 5 |
| AT3G12280 | RBR | 5 |
| AT5G61210 | SNAP33 (SYNAPTOSOMAL-ASSOCIATED PROTEIN 33); T-SNARE | 5 |
| AT5G48600 | structural maintenance of chromosomes (SMC) family protein, similar | 5 |
| AT5G55220 | trigger factor type chaperone family protein, contains Pfam profiles | 5 |
| AT3G60740 | tubulin folding cofactor D | 5 |
| AT3G19770 | vacuolar sorting protein 9 domain-containing protein | 5 |
| AT5G05560 | APC1 | 4 |
| AT1G06590 | APC5 | 4 |
| AT3G28730 | ATHMG (HIGH MOBILITY GROUP, STRUCTURE-SPECIFIC | 4 |
| AT3G53230 | CDC48, putative | 4 |
| AT3G01610 | CDC48C \| AAA-TYPE ATPASE FAMILY PROTEIN, CONTAINS | 4 |
| AT1G18040 | CDKD;3 | 4 |
| AT5G03340 | CELL DIVISION CONTROL PROTEIN 48 HOMOLOG E; ATPASE | 4 |
| AT5G11300 | CYCA2;2 | 4 |
| AT1G15570 | CYCA2;3 | 4 |
| AT1G80370 | CYCA2;4 | 4 |
| AT2G26760 | CYCB1;4 | 4 |
| AT2G17620 | CYCB2;1 | 4 |
| AT4G35620 | CYCB2;2 | 4 |
| AT3G50070 | CYCD3;3 | 4 |
| AT3G05330 | cyclin family low similarity to microtubule-binding protein TANGLED1 | 4 |
| AT5G05490 | DIF1/SYN1 | 4 |
| AT1G69770 | DNA (cytosine-5)-methyltransferase CMt3; Chromomethylase 3; | 4 |
| AT4G02460 | DNA mismatch repair protein | 4 |
| AT1G67320 | DNA primase, large subunit family, contains Pfam profile PF04104: | 4 |
| AT4G31210 | DNA topoisomerase family protein, similar to DNA Topoisomerase I | 4 |
| AT5G63920 | DNA topoisomerase III | 4 |
| AT5G06110 | DNAJ heat shock N-terminal domain-containing protein / cell division | 4 |
| AT1G78650 | expressed protein, weak similarity to DNA polymerase delta subunit 3 | 4 |
| AT2G13330 | F14O4.9 | 4 |
| AT2G23430 | KRP1 | 4 |
| AT2G20980 | MCM10 | 4 |
| AT2G29570 | PCNA2 | 4 |
| AT4G14150 | phragmoplast-associated kinesin-related protein (PAKRP1) | 4 |
| AT3G23670 | phragmoplast-associated kinesin-related protein, putative, similar to | 4 |
| AT5G58720 | PRLI-INTERACTING FACTOR, PUTATIVE | 4 |
| AT1G53710 | PROTEIN SERINE/THREONINE PHOSPHATASE | 4 |
| AT3G02920 | replication protein-related, similar to replication protein A 30kDa | 4 |
| AT4G22970 | similar to hypothetical protein [Arabidopsis thaliana] | 4 |
| AT5G61460 | SMC-like protein | 4 |
| AT1G65660 | SMP1 (SWELLMAP 1); NUCLEIC ACID BINDING | 4 |
| AT5G62000 | transcriptional factor B3 family protein / auxin-responsive factor | 4 |
| AT5G16750 | transducin family protein / WD-40 repeat family protein contains 8 | 4 |
| AT2G14400 | transposable element gene | 4 |
| AT2G42260 | UVI4 | 4 |
| AT1G57820 | VARIANT IN METHYLATION 1 | 4 |
| AT3G02820 | zinc knuckle (CCHC-type) family protein, contains Pfam domain, | 4 |
| AT5G22010 | AAA-TYPE ATPASE FAMILY PROTEIN / BRCT DOMAIN- | 3 |
| AT2G14120 | ADL2b | 3 |
| AT5G57160 | ATLIG4 (ARABIDOPSIS THALIANA DNA LIGASE IV) | 3 |
| AT5G45720 | ATP BINDING / DNA-DIRECTED DNA POLYMERASE/ | 3 |
| AT1G66730 | ATP DEPENDENT DNA LIGASE FAMILY PROTEIN | 3 |
| AT2G32590 | barren family protein, low similarity to SP:Q9Y7R3 Condensin | 3 |
| AT3G19590 | BUB3-like2 | 3 |
| AT4G22910 | CCS52A1 | 3 |
| AT4G11920 | CCS52A2 | 3 |
| AT4G33270 | cdc20-1 | 3 |
| AT3G25100 | CDC45 (Cell division cycle 45)-like protein; Putative cell division cyle | 3 |
| AT3G09840 | CDC48 | 3 |
| AT1G20930 | CDKB2;2 | 3 |
| AT1G73690 | CDKD;1 | 3 |
| AT4G28980 | CDKF;1 | 3 |
| AT2G32900 | centromere/kinetochore protein, putative (ZW10) | 3 |
| AT5G62410 | Chromosome assembly protein homolog | 3 |
| AT1G44110 | CYCA1;1 | 3 |
| AT1G47210 | CYCA3;2 | 3 |
| AT4G37490 | CYCB1;1 | 3 |
| AT1G76310 | CYCB2;4 | 3 |
| AT4G34160 | CYCD3;1 | 3 |
| AT4G34090 | CYL1 | 3 |
| AT3G20550 | DDL (DAWDLE) | 3 |
| AT5G14960 | DEL2 | 3 |
| AT4G09140 | DNA mismatch repair protein MLH1 | 3 |
| AT3G18524 | DNA mismatch repair protein MSH2 (MSH2) identical to SP\|O24617 | 3 |
| AT4G02070 | DNA mismatch repair protein MSH6-1 (MSH6-1) (AGAA.3) | 3 |
| AT5G55300 | DNA topoisomerase I | 3 |
| AT3G17830 | DNAJ HEAT SHOCK FAMILY PROTEIN | 3 |
| AT1G80030 | DNAJ HEAT SHOCK PROTEIN, PUTATIVE | 3 |
| AT1G08840 | EMB2411 \| DNA REPLICATION HELICASE, PUTATIVE, SIMILAR | 3 |
| AT4G37120 | expressed protein | 3 |
| AT5G58870 | FTSH9 (FTSH PROTEASE 9); ATP-DEPENDENT PEPTIDASE/ | 3 |
| AT1G13980 | GN (GNOM) | 3 |
| AT5G24330 | Histone-lysine N-methyltransferase AtXR6; Protein SEt DOMAIN | 3 |
| AT5G06830 | IDENTICAL TO CDK5RAP3-LIKE PROTEIN [ARABIDOPSIS | 3 |
| AT1G49620 | KRP7 | 3 |
| AT1G71830 | leucine-rich repeat family protein / protein kinase family protein, | 3 |
| AT1G80080 | leucine-rich repeat family protein, contains leucine rich-repeat | 3 |
| AT3G25980 | MAD2-like | 3 |
| AT5G51600 | MAP65-3 | 3 |
| AT1G44900 | MCM2 | 3 |
| AT2G16440 | MCM4 | 3 |
| AT2G07690 | MCM5 | 3 |
| AT5G54260 | MRE11 \| DNA REPAIR AND MEIOSIS PROTEIN (MRE11), | 3 |
| AT5G58230 | MSI1 (MULTICOPY SUPRESSOR OF IRA1) | 3 |
| AT1G61000 | Nuf2 family protein contains Pfam PF03800: Nuf2 family | 3 |
| AT5G 16690 | ORC3 | 3 |
| AT1G72150 | PATL1 (PATELLIN 1); TRANSPORTER | 3 |
| AT1G50840 | poll-like DNA polymerase, putative, similar to Poll-like DNA | 3 |
| AT1G80190 | PSF1 (GINS complex DNA replication) | 3 |
| AT2G45280 | putative RAD51C-like DNA repair protein; supported by cDNA: | 3 |
| AT2G31970 | RAD50 DNA REPAIR-RECOMBINATION PROTEIN (RAD50), | 3 |
| AT5G08020 | Replication factor A-like protein | 3 |
| AT2G21790 | Ribonucleoside-diphosphate reductase large subunit; AtRNR1; | 3 |
| AT3G27060 | ribonucleoside-diphosphate reductase small chain, putative / | 3 |
| AT2G24490 | RPA2 | 3 |
| AT4G 18590 | RPA3 | 3 |
| AT1G30690 | SEC14 cytosolic factor family protein / phosphoglyceride transfer | 3 |
| AT1G74560 | similar to nucleosome assembly protein (NAP) family protein | 3 |
| AT5G04320 | similar to unknown protein [Arabidopsis thaliana] | 3 |
| AT5G49010 | SLD5 (DNA replication) | 3 |
| AT5G51330 | SWI1 (SWITCH1); PHOSPHOLIPASE C | 3 |
| AT4G02110 | TOPBP1 | 3 |
| AT2G01550 | transposable element gene | 3 |
| AT2G15410 | transposable element gene | 3 |
| AT4G33790 | ACYL COA REDUCTASE, PUTATIVE | 2 |
| AT5G22420 | ACYL COA REDUCTASE, PUTATIVE | 2 |
| AT4G16420 | ADA2B (PROPORZ1); DNA BINDING /TRANSCRIPTION FACTOR | 2 |
| AT1G78580 | alpha, alpha-trehalose-phosphate synthase, UDP-forming, putative | 2 |
| AT1G54960 | ANP2 (ARABIDOPSIS NPK1-RELATED PROTEIN KINASE 2); | 2 |
| AT4G37750 | ANT | 2 |
| AT1G78770 | APC6 | 2 |
| AT2G39090 | APC7 | 2 |
| AT3G48150 | APC8 | 2 |
| AT4G30080 | ARF16 (AUXIN RESPONSE FACTOR 16); MIRNA BINDING / | 2 |
| AT1G50240 | armadillo/beta-catenin repeat family protein, contains Pfam profile: | 2 |
| AT1G67370 | ASY1 (ASYNAPTIC 1); DNA BINDING | 2 |
| AT3G12400 | ATELC/ELC; ubiquitin binding | 2 |
| AT3G52115 | ATGR1 (GAMMA RESPONSE 1) | 2 |
| AT5G55230 | ATMAP65-1 (MICROTUBULE-ASSOCIATED PROTEINS 65-1); | 2 |
| AT3G27730 | ATP-dependent DNA helicase, putative | 2 |
| AT5G40820 | ATR | 2 |
| AT5G16850 | ATTERT (TELOMERASE REVERSE TRANSCRIPTASE); | 2 |
| AT4G00020 | BRCA2A (BREAST CANCER 2 LIKE 2A, EMBRYO SAC | 2 |
| AT5G01630 | BRCA2B_ATBRCA2(V)_BRCA2(V)_BRCA2B (breast cancer 2 like | 2 |
| AT2G13680 | CALS5 (CALLOSE SYNTHASE 5); 1,3-BETA-GLUCAN SYNTHASE | 2 |
| AT2G20000 | cdc27b | 2 |
| AT2G03670 | CDC48B; ATPASE | 2 |
| AT1G76540 | CDKB2;1 | 2 |
| AT5G64960 | CDKC;2 | 2 |
| AT5G63370 | CDKG;1 | 2 |
| AT3G02450 | CELL DIVISION PROTEIN FTSH, PUTATIVE | 2 |
| AT5G55280 | cell division protein FtsZ, chloroplast, putative | 2 |
| AT5G37630 | chromosome condensation family protein, contains pfam profile: | 2 |
| AT1G09600 | CKL11 | 2 |
| AT4G22940 | CKL4 | 2 |
| AT5G44290 | CKL5 | 2 |
| AT1G03740 | CKL6 | 2 |
| AT1G54610 | CKL9 | 2 |
| AT2G27970 | CKS2 | 2 |
| AT5G40840 | cohesion family protein SYN2 (SYN2) | 2 |
| AT4G02570 | cullin family protein NCBI Chain C, Crystal Structure Of the Ddb1- | 2 |
| AT1G26830 | cullin3a | 2 |
| AT5G06150 | CYCB1;2 | 2 |
| AT1G34460 | CYCB1;5 | 2 |
| AT5G48640 | CYCC1;1 | 2 |
| AT2G26430 | CYCL1 | 2 |
| AT4G19560 | CYCT1;2 | 2 |
| AT5G45190 | CYCT1;5 | 2 |
| AT3G01330 | DEL3 | 2 |
| AT4G35520 | DNA mismatch repair family protein | 2 |
| AT1G65070 | DNA MISMATCH REPAIR MUTS FAMILY PROTEIN | 2 |
| AT1G67630 | DNA polymerase alpha subunit B family, contains Pfam profile: | 2 |
| AT1G09815 | DNA polymerase delta subunit 4 family, contains similarity to Swiss- | 2 |
| AT5G64420 | DNA polymerase V family, contains Pfam domain PF04931: DNA | 2 |
| AT1G14460 | DNA polymerase-related, weak similarity to DNA polymerase III | 2 |
| AT5G55310 | DNA topoisomerase I, putative | 2 |
| AT5G44610 | DREPP PLASMA MEMBRANE POLYPEPTIDE-RELATED | 2 |
| AT2G36010 | E2Fa | 2 |
| AT3G11220 | ELP4 | 2 |
| AT4G32700 | Encodes a homolog of Drosophila MUS308 and mammalian DNA | 2 |
| AT5G64630 | Encodes the second largest subunit of the chromatin assembly factor- | 2 |
| AT4G24790 | expressed protein, ; expression supported by MPSS | 2 |
| AT3G24495 | Expressed protein; GC donor splice site at exon 11; supported by | 2 |
| AT4G23940 | FTSH PROTEASE, PUTATIVE | 2 |
| AT5G01230 | FtsJ-like methyltransferase family protein, contains Pfam profile: | 2 |
| AT1G48270 | GCR1 (G-PROTEIN-COUPLED RECEPTOR 1) | 2 |
| AT1G10270 | GRP23 (GLUTAMINE-RICH PROTEIN23); BINDING | 2 |
| AT2G26890 | GRV2 (KATAMARI2); binding / heat shock protein binding | 2 |
| AT4G32880 | homeobox-leucine zipper transcription factor (HB-8), identical to HD- | 2 |
| AT5G46920 | intron maturase, type II family protein | 2 |
| AT5G54670 | kinesin-like protein C (KATC) | 2 |
| AT3G 19150 | KRP6 | 2 |
| AT5G44635 | MCM6 | 2 |
| AT4G02060 | MCM7 | 2 |
| AT3G09660 | MCM8 | 2 |
| AT2G14050 | MCM9 | 2 |
| AT1G77320 | ME11 (MEIOSIS DEFECTIVE 1); TRANSCRIPTION COACTIVATOR | 2 |
| AT3G22880 | meiotic recombination protein, putative | 2 |
| AT5G50110 | METHYLTRANSFERASE-RELATED | 2 |
| AT5G24020 | MIND (ACCUMULATION AND REPLICATION OF CHLOROPLAST | 2 |
| AT3G24320 | mismatch binding protein, putative; similar to mismatch binding protein | 2 |
| AT5G49880 | MITOTIC CHECKPOINT FAMILY PROTEIN | 2 |
| AT2G35630 | MOR1 \| MICROTUBULE ORGANIZATION 1 PROTEIN (MOR1), | 2 |
| AT4G17380 | MSH4, similar to DNA mismatch repair protein MSH2 | 2 |
| AT1G63680 | Mur ligase family protein, contains Pfam profile: PF01225 Mur ligase | 2 |
| AT5G11510 | MYB 3R4 | 2 |
| AT3G02680 | NBS1 (NIJMEGEN BREAKAGE SYNDROME 1) | 2 |
| AT3G57060 | non-SMC condensin subunit, XCAP-D2/Cnd1 family protein, similar to | 2 |
| AT4G14700 | ORC1A | 2 |
| AT4G29910 | ORC5 | 2 |
| AT4G30825 | pentatricopeptide (PPR) repeat-containing protein, contains Pfam | 2 |
| AT5G14520 | pescadillo-related | 2 |
| AT2G13840 | PHP DOMAIN-CONTAINING PROTEIN | 2 |
| AT3G63120 | PLP1;1 | 2 |
| AT1G50230 | protein kinase family protein, contains protein kinase domain, | 2 |
| AT3G63130 | RAN GTPase activating protein 1 (RanGAP1), contains Pfam | 2 |
| AT1G16590 | REV7 (REVERSIONLESS 7); DNA BINDING | 2 |
| AT5G27740 | RFC3 | 2 |
| AT1 G21690 | RFC4 | 2 |
| AT1G77470 | RFC5 | 2 |
| AT1G48380 | RHL1 (ROOT HAIRLESS 1) | 2 |
| AT4G33495 | RPD1 (ROOT PRIMORDIUM DEFECTIVE 1) | 2 |
| AT5G35770 | SAP (STERILE APETALA); TRANSCRIPTION FACTOR | 2 |
| AT1G72160 | SEC14 CYTOSOLIC FACTOR FAMILY PROTEIN / | 2 |
| AT4G09160 | SEC14 CYTOSOLIC FACTOR FAMILY PROTEIN / | 2 |
| AT3G51670 | SEC14 cytosolic factor family protein / phosphoglyceride transfer | 2 |
| AT5G03730 | SERINE/THREONINE-PROTEIN KINASE CTR1 | 2 |
| AT4G14180 | SIMILAR TO OS04G0347800 [ORYZA SATIVA (JAPONICA | 2 |
| AT1G08260 | SIMILAR TO POL2A. DNA POLYMERASE EPSILON CATALYTIC | 2 |
| AT2G27170 | similar to SMC2-like condensin, putative (SMC2) (TITAN3) | 2 |
| AT5G24630 | SIMILAR TO UNKNOWN PROTEIN [ARABIDOPSIS THALIANA] | 2 |
| AT3G10440 | SIMILAR TO UNKNOWN PROTEIN [ARABIDOPSIS THALIANA] | 2 |
| AT3G17590 | SNF5 homolog BSH (bsh) mRNA, complete cds | 2 |
| AT1G49040 | stomatal cytokinesis defective/ SCD1 protein (SCD1) | 2 |
| AT5G15920 | structural maintenance of chromosomes (SMC) family protein (MSS2), | 2 |
| AT1G04110 | subtilase family protein, contains similarity to subtilisin-like protease | 2 |
| AT3G04740 | SWP (STRUWWELPETER) | 2 |
| AT1G08560 | syntaxin-related protein KNOLLE | 2 |
| AT3G62980 | TIR1 (TRANSPORT INHIBITOR RESPONSE 1); ubiquitin-protein | 2 |
| AT1G30660 | toprim domain-containing protein | 2 |
| AT1G30680 | TOPRIM DOMAIN-CONTAINING PROTEIN | 2 |
| AT4G10710 | transcriptional regulator-related | 2 |
| AT3G44540 | TRANSDUCIN FAMILY PROTEIN / WD-40 REPEAT FAMILY | 2 |
| AT2G15380 | transposable element gene | 2 |
| AT2G15540 | transposable element gene | 2 |
| AT2G18820 | transposable element gene | 2 |
| AT2G28980 | transposable element gene | 2 |
| AT4G04000 | transposable element gene | 2 |
| AT3G54670 | TTN8 \| SIMILAR TO STRUCTURAL MAINTENANCE OF | 2 |
| AT3G20060 | UBC19 (UBIQUITIN-CONJUGATING ENZYME 19); UBIQUITIN- | 2 |
| AT1G50490 | UBC20 | 2 |
| AT1G02970 | WEE1 | 2 |
| AT5G08290 | YLS8 (yellow-leaf-specific gene 8); catalytic | 2 |
| AT4G02980 | ABP1 (ENDOPLASMIC RETICULUM AUXIN BINDING PROTEIN 1) | 1 |
| AT5G22500 | ACYL COA REDUCTASE, PUTATIVE / MALE-STERILITY PROTEIN, | 1 |
| AT1G14830 | ADL1C/ADL5 (DYNAMIN-LIKE PROTEIN 5); GTP binding / GTPase | 1 |
| AT4G33650 | ADL2a | 1 |
| AT5G61960 | AML1 (ARABIDOPSIS MEI2-LIKE PROTEIN 1); RNA binding | 1 |
| AT4G01540 | ANAC068/NTM1 (NAC WITH TRANSMEMBRANE MOTIF1); | 1 |
| AT2G18290 | APC10 | 1 |
| AT2G04660 | APC2 | 1 |
| AT4G21530 | APC4 | 1 |
| AT3G54710 | ATCDT1B/CDT1/CDT1B (ARABIDOPSIS HOMOLOG OF YEAST | 1 |
| AT2G20190 | ATCLASP/CLASP; binding | 1 |
| AT4G18820 | ATP BINDING / DNA-DIRECTED DNA POLYMERASE/ | 1 |
| AT4G21800 | ATP-binding family protein | 1 |
| AT3G49780 | ATPSK3 \| PHYTOSULFOKINES 3 (PSK3), IDENTICAL TO | 1 |
| AT1G58470 | ATRBP1 (ARABIDOPSIS THALIANA RNA-BINDING PROTEIN 1); | 1 |
| AT4G00100 | ATRPS13A (RIBOSOMAL PROTEIN S13A); structural constituent of | 1 |
| AT3G20780 | ATTOP6B (BRASSINOSTEROID INSENSITIVE 3, ROOT HAIRLESS | 1 |
| AT2G28350 | auxin-responsive factor (ARF10) | 1 |
| AT1G69400 | BUB3-likel | 1 |
| AT5G27080 | cdc20-3 | 1 |
| AT5G03455 | cdc25 | 1 |
| AT2G38620 | CDKB1;2 | 1 |
| AT5G10270 | CDKC;1 | 1 |
| AT1G66750 | CDKD;2 | 1 |
| AT5G63610 | CDKE;1 | 1 |
| AT1G67580 | CDKG;2 | 1 |
| AT2G46520 | CELLULAR APOPTOSIS SUSCEPTIBILITY PROTEIN, PUTATIVE / | 1 |
| AT2G25170 | chromatin remodeling factor CHD3 (PICKLE) | 1 |
| AT2G26280 | CID7; ATP BINDING / DAMAGED DNA BINDING | 1 |
| AT5G39420 | CKL1 | 1 |
| AT1G57700 | CKL10 | 1 |
| AT1G71530 | CKL12 | 1 |
| AT4G10010 | CKL13 | 1 |
| AT1G33770 | CKL14 | 1 |
| AT1G74330 | CKL2 | 1 |
| AT5G50860 | CKL7 | 1 |
| AT2G27960 | CKS1 | 1 |
| AT1G02980 | cullin family protein, similar to cullin 1 (Homo sapiens) Gl:3139077; | 1 |
| AT1G69670 | cullin3b | 1 |
| AT5G46210 | cullin4 | 1 |
| AT1G47220 | CYCA3;3 | 1 |
| AT1G47230 | CYCA3;4 | 1 |
| AT1G20590 | CYCB2;5 | 1 |
| AT1G16330 | CYCB3;1 | 1 |
| AT2G22490 | CYCD2;1 | 1 |
| AT5G67260 | CYCD3;2 | 1 |
| AT5G65420 | CYCD4;1 | 1 |
| AT4G03270 | CYCD6;1 | 1 |
| AT5G27620 | CYCH;1 | 1 |
| AT3G21870 | CYCLIN FAMILY PROTEIN, SIMILAR TO CYCLIN 2 | 1 |
| AT5G50375 | cyclopropyl isomerase (CPI1) | 1 |
| AT2G45080 | CYCP3;1 (CYCLIN P3;1); CYCLIN-DEPENDENT PROTEIN KINASE | 1 |
| AT3G60550 | CYCP3;2 | 1 |
| AT5G61650 | CYCP4;2 \| CYCLIN FAMILY PROTEIN, SIMILAR TO CYCLIN 2 | 1 |
| AT1G35440 | CYCT1;1 | 1 |
| AT1G27630 | CYCT1;3 | 1 |
| AT4G19600 | CYCT1;4 | 1 |
| AT1G01040 | DEAD/DEAH box helicase carpel factory / CAF | 1 |
| AT3G48160 | DEL1 | 1 |
| AT5G54090 | DNA mismatch repair MutS family protein | 1 |
| AT2G42120 | DNA polymerase delta small subunit | 1 |
| AT1G10520 | DNA polymerase lambda (POLL) | 1 |
| AT2G32000 | DNA TOPOISOMERASE FAMILY PROTEIN | 1 |
| AT5G63960 | DNA-directed DNA polymerase delta catalytic subunit, putative | 1 |
| AT2G27120 | DNA-directed DNA polymerase epsilon catalytic subunit, putative, | 1 |
| AT2G22360 | DNAJ heat shock family protein | 1 |
| AT3G60190 | dynamin-like protein E | 1 |
| AT5G13680 | ELO2 | 1 |
| AT1G12360 | ENCODES A SEC1 PROTEIN AND EXPRESSED THROUGHOUT | 1 |
| AT3G23580 | ENCODES ONE OF THE 3 RIBONUCLEOTIDE REDUCTASE (RNR) | 1 |
| AT1G50250 | FTSH1 (FTSH PROTEASE 1); ATP-DEPENDENT PEPTIDASE/ | 1 |
| AT5G53170 | FTSH11 (FTSH PROTEASE 11); ATP-DEPENDENT PEPTIDASE/ | 1 |
| AT2G36250 | FTSZ2-1 (FTSZ HOMOLOG 2-1); STRUCTURAL MOLECULE | 1 |
| AT3G52750 | FTSZ2-2 (FtsZ2-2); structural molecule | 1 |
| AT2G22475 | GRAM DOMAIN-CONTAINING PROTEIN / ABA-RESPONSIVE | 1 |
| ATMG01250 | HYPOTHETICAL PROTEIN | 1 |
| AT5G08550 | ILP1 (INCREASED LEVEL OF POLYPLOIDY1-1D); TRANSLATION | 1 |
| AT1G30010 | INTRON MATURASE, TYPE II FAMILY PROTEIN | 1 |
| AT4G21270 | kinesin-like protein A (KATA) | 1 |
| AT5G35520 | kinetochore protein-related | 1 |
| AT3G50630 | KRP2 | 1 |
| AT5G48820 | KRP3 | 1 |
| AT2G32710 | KRP4 | 1 |
| AT3G24810 | KRP5 | 1 |
| AT2G26330 | leucine-rich repeat protein kinase, putative (ERECtA), identical to | 1 |
| AT3G56700 | MALE STERILITY PROTEIN, PUTATIVE | 1 |
| AT4G30820 | MAT1 | 1 |
| AT1G66170 | MMD1 (MALE MEIOCYTE DEATH 1); DNA BINDING | 1 |
| AT4G20900 | MS5 (MALE-STERILE 5) | 1 |
| AT1G79150 | NOC3_DNA replication initiation | 1 |
| AT2G01840 | non-LTR retrotransposon family (LINE), has a 9.6e-34 P-value blast | 1 |
| AT2G35190 | NPSN11 (NOVEL PLANT SNARE 11); protein transporter | 1 |
| AT1G77620 | NUCLEOSIDE-TRIPHOSPHATASE/ NUCLEOTIDE BINDING | 1 |
| AT4G12620 | ORC1 | 1 |
| AT3G44550 | OXIDOREDUCTASE, ACTING ON THE CH-CH GROUP OF | 1 |
| AT3G44560 | OXIDOREDUCTASE, ACTING ON THE CH-CH GROUP OF | 1 |
| AT5G10480 | PAS2 (PASTICCINO 2) | 1 |
| AT1G07370 | PCNA1 | 1 |
| AT1G71440 | PFI_TFCE_PFI (PFIFFERLING) | 1 |
| AT2G18040 | PIN1 | 1 |
| AT5G22130 | PNT1 (PEANUT 1); MANNOSYLTRANSFERASE/ TRANSFERASE, | 1 |
| AT3G20540 | POLGAMMA1 (POLYMERASE GAMMA 1); DNA BINDING / DNA- | 1 |
| AT4G14713 | PPD1 (PEAPOD 1) | 1 |
| AT1G49180 | protein kinase family protein | 1 |
| AT3G12530 | PSF2 (GINS complex DNA replication) | 1 |
| AT2G17910 | putative non-LTR retroelement reverse transcriptase | 1 |
| AT4G36390 | radical SAM domain-containing protein / TRAM domain-containing | 1 |
| AT1G63160 | replication factor, putative; similar to Gl:4972952 from (Mus musculus) | 1 |
| AT5G45400 | replication protein, putative, similar to replication protein A 70kDa | 1 |
| AT4G19130 | replication protein-related, similar to replication protein A 70kDa | 1 |
| AT4G20520 | RNA binding / RNA-directed DNA polymerase | 1 |
| AT2G20580 | RPN1 | 1 |
| AT3G54220 | SCR (SCARECROW); TRANSCRIPTION FACTOR | 1 |
| AT1G14750 | SDS | 1 |
| AT1 G22530 | SEC14 cytosolic factor family protein | 1 |
| AT1G34210 | SERK2 (SOMATIC EMBRYOGENESIS RECEPTOR-LIKE KINASE | 1 |
| AT5G52800 | SIMILAR TO PREDICTED: HYPOTHETICAL PROTEIN [RATTUS | 1 |
| AT5G58650 | SIMILAR TO UNKNOWN PROTEIN [ARABIDOPSIS THALIANA] | 1 |
| AT1G75950 | SKP1 (ARABIDOPSIS SKP1 HOMOLOGUE); ubiquitin-protein ligase | 1 |
| AT3G47460 | SMC2-like condensin, putative/chromosome assembly protein | 1 |
| AT2G02480 | STI (STICHEL); ATP BINDING / DNA-DIRECTED DNA | 1 |
| AT5G07660 | structural maintenance of chromosomes (SMC) family protein | 1 |
| AT3G51770 | tetratricopeptide repeat (TPR)-containing protein | 1 |
| AT5G23070 | thymidine kinase, putative, similar to thymidine kinase (Oryza sativa) | 1 |
| AT3G01780 | TPLATE | 1 |
| AT4G07600 | transposable element gene | 1 |
| AT4G09710 | transposable element gene | 1 |
| AT4G14470 | transposable element gene | 1 |
| AT4G04230 | transposable element gene | 1 |
| AT3G18730 | TSK (TONSOKU) | 1 |
| AT1G22275 | unknown protein | 1 |
| AT5G42270 | VAR1 (VARIEGATED 1); ATP-DEPENDENT PEPTIDASE/ ATPASE/ | 1 |
| AT2G33880 | WOX9 (STIMPY); transcription factor | 1 |
| AT1G24490 | ALB4 (ALBINA 4) | 0 |
| AT5G28640 | AN3 | 0 |
| AT3G05870 | APC11 | 0 |
| AT2G37630 | AS1/ATMYB91/ATPHAN/MYB91 (ASYMMETRIC LEAVES 1, MYB | 0 |
| AT3G21850 | ASK9 (ARABIDOPSIS SKPL-LIKE 9); ubiquitin-protein ligase | 0 |
| AT4G32200 | ASY2; DNA binding | 0 |
| AT3G33520 | ATARP6; structural constituent of cytoskeleton | 0 |
| AT3G48190 | ataxia-telangiectasia mutated protein AtATM | 0 |
| AT5G05620 | ATGCP2/TUBG2 (GAMMA-TUBULIN); structural molecule | 0 |
| AT3G20475 | ATP binding / damaged DNA binding | 0 |
| AT1G49250 | ATP dependent DNA ligase family protein | 0 |
| AT1G13590 | ATPSK1 (PHYTOSULFOKINE 1 PRECURSOR); growth factor | 0 |
| AT2G22860 | ATPSK2_ATPSK2 (PHYTOSULFOKINE 2 PRECURSOR); growth | 0 |
| AT5G65870 | ATPSK5 (PHYTOSULFOKINE 5 PRECURSOR); growth factor | 0 |
| AT4G37720 | ATPSK6 (PHYTOSULFOKINE 6 PRECURSOR); growth factor | 0 |
| AT1G14410 | ATWHY1/PTAC1 (A. THALIANA WHIRLY 1); DNA binding / telomeric | 0 |
| AT5G09790 | ATXR5 (SETDOMAIN GROUP 15); DNA binding | 0 |
| AT3G24730 | catalytic | 0 |
| AT3G48750 | CDKA;1 | 0 |
| AT3G54180 | CDKB1;1 | 0 |
| AT5G66130 | cell cycle checkpoint protein-related, weak similarity to cell cycle | 0 |
| Q94G54 | Cell division control protein 6 | 0 |
| Q9C8M6 | Cell division control protein, putative; 15914-18846 | 0 |
| Q9LFB3 | Cell division-like protein | 0 |
| AT1G53050 | CKL15 | 0 |
| AT3G05050 | CKL8 | 0 |
| AT3G15170 | CUC1 (CUP-SHAPED COTYLEDON1); transcription factor | 0 |
| AT1G20610 | CYCB2;3 | 0 |
| AT5G48630 | CYCC1;2 | 0 |
| AT5G10440 | CYCD4;2 | 0 |
| AT5G02110 | CYCD7;1 | 0 |
| AT2G01905 | CYCJ18 | 0 |
| Q38818 | Cyclin 2 | 0 |
| AT2G44740 | CYCLIN FAMILY PROTEIN, SIMILAR TO CYCLIN 2 | 0 |
| AT5G07450 | CYCLIN FAMILY PROTEIN, SIMILAR TO CYCLIN 2 | 0 |
| AT1G22840 | cytochrome c, putative | 0 |
| AT4G26701 | DNA binding / DNA topoisomerase type I | 0 |
| AT3G15960 | DNA mismatch repair MutS family protein | 0 |
| AT5G22110 | DNA POLYMERASE EPSILON SUBUNIT B FAMILY, CONTAINS | 0 |
| AT5G64520 | DNA repair protein-related | 0 |
| AT1G63990 | DNA topoisomerase VIA, putative (SPO11-2) | 0 |
| AT4G39960 | DNAJ heat shock family protein | 0 |
| AT5G62450 | DOMINO1 | 0 |
| AT5G03415 | DPb | 0 |
| AT5G50320 | ELO3 | 0 |
| Q9FMR4 | Emb\|CAB92117.1 | 0 |
| AT1G24590 | ENCODES A MEMBER OF THE ERF (ETHYLENE RESPONSE | 0 |
| AT5G26680 | ENDONUCLEASE, PUTATIVE, SIMILAR TO SWISS-PROT:P39748 | 0 |
| AT1G22260 | expressed protein | 0 |
| Q9M874 | F16B3.31 protein | 0 |
| Q9FX75 | F19K19.10 protein | 0 |
| Q9FYF5 | F1N21.20 | 0 |
| 081303 | F6N15.14 protein (Putative BRCA2 homolog) | 0 |
| AT2G14650 | F9D12.11 protein (contains similarity to reverse transcriptases) | 0 |
| AT3G24140 | FMA (FAMA); DNA binding / transcription activator/ transcription factor | 0 |
| AT2G22942 | growth factor | 0 |
| AT5G42080 | GTP-binding protein / phragmoplastin, putative | 0 |
| AT1G47840 | hexokinase, putative | 0 |
| AT5G03410 | histidyl-tRNA synthetase - like protein | 0 |
| AT2G44950 | HUB1 | 0 |
| AT2G44960 | HUB1 | 0 |
| AT3G52630 | hypothetical protein | 0 |
| AT1G74350 | intron maturase, type II family protein | 0 |
| AT5G03150 | JKD (JACKDAW); nucleic acid binding / protein binding / protein | 0 |
| Q2V0Z5 | Kinetochore protein | 0 |
| AT2G30410 | KIS (KIESEL); unfolded protein binding | 0 |
| ATMG00520 | MATR | 0 |
| AT5G22260 | MS1 (MALE STERILITY 1); DNA binding | 0 |
| AT3G11980 | MS2 (MALE STERILITY 2) | 0 |
| AT2G01120 | ORC4 | 0 |
| AT4G27650 | PEL1 (PELOTA); translation release factor | 0 |
| AT2G01500 | PFS2 (PRETTY FEW SEEDS 2); transcription factor | 0 |
| AT3G44735 | PSK1 (PHYTOSULFOKINE 3 PRECURSOR); growth factor | 0 |
| Q9M8S3 | Putative CCHC-type zinc finger protein | 0 |
| AT4G39920 | putative protein | 0 |
| AT5G22370 | putative protein; similar to unknown protein (emb\|CAB92117.1) | 0 |
| AT2G07660 | putative retroelement pol polyprotein | 0 |
| 080896 | Putative uncharacterized protein At2g32590 | 0 |
| Q9M013 | Putative uncharacterized protein F7A7_150 | 0 |
| 082745 | Putative uncharacterized protein F7H19.150 (Putative | 0 |
| Q9C953 | Putative uncharacterized protein T7P1.14 | 0 |
| AT4G20050 | QRT3 (QUARTET 3) | 0 |
| AT2G06510 | replication protein, putative similar to replication protein A 70kDa | 0 |
| AT4G29090 | reverse transcriptase, putative / RNA-dependent DNA polymerase, | 0 |
| AT2G25100 | ribonuclease HII family protein | 0 |
| AT5G40942 | RNR2B | 0 |
| AT2G36985 | ROT4 (ROTUNDIFOLIA4) | 0 |
| AT4G18730 | RPL16B (ribosomal protein L16B); structural constituent of ribosome | 0 |
| AT1G17235 | RTFL11 (ROTUNDIFOLIA LIKE 11) | 0 |
| AT4G37650 | SHR (SHORT ROOT); transcription factor | 0 |
| AT5G04470 | SIM | 0 |
| AT3G55490 | similar to TTN10 (TITAN 10) | 0 |
| AT5G62440 | similar to unnamed protein product [Vitis vinifera] (GB:CA064547.1) | 0 |
| Q9FGA0 | Similarity to glucose inhibited division protein B | 0 |
| AT1G20330 | SMT2 (STEROL METHYLTRANSFERASE 2) | 0 |
| AT4G27330 | SPL (SPOROCYTELESS) | 0 |
| AT1G02065 | SPL8 (SQUAMOSA PROMOTER BINDING PROTEIN-LIKE 8); DNA | 0 |
| AT3G07800 | thymidine kinase, putative, similar to thymidine kinase (Oryza sativa) | 0 |
| AT2G05610 | transposable element gene | 0 |
| AT4G08830 | transposable element gene | 0 |
| AT4G10580 | transposable element gene | 0 |
| Q9FLP1 | Trigger factor-like protein | 0 |
| AT1G19080 | TTN10 (TITAN 10) | 0 |
| AT3G61650 | TUBG1 (GAMMA-TUBULIN); STRUCTURAL MOLECULE | 0 |
| AT1G05560 | UGT1 (UDP-glucosyl transferase 75B1); UDP-glycosyltransferase/ | 0 |
| AT1G47200 | WPP2 (WPP domain protein 2) | 0 |
| AT5G57450 | XRCC3 \| DNA REPAIR FAMILY PROTEIN, CONTAINS SIMILARITY | 0 |

**Table 6: New candidate cell cycle proteins.**

| | | |
|---|---|---|
| Overview of 40 putative new cell cycle proteins identified in the core dataset containing at least 2 of the cell cycle-related features listed in Table S4. The number of identified features per protein is shown. | | |

| Locus | Description | # of |
|---|---|---|
| AT2G44580 | DCC1 | 6 |
| AT3G57860 | UVI4-like | 5 |
| AT4G38900 | bZIP protein | 5 |
| AT1G32310 | F27G20.14 | 5 |
| AT4G17020 | transcription factor-related | 4 |
| AT5G09900 | EMB2107, RPN5A, MSA | 4 |
| AT5G40460 | expressed | 4 |
| AT3G49240 | EMB1796 | 4 |
| AT2G17350 | similar to hypothetical protein [Vitis vinifera] (GB:CAN62847.1) | 4 |
| AT5G24690 | similar to RER1 (RETICULATA-RELATED 1) [Arabidopsis thaliana] | 4 |
| AT4G25550 | M7J2_80 | 4 |
| AT3G15180 | proteasome-related | 3 |
| AT2G36130 | peptidyl-prolyl cis-trans isomerase, putative / cyclophilin, putative / rotamase | 3 |
| AT3G16060 | kinesin motor family protein | 3 |
| AT3G10180 | kinesin motor protein-related | 3 |
| AT5G65460 | KCA2 | 3 |
| AT5G05780 | RPN8A, AE3, ATHMOV34 | 3 |
| AT1G06070 | bZIP transcription factor, putative (bZIP69) | 3 |
| AT1G71380 | ATGH9B3, ATCEL3 | 3 |
| AT3G05530 | ATS6A.2, RPT5A | 2 |
| AT1G01880 | DNA repair protein, putative | 2 |
| AT4G34150 | C2 domain-containing protein | 2 |
| AT5G13030 | similar to hypothetical protein OsI_021963 [Oryza sativa ] (GB:EAZ00731.1) | 2 |
| AT4G14310 | DL3195C | 2 |
| AT5G26360 | chaperonin, putative | 2 |
| AT5G49510 | VHL binding protein, putative / prefoldin, putative | 2 |
| AT3G20050 | ATTCP-1 | 2 |
| AT1G29150 | RPN6, ATS9 | 2 |
| AT3G15970 | Ran-binding protein 1 domain-containing protein / RanBP1 domain-containing | 2 |
| AT4G28230 | similar to unnamed protein product [Vitis vinifera] (GB:CA041238.1) | 2 |
| AT5G23540 | 26S proteasome regulatory subunit, putative | 2 |
| AT5G41190 | similar to hypothetical protein [Vitis vinifera] (GB:CAN66411.1) | 2 |
| AT2G03820 | nonsense-mediated mRNA decay NMD3 family protein | 2 |
| AT1G20480 | 4-coumarate--CoA ligase family protein / 4-coumaroyl-CoA synthase family | 2 |
| AT1G09270 | importin alpha-1 subunit, putative (IMPA4) | 2 |
| AT1G23190 | phosphoglucomutase, cytoplasmic, putative / glucose phosphomutase, | 2 |
| AT2G06990 | HEN2 | 2 |
| AT5G58290 | RPT3 | 2 |
| AT5G20920 | EMB1401, EIF2 BETA | 2 |
| AT1G20200 | EMB2719 | 2 |

| | | |
|---|---|---|
| Overview of 82 putative new cell cycle proteins identified in the non-core dataset containing at least 2 of the cell cycle-related features listed in Table S4. The number of identified features per protein is shown. | | |

| Locus | Description | # of motifs |
|---|---|---|
| AT5G18620 | CHR17 | 6 |
| AT4G38780 | splicing factor, putative | 5 |
| AT1G01970 | pentatricopeptide (PPR) repeat-containing protein | 5 |
| AT4G38900 | bZIP protein | 5 |
| AT4G17020 | transcription factor-related | 4 |
| AT2G46020 | ATBRM, CHR2, BRM | 4 |
| AT5G10800 | RNA recognition motif (RRM)-containing protein | 4 |
| AT5G40460 | expressed | 4 |
| AT1G36160 | AT-ACC1, EMB22, GK, PAS3, ACC1 | 4 |
| AT1G77180 | chromatin protein family | 4 |
| AT1G16520 | similar to unknown protein [Arabidopsis thaliana] (TAIR:AT1G56080.1) | 4 |
| AT3G49240 | EMB1796 | 4 |
| AT4G24680 | similar to unnamed protein product [Vitis vinifera] (GB:CA064289.1) | 4 |
| AT2G25730 | binding / heme binding | 4 |
| AT5G38480 | RCI1, GRF3 | 4 |
| AT4G25550 | M7J2_80 | 4 |
| AT3G05190 | aminotransferase class IV family protein | 4 |
| AT4G35740 | RecQI3 | 4 |
| AT3G62240 | zinc finger (C2H2 type) family protein | 3 |
| AT3G45970 | EXPL1, ATEXPL1, ATHEXP BETA 2.1, ATEXLA1 | 3 |
| AT3G56690 | CIP111 | 3 |
| AT3G56860 | UBA2A | 3 |
| AT3G01280 | porin, putative | 3 |
| AT3G20390 | endoribonuclease L-PSP family protein | 3 |
| AT5G03740 | HDT3, HD2C | 3 |
| AT5G25210 | similar to unknown protein [Arabidopsis thaliana] (TAIR:AT4G32030.1) | 3 |
| AT1G48920 | ATNUC-L1, PARL1 | 3 |
| AT5G58150 | leucine-rich repeat transmembrane protein kinase, putative | 3 |
| AT1G17340 | phosphoinositide phosphatase family protein | 3 |
| AT1G23860 | RSZP21, SRZ21, SRZ-21 | 3 |
| AT2G16950 | protein transporter | 3 |
| AT1G06070 | bZIP transcription factor, putative (bZIP69) | 3 |
| AT1G71380 | ATGH9B3, ATCEL3 | 3 |
| AT2G44350 | CSY4, ATCS | 3 |
| AT1G30860 | protein binding / zinc ion binding | 3 |
| AT3G47930 | ATGLDH | 3 |
| AT1G03060 | WD-40 repeat family protein / beige-related | 3 |
| AT4G21860 | MSRB2 | 3 |
| AT2G30470 | HSI2 | 2 |
| AT1G80720 | mitochondrial glycoprotein family protein / MAM33 family protein | 2 |
| AT1G09760 | U2A' | 2 |
| AT1G52360 | coatomer protein complex, subunit beta 2 (beta prime), putative | 2 |
| AT1G22300 | GF14 EPSILON, GRF10 | 2 |
| AT3G13640 | ATRLI1 | 2 |
| AT5G12140 | ATCYS1 | 2 |
| AT1G66340 | EIN1, ETR, ETR1 | 2 |
| AT1G42440 | similar to unknown protein [Arabidopsis thaliana] (TAIR:AT1G06720.1) | 2 |
| AT4G09320 | NDPK1 | 2 |
| AT5G54900 | ATRBP45A | 2 |
| AT1G09100 | RPT5B | 2 |
| AT1G52730 | transducin family protein / WD-40 repeat family protein | 2 |
| AT3G49430 | SRP34A | 2 |
| AT3G53880 | aldo/keto reductase family protein | 2 |
| AT4G16630 | DEAD/DEAH box helicase, putative (RH28) | 2 |
| AT2G03820 | nonsense-mediated mRNA decay NMD3 family protein | 2 |
| AT1G23190 | phosphoglucomutase, cytoplasmic, putative / glucose phosphomutase, | 2 |
| AT3G01540 | ATDRH1, DRH1 | 2 |
| AT5G20920 | EMB1401, EIF2 BETA | 2 |
| AT4G14310 | DL3195C | 2 |
| AT1G09430 | ACLA-3 | 2 |
| AT2G33340 | transducin family protein / WD-40 repeat family protein | 2 |
| AT1G01880 | DNA repair protein, putative | 2 |
| AT5G62350 | invertase/pectin methylesterase inhibitor family protein / DC 1.2 homolog | 2 |
| AT4G34150 | C2 domain-containing protein | 2 |
| AT5G46780 | VQ motif-containing protein | 2 |
| AT5G26360 | chaperonin, putative | 2 |
| AT4G38440 | similar to hypothetical protein [Vitis vinifera] (GB:CAN83259.1) | 2 |
| AT3G29800 | AAA-type ATPase family | 2 |
| AT5G58290 | RPT3 | 2 |
| AT3G20050 | ATTCP-1 | 2 |
| AT1G19520 | NFD5 | 2 |
| AT4G26870 | aspartyl-tRNA synthetase, putative / aspartate--tRNA ligase, putative | 2 |
| AT1G56080 | similar to unknown protein [Arabidopsis thaliana] (TAIR:AT1G16520.1) | 2 |
| AT5G12380 | annexin, putative | 2 |
| AT4G28230 | similar to unnamed protein product [Vitis vinifera] (GB:CA041238.1) | 2 |
| AT2G06210 | VIP6, ELF8 | 2 |
| ATCG00480 | ATPB | 2 |
| AT5G02530 | RNA and export factor-binding protein, putative | 2 |
| AT1G31760 | SWIB complex BAF60b domain-containing protein | 2 |
| AT5G06680 | ATSPC98, SPC98 | 2 |
| AT2G16570 | ATASE1, ATASE | 2 |
| AT1G62390 | octicosapeptide/Phox/Bem1p (PB1) domain-containing protein / | 2 |
| AT1G43800 | acyl-(acyl-carrier-protein) desaturase, putative / stearoyl-ACP | 2 |

**Table 7: Overview of microarray experiments used to calculate the transcript PCCs.**

| | |
|---|---|
| List of experiments used to build an *Arabidopsis* ATH1 micro-array compendium of 518 experiments focused on cell cycle or plant growth and development used to calculate the transcript Pearson correlation coefficients. | |

| Description | Reference |
|---|---|
| co-2;3 days long day condition; apical region; rosett stage | AtGenExpress;/share/nasc/AtGenExpre ss_dev_series_mutants_and_other_eco types/CEL/co-3-1.CEL |
| Col-0;3 days long day condition; apical region; rosett stage | AtGenExpress;/share/nasc/AtGenExpre ss_dev_series_mutants_and_other_eco types/CEL/Col-3-1.CEL |
| ft-2;3 days long day condition; apical region; rosett stage | AtGenExpress;/share/nasc/AtGenExpre ss_dev_series_mutants_and_other_eco types/CEL/ft-3-1.CEL |
| Ler;3 days long day condition; apical region; rosett stage | AtGenExpress;/share/nasc/AtGenExpre ss_dev_series_mutants_and_other_eco types/CEL/Ler-3-1.CEL |
| Ify-12;3 days long day condition; apical region; rosett stage | AtGenExpress;/share/nasc/AtGenExpre ss_dev_series_mutants_and_other_eco types/CEL/Ify-3-1.CEL |
| co-2;5 days long day condition; apical region; rosett stage | AtGenExpress;/share/nasc/AtGenExpre ss_dev_series_mutants_and_other_eco types/CEL/co-5-1.CEL |
| Col-0;5 days long day condition; apical region; rosett stage | AtGenExpress;/share/nasc/AtGenExpre ss_dev_series_mutants_and_other_eco types/CEL/Col-5-1.CEL |
| ft-2;5 days long day condition; apical region; rosett stage | AtGenExpress;/share/nasc/AtGenExpre ss_dev_series_mutants_and_other_eco types/CEL/ft-5-1.CEL |
| Ler;5 days long day condition; apical region; rosett stage | AtGenExpress;/share/nasc/AtGenExpre ss_dev_series_mutants_and_other_eco types/CEL/Ler-5-1.CEL |
| Ify-12;5 days long day condition; apical region; rosett stage | AtGenExpress;/share/nasc/AtGenExpre ss_dev_series_mutants_and_other_eco types/CEL/Ify-5-1.CEL |
| co-2;7 days long day condition; apical region; rosett stage | AtGenExpress;/share/nasc/AtGenExpre ss_dev_series_mutants_and_other_eco types/CEL/co-7-1.CEL |
| Col-0;7 days long day condition; apical region; rosett stage | AtGenExpress;/share/nasc/AtGenExpre ss_dev_series_mutants_and_other_eco types/CEL/Col-7-1.CEL |
| ft-2;7 days long day condition; apical region; rosett stage | AtGenExpress;/share/nasc/AtGenExpre ss_dev_series_mutants_and_other_eco types/CEL/ft-7-1.CEL |
| Ler;7 days long day condition; apical region; rosett stage | AtGenExpress;/share/nasc/AtGenExpre ss_dev_series_mutants_and_other_eco types/CEL/Ler-7-1.CEL |
| Ify-12;7 days long day condition; apical region; rosett stage | AtGenExpress;/share/nasc/AtGenExpre ss_dev_series_mutants_and_other_eco types/CEL/Ify-7-1.CEL |
| co-2;no treatment; apical region; rosett stage | AtGenExpress;/share/nasc/AtGenExpre ss_dev_series_mutants_and_other_eco types/CEL/co-0-1.CEL |
| Col-0;no treatment; apical region; rosett stage | AtGenExpress;/share/nasc/AtGenExpre ss_dev_series_mutants_and_other_eco types/CEL/Col-0-1.CEL |
| ft-2;no treatment; apical region; rosett stage | AtGenExpress;/share/nasc/AtGenExpre ss_dev_series_mutants_and_other_eco types/CEL/ft-0-1.CEL |
| Ler;no treatment; apical region; rosett stage | AtGenExpress;/share/nasc/AtGenExpre ss_dev_series_mutants_and_other_eco types/CEL/Ler-0-1.CEL |
| Ify-12;no treatment; apical region; rosett stage | AtGenExpress;/share/nasc/AtGenExpre ss_dev_series_m utants_and_other_eco types/CEL/Ify-0-1.CEL |
| Wt roots; 7 days; continuous light; soil | AtGenExpress;/share/nasc/AtGenExpre ss_dev_series_roots/CEUATGE_3_A.C EL |
| Wt roots 17 days continuous light soil | AtGenExpress;/share/nasc/AtGenExpre ss_dev_series_roots/CEUATGE_9_A.C EL |
| Wt root 15 days long day (16/8) 1x MS agar, 1% sucrose | AtGenExpress;/share/nasc/AtGenExpre ss_dev_series_roots/CEUATGE_93_A. CEL |
| Wt root 8 days continuous light 1x MS agar | AtGenExpress;/share/nasc/AtGenExpre ss_dev_series_roots/CEL/ATGE_94_A. CEL |
| Wt root 8 days continuous light 1x MS agar, 1% sucrose | AtGenExpress;/share/nasc/AtGenExpre ss_dev_series_roots/CEUATGE_95_A. CEL |
| Wt root 21 days continuous light 1x MS agar | AtGenExpress;/share/nasc/AtGenExpre ss_dev_series_roots/CEUATGE_98_A. CEL |
| Wt root 21 days continuous light 1x MS agar, 1% sucrose | AtGenExpress;/share/nasc/AtGenExpre ss_dev_series_roots/CEUATGE_99_A. CEL |
| Wt seedling, green parts 21 days continuous light 1x MS agar | AtGenExpress;/share/nasc/AtGenExpre ss_dev_series_seedtings_and_whote_p lants/CEL/ATGE_100_A.CEL |
| Wt seedling, green parts 21 days continuous light 1x MS agar, 1% sucrose | AtGenExpress;/share/nasc/AtGenExpre ss_dev_series_seedlings_and_whole_p lants/CEL/ATGE_101_A.CEL |
| Wt developmental drift, entire rosette after transition to flowering, but before bolting 21 days continuous light soil | AtGenExpress;/share/nasc/AtGenExpre ss_dev_series_seedlings_and_whole_p tants/CEL/ATGE_22_A.CEL |
| Wt as above 22 days continuous light soil | AtGenExpress;/share/nasc/AtGenExpre ss_dev_series_seedlings_and_whole_p lants/CEL/ATGE_23_A.CEL |
| Wt as above 23 days continuous light soil | AtGenExpress;/share/nasc/AtGenExpre ss_dev_series_seedlings_and_whole_p lants/CEL/ATGE_24_A.CEL |
| Wt seedling, green parts; 7 days; continuous light; soil | AtGenExpress;/share/nasc/AtGenExpre ss_dev_series_seedlings_and_whole_p lants/CEL/ATGE_7_A2.CEL |
| Wt seedling, green parts 8 days continuous light 1x MS agar | AtGenExpress;/share/nasc/AtGenExpre ss_dev_series_seedlings_and_whole_p lants/CEL/ATGE_96_A.CEL |
| Wt seedling, green parts 8 days continuous light 1x MS agar, 1% sucrose | AtGenExpress;/share/nasc/AtGenExpre ss_dev_series_seedlings_and_whole_p lants/CEL/ATGE_97_A.CEL |
| Wt siliques, w/ seeds stage 3; mid globular to early heart embryos 8 wk long day (16/8) soil | AtGenExpress;/share/nasc/AtGenExpre ss_dev_series_siliques_and_seeds/CE L/ATGE_76_B.CEL |
| Wt siliques, w/ seeds stage 4; early to late heart embryos 8 wk long day (16/8) soil | AtGenExpress;/share/nasc/AtGenExpre ss_dev_series_siliques_and_seeds/CE L/ATGE_77_D.CEL |
| Wt siliques, w/ seeds stage 5; late heart to mid torpedo embryos 8 wk long day (16/8) soil | AtGenExpress;/share/nasc/AtGenExpre ss_dev_series_siliques_and_seeds/CE L/ATGE_78_D.CEL |
| Wt seeds, stage 6, w/o siliques; mid to late torpedo embryos 8 wk long day (16/8) soil | AtGenExpress;/share/nasc/AtGenExpre ss_dev_series_siliques_and_seeds/CE L/ATGE_79_A.CEL |
| Wt seeds, stage 7, w/o siliques; late torpedo to early walking-stick embryos 8 wk long day (16/8) soil | AtGenExpress;/share/nasc/AtGenExpre ss_dev_series_siliques_and_seeds/CE L/ATGE_81_A.CEL |
| Wt seeds, stage 8, w/o siliques; walking-stick to early curled cotyledons | AtGenExpress;/share/nasc/AtGenExpre ss_dev_series_siliques_and_seeds/CE L/ATGE_82_A.CEL |
| Wt seeds, stage 9, w/o siliques; curled cotyledons to early green cotyledons embryos ; 8 wk long day (16/8) soil | AtGenExpress;/share/nasc/AtGenExpre ss_dev_series_siliques_and_seeds/CE L/ATGE_83_A.CEL |
| Wt seeds, stage 10, w/o siliques; green cotyledons embryos 8 wk long day (16/8) soil | AtGenExpress;/share/nasc/AtGenExpre ss_dev_series_siliques_and_seeds/CE L/ATGE_84_A.CEL |
| Wt flowers stage 9 21+ days continuous light soil | AtGenExpress;/share/nasc/AtGenExpre ss_Developmental_series_flowers_and _pollen/CEL/ATGE_31_A2.CEL |
| Wt flowers stage 10/11 21+ days continuous light soil | AtGenExpress;/share/nasc/AtGenExpre ss_Developmental_series_flowers_and _pollen/CEL/ATGE_32_A2.CEL |
| Wt flowers stage 12 21+ days continuous light soil | AtGenExpress;/share/nasc/AtGenExpre ss_Developmental_series_flowers_and _pollen/CEL/ATGE_33_A.CEL |
| Wt flowers stage 12, sepals 21+ days continuous light soil | AtGenExpress;/share/nasc/AtGenExpre ss_Developmental_series_flowers_and _pollen/CEL/ATGE_34_A.CEL |
| Wt flowers stage 12, petals 21+ days continuous light soil | AtGenExpress;/share/nasc/AtGenExpre ss_Developmental_series_flowers_and _pollen/CEL/ATGE_35_A.CEL |
| Wt flowers stage 12, stamens 21+ days continuous light soil | AtGenExpress;/share/nasc/AtGenExpre ss_Developmental_series_flowers_and _pollen/CEL/ATGE_36_A.CEL |
| Wt flowers stage 12, carpels 21+ days continuous light soil | AtGenExpress;/share/nasc/AtGenExpre ss_Developmental_series_flowers_and _pollen/CEL/ATGE_37_A.CEL |
| Wt flowers stage 15 21 + days continuous light soil | AtGenExpress;/share/nasc/AtGenExpre ss_Developmental_series_flowers_and _pollen/CEL/ATGE_39_ACEL |
| Wt flowers stage 15, pedicels 21+ days continuous light soil | AtGenExpress;/share/nasc/AtGenExpre ss_Developmental_series_flowers_and _pollen/CEL/ATGE_40_A.CEL |
| Wt flowers stage 15, sepals 21+ days continuous light soil | AtGenExpress;/share/nasc/AtGenExpre ss_Developmental_series_flowers_and _pollen/CEL/ATGE_41_A.CEL |
| Wt flowers stage 15, petals 21+ days continuous light soil | AtGenExpress;/share/nasc/AtGenExpre ss_Developmental_series_flowers_and _pollen/CEL/ATGE_42_B.CEL |
| Wt flowers stage 15, stamen 21+ days continuous light soil | AtGenExpress;/share/nasc/AtGenExpre ss_Developmental_series_flowers_and _pollen/CEL/ATGE_43_A.CEL |
| Wt flowers stage 15, carpels 21 + days continuous light soil | AtGenExpress;/share/nasc/AtGenExpress_Developmental_series_flowers_and _pollen/CEL/ATGE_45_A.CEL |
| Wt mature pollen 6 wk continuous light soil | AtGenExpress;/share/nasc/AtGenExpre ss_Developmental_series_flowers_and _pollen/CEL_ATGE_73_A.CEL |
| Wt flower 28 days long day (16/8) soil | AtGenExpress;/share/nasc/AtGenExpre ss_Developmental_series_flowers_and _pollen/CEL/ATGE_92_A.CEL |
| clv3-7 flower stage 12; multi-carpel gynoeceum; enlarged meristem; increased organ number; 21+ days continuous light soil | AtGenExpress;/share/nasc/AtGenExpre ss_Developmental_series_flowers_and _pollen/CEL/ATGE_53_A.CEL |
| Ify-12 flower stage 12; shoot characteristics; most organs leaf-like 21+ days continuous light soil | AtGenExpress;/share/nasc/AtGenExpre ss_Developmental_series_flowers_and _pollen/CEL/ATGE_54_A.CEL |
| ap1-15 flower stage 12; sepals replaced by leaf-like organs, petals mostly lacking, 2° flowers ; 21+ days continuous light soil | AtGenExpress;/share/nasc/AtGenExpre ss_Developmental_series_flowers_and _pollen/CEL/ATGE_55_A.CEL |
| ap2-6 flower stage 12; no sepals or petals 21+ days continuous light soil | AtGenExpress;/share/nasc/AtGenExpre ss_Developmental_series_flowers_and _pollen/CEL/ATGE_56_A.CEL |
| ap3-6 flower stage 12; no petals or stamens 21+ days continuous light soil | AtGenExpress;/share/nasc/AtGenExpre ss_Developmental_series_flowers_and _pollen/CEL/ATGE_57_A.CEL |
| ag-12 flower stage 12; no stamens or carpels 21+ days continuous light soil | AtGenExpress;/share/nasc/AtGenExpre ss_Developmental_series_flowers_and _pollen/CEL/ATGE_58_A.CEL |
| ufo-1 flower stage 12; filamentous organs in whorls two and three 21+ days continuous light soil | AtGenExpress;/share/nasc/AtGenExpre ss_Developmental_series_flowers_and _pollen/CEL/ATGE_59_A.CEL |
| Wt cotyledons; 7 days; continuous light; soil | AtGenExpress;/share/nasc/AtGenExpre ss_Developmental_series_leaves/CEL/ ATGE_1_A.CEL |
| Wt rosette leaf #4, 1 cm long 10 days continuous light soil | AtGenExpress;/share/nasc/AtGenExpre ss_Developmental_series_leaves/CEL/ ATGE_10_A.CEL |
| gl1-T rosette leaf #4, 1 cm long 10 days continuous light soil | AtGenExpress;/share/nasc/AtGenExpre ss_Developmental_series_leaves/CEL/ ATGE_11_A.CEL |
| Wt rosette leaf # 217 days continuous light soil | AtGenExpress;/share/nasc/AtGenExpre ss_Developmental_series_leaves/CEL/ ATGE_12_A.CEL |
| Wt rosette leaf # 417 days continuous light soil | AtGenExpress;/share/nasc/AtGenExpre ss_Developmental_series_leaves/CEL/ ATGE_13_A.CEL |
| Wt rosette leaf # 617 days continuous light soil | AtGenExpress;/share/nasc/AtGenExpre ss_Developmental_series_leaves/CEL/ ATGE_14_A.CEL |
| Wt rosette leaf # 817 days continuous light soil | AtGenExpress;/share/nasc/AtGenExpre ss_Developmental_series_leaves/CEL/ ATGE_15_A.CEL |
| Wt rosette leaf # 10 17 days continuous light soil | AtGenExpress;/share/nasc/AtGenExpre ss_Developmental_series_leaves/CEL/ ATGE_16_A.CEL |
| Wt rosette leaf # 12 17 days continuous light soil | AtGenExpress;/share/nasc/AtGenExpre ss_Developmental_series_leaves/CEL/ ATGE_17_A.CEL |
| gl1-T rosette leaf # 12 17 days continuous light soil | AtGenExpress;/share/nasc/AtGenExpre ss_Developmental_series_leaves/CEL/ATGE_18_A.CEL |
| Wt leaf 7, petiole 17 days continuous light soil | AtGenExpress;/share/nasc/AtGenExpre ss_Developmental_series_leaves/CEL/ ATGE_19_A.CEL |
| Wt leaf 7, proximal half 17 days continuous light soil | AtGenExpress;/share/nasc/AtGenExpre ss_Developmental_series_leaves/CEL/ ATGE_20_A.CEL |
| Wt leaf 7, distal half 17 days continuous light soil | AtGenExpress;/share/nasc/AtGenExpre ss_Developmental_series_leaves/CEL/ ATGE_21_A.CEL |
| Wt senescing leaves 35 days continuous light soil | AtGenExpress;/share/nasc/AtGenExpre ss_Developmental_series_leaves/CEL/ ATGE_25_A.CEL |
| Wt cauline leaves 21+ days continuous light soil | AtGenExpress;/share/nasc/AtGenExpre ss_Developmental_series_leaves/CEL/ ATGE_26_A.CEL |
| Wt leaves 1 + 2; 7 days; continuous light; soil | AtGenExpress;/share/nasc/AtGenExpre ss_Developmental_series_leaves/CEL/ ATGE_5_A.CEL |
| Wt vegetative rosette 7 days short day (10/14) soil | AtGenExpress;/share/nasc/AtGenExpre ss_Developmental_series_leaves/CEL/ ATGE_87_A.CEL |
| Wt vegetative rosette 14 days short day (10/14) soil | AtGenExpress;/share/nasc/AtGenExpre ss_Developmental_series_leaves/CEL/ ATGE_89_A.CEL |
| Wt vegetative rosette 21 days short day (10/14) soil | AtGenExpress;/share/nasc/AtGenExpre ss_Developmental_series_leaves/CEL/ ATGE_90_A.CEL |
| Wt leaf 15 days long day (16/8) 1x MS agar, 1% sucrose | AtGenExpress;/share/nasc/AtGenExpre ss_Developmental_series_leaves/CEL/ ATGE_91_A.CEL |
| Wt hypocotyl; 7 days; continuous light; soil | AtGenExpress;/share/nasc/AtGenExpre ss_Developmental_series_shoots_and_ stems/CEL/ATGE_2_A.CEL |
| Wt stem, 2nd internode 21+ days continuous light soil | AtGenExpress;/share/nasc/AtGenExpre ss_Developmental_series_shoots_and_ stems/CEL/ATGE_27_A.CEL |
| Wt 1st node 21+ days continuous light soil | AtGenExpress;/share/nasc/AtGenExpre ss_Developmental_series_shoots_and_ stems/CEL/ATGE_28_A2.CEL |
| Wt shoot apex, inflorescence (after bolting) 21 days continuous light soil | AtGenExpress;/share/nasc/AtGenExpre ss_Developmental_series_shoots_and_ stems/CEL/ATGE_29_A2.CEL |
| Wt shoot apex, vegetative + young leaves; 7 days; continuous light; soil | AtGenExpress;/share/nasc/AtGenExpre ss_Developmental_series_shoots_and_ stems/CEL/ATGE_4_A.CEL |
| Wt shoot apex, vegetative; 7 days; continuous light; soil | AtGenExpress;/share/nasc/AtGenExpre ss_Developmental_series_shoots_and_ stems/CEL/ATGE_6_A.CEL |
| Wt shoot apex, transition (before bolting) 14 days continuous light soil | AtGenExpress;/share/nasc/AtGenExpre ss_Developmental_series_shoots_and_ stems/CEL/ATGE_8_A.CEL |
| clv3-7 shoot apex, inflorescence (after bolting) 21+ days continuous light soil | AtGenExpress;/share/nasc/AtGenExpre ss_Developmental_series_shoots_and_ stems/CEL/ATGE_46_A.CEL |
| Ify-12 shoot apex, inflorescence (after bolting) 21+ days continuous light soil | AtGenExpress;/share/nasc/AtGenExpre ss_Developmental_series_shoots_and_ stems/CEL/ATGE_47_A.CEL |
| ap1-15 shoot apex, inflorescence (after bolting) 21+ days continuous light soil | AtGenExpress;/share/nasc/AtGenExpre ss_Developmental_series_shoots_and_ stems/CEL/ATGE_48_A.CEL |
| ap2-6 shoot apex, inflorescence (after bolting) 21+ days continuous light soil | AtGenExpress;/share/nasc/AtGenExpre ss_Developmental_series_shoots_and_ stems/CEL/ATGE_49_A.CEL |
| ap3-6 shoot apex, inflorescence (after bolting) 21+ days continuous light soil | AtGenExpress;/share/nasc/AtGenExpre ss_Developmental_series_shoots_and_ stems/CEL/ATGE_50_A.CEL |
| ag-12 shoot apex, inflorescence (after bolting) 21+ days continuous light soil | AtGenExpress;/share/nasc/AtGenExpre ss_Developmental_series_shoots_and_ stems/CEL/ATGE_51_A.CEL |
| ufo-1 shoot apex, inflorescence (after bolting) 21+ days continuous light soil | AtGenExpress;/share/nasc/AtGenExpre ss_Developmental_series_shoots_and_ stems/CEL/ATGE_52_A.CEL |
| Root Quiescent Center;AGL42;quiescent center, non-specific pattern when roots grow old/long;QC only and at very early stages | Birnbaum_arexdb; |
| Root Phloem;APL ;developing pSE and mSE and CC between the two : loss of GFP in mature pSE is accompnied by strong GFP in neighboring CC; GFP reappears in immaure mSE; | Birnbaum_arexdb; |
| Root Cortex;C1 (or 315.1.1) (AT1G09750 promoter);cortex;from elongation to at least immature root hair dif. | Birnbaum_arexdb; |
| Root Epidermis;GL2;atrichoblasts;from QC and up | Birnbaum_arexdb; |
| Root Ground tissue;J0571 ;ground: endo + cortex + qc;from tip all the way up | Birnbaum_arexdb; |
| Lateral Root Cap;J3411;lateral root cap plus epidermis;from tip to exactly where? at least the differentiation zone; GFP is about 5X weaker in dif. zone | Birnbaum_arexdb; |
| Root Columella;PET111;columnella - tier 2,3,4; | Birnbaum_arexdb; |
| Root Xylem;S18 (477.1.3) (AT5G12870 promoter);maturing xylem cells;starts from mid-elongation and stops half way; GFP switches from pSE to mSE | Birnbaum_arexdb; |
| Root Endodermis;SCR;Marks the QC and the endodermis at least past the mature hair zone;from QC all the way up? least past mature hair zone | Birnbaum_arexdb; At |
| Tissue microdissection;Stagel;all radial tissues;where the root tip reaches its full diameter (about 0.15 mm from the root | Birnbaum_arexdb; tip) |
| Tissue microdissection;Stagell;all radial tissues;where cells transition from being optically dense to a more transparent appearance as they begin longitudinal expansion | Birnbaum_arexdb; |
| Tissue microdissection;Stagelll;all radial tissues;where root hairs are fully elongated (about 0.45 to 2 mm from the root | Birnbaum_arexdb; tip) |
| Root Stele;WOL;stele;from QC and starts to fade from elongation zone | Birnbaum_arexdb; |
| Campb-Col0-Suc | Malcolm_Campbell_1 ;/share/nasc/Malc olm_Campbell_1/CEUMC001_ATH1_A 1-Campb-317.CEL |
| Campb-Co10-noSuc | Malcolm_Campbell_1;/share/nasc/Malc olm_Campbell_1/CEL/MC001_ATH1_A 4-Campb-320.CEL |
| Campb-MYB61-Suc | Malcolm_Campbell_1;/share/nasc/Malc olm_Campbell_1/CEL/MC001_ATH1_A 7-Campb-323.CEL |
| Campb-MYB61-noSuc | Malcolm_Campbell_1;/share/nasc/Malc olm_Campbell_1/CEL/MC001_ATH1_A 10-Campb-326.CEL |
| Campb-MYB61oe-Suc | Malcolm_Campbell_1;/share/nasc/Malc olm_Campbell_1/CEL/MC001_ATH1_A 13-Campb-329(new).CEL |
| Campb-MYB61oe-noSuc | Malcolm_CampbelL_1;/share/nasc/Malc olm_Campbell_1/CEL/MC001_ATH1_A 16-Campb-332.CEL |
| col;hypocotyl | Malcolm_Campbell_2;/share/nasc/Malc olm_Campbell_2/CEL/MC002_ATH1_A 10.1-dubos-wth.CEL |
| max4;hypocotyl | Malcolm_Campbell_2;/share/nasc/Malc olm_Campbell_2/CEL/MC002_ATH1_A 11.1-dubos-mxh.CEL |
| axr1;hypocotyl | Malcolm_Campbell_2;/share/nasc/Malc olm_Campbell_2/CEL/MC002_ATH1_A 12.1-dubos-arh.CEL |
| col;xylem | Malcolm_Campbell_2;/share/nasc/Malc olm_Campbell_2/CEL/MC002_ATH1_A 1.1-dubos-wtx.CEL |
| col;cork | Malcolm_Campbell_2;/share/nasc/Malc olm_Campbell_2/CEL/MC002_ATH1_A 2.1-dubos-wtc.CEL |
| myb61 ko;xylem | Malcolm_Campbell_2;/share/nasc/Malc olm_Campbell_2/CEL/MC002_ATH1_A 3.1-dubos-6kx.CEL |
| myb61 ko;cork | Malcolm_Campbell_2;/share/nasc/Malc olm_Campbell_2/CEL/MC002_ATH1_A 4.1-dubos-6kc.CEL |
| myb50 ko;xylem | Malcolm_Campbell_2;/share/nasc/Malc olm_Campbell_2/CEL/MC002_ATH1_A 5.1-dubos-Skx.CEL |
| myb50;cork | Malcolm_Campbell_2;/share/nasc/Malc olm_Campbell_2/CEL/MC002_ATH1_A 6.1-dubos-5kc.CEL |
| ler;hypocotyl | Malcolm_Campbell_2;/share/nasc/Malc olm_Campbell_2/CEL/MC002_ATH1_A 7.1-dubos-wLh.CEL |
| abi1;hypocotyl (ler background) | Malcolm_Campbell_2;/share/nasc/Malc olm_Campbell_2/CEL/MC002_ATH1_A 8.1-dubos-aih.CEL |
| aba1;hypocotyl (ler background) | Malcolm_Campbell_2;/share/nasc/Malc olm_Campbell_2/CEL/MC002_ATH1_A 9.1-dubos-aah.CEL |
| seedling; Col0; 10nM BL for 1h | AtGenExpress;/share/nasc/AtGen_Bras sinolide_timecource_wt_det2_seedlings /CEL/RIKENGODA16A.cel |
| seedling; Col0; mock treatment for 3h | AtGenExpress;/share/nasc/AtGen_Bras sinolide_timecource_wt_det2_seedlings /CEL/RIKENGODA17AC.cel |
| seedling; Col0; mock treatment for 30min | AtGenExpress;/share/nasc/AtGen_Bras sinolide_timecource_wt_det2_seedlings /CEL/RIKENGODA1AC.cel |
| seedling; Col0; 10nM BL for 3h | AtGenExpress;/share/nasc/AtGen_Bras sinolide_timecource_wt_det2_seedlings /CEL/RIKENGODA24A.cel |
| seedling; de-etiolated; mock treatment for 30min | AtGenExpress;/share/nasc/AtGen_Bras sinolide_timecource_wt_det2_seedlings /CEL/RIKENGODA31A.cel |
| seedling; de-etiolated; 10nM BL for 30min | AtGenExpress;/share/nasc/AtGen_Bras sinolide_timecource_wt_det2_seedlings /CEL/RIKENGODA32A.cel |
| seedling; de-etiolated; mock treatment for 1 h | AtGenExpress;/share/nasc/AtGen_Bras sinolide_timecource_wt_det2_seedlings /CEL/RIKENGODA33A.cel |
| seedling; de-etiolated; 10nM BL for 1h | AtGenExpress;/share/nasc/AtGen_Bras sinolide_timecource_wt_det2_seedlings /CEL/RIKENGODA34A.cel |
| seedling; de-etiolated; mock treatment for 3h | AtGenExpress;/share/nasc/AtGen_Bras sinolide_timecource_wt_det2_seedlings /CEL/RIKENGODA35A.cel |
| seedling; de-etiolated; 10nM BL for 3h | AtGenExpress;/share/nasc/AtGen_Bras sinolide_timecource_wt_det2_seedlings /CEL/RIKENGODA36A.cel |
| seedling; Col0; 10nM BL for 30min | AtGenExpress;/share/nasc/AtGen_Bras sinolide_timecource_wt_det2_seedlings /CEL/RIKENGODA8A.cel |
| seedling; Col0; mock treatment for 1 h | AtGenExpress;/share/nasc/AtGen_Bras sinolide_timecource_wt_det2_seedlings /CEL/RIKENGODA9AC.cel |
| complete plant; 10uM brassinazole 91 (Brz91) (brassinosteroid biosynthesis inhibitor) for 12h | AtGenExpress;/share/nasc/AtGen_effec t_brassinosteroid_inhibitors_seeds/CEL /RIKENGODA10A4.cel |
| complete plant; mock treatment for 3h | AtGenExpress;/share/nasc/AtGen_effec t_brassinosteroid_inhibitors_seeds/CEL /RIKENGODA1A4.cel |
| complete plant; mock treatment for 3h | AtGenExpress;/share/nasc/AtGen_effec t_brassinosteroid_inhibitors_seeds/CEL /RIKENGODA28A4.cel |
| complete plant; mock treatment for 12h | AtGenExpress;/share/nasc/AtGen_effec t_brassinosteroid_inhibitors_seeds/CEL /RIKENGODA2A4.cel |
| complete plant; 3uM brassinazole 220 (Brz220) (brassinosteroid biosynthesis inhibitor) for 3h | AtGenExpress;/share/nasc/AtGen_effec t_brassinosteroid_inhibitors_seeds/CEL /RIKENGODA30A4.cel |
| complete plant; 10uM brassinazole 220 (Brz220) (brassinosteroid biosynthesis inhibitor) for 3h | AtGenExpress;/share/nasc/AtGen_effec t_brassinosteroid_inhibitors_seeds/CEL /RIKENGODA7A4.cel |
| complete plant; 10uM paclobutrazol (GA biosynthesis inhibitor) for 3h | AtGenExpress;/share/nasc/AtGen_effec t_GA_inhibitors_on_seeds/CEL/RIKEN GODA11A2.cel |
| complete plant; 10uM paclobutrazol (GA biosynthesis inhibitor) for 12h | AtGenExpress;/share/nasc/AtGen_effec t_GA_inhibitors_on_seeds/CEL/RIKEN GODA12A2.cel |
| complete plant; 10uM prohexadione (GA biosynthesis inhibitor) for 3h | AtGenExpress;/share/nasc/AtGen_effec t_GA_inhibitors_on_seeds/CEL/RIKEN GODA13A2.cel |
| complete plant; 10uM prohexadione (GA biosynthesis inhibitor) for 12h | AtGenExpress;/share/nasc/AtGen_effec t_GA_inhibitors_on_seeds/CEL/RiKEN GODA14A2.cel |
| complete plant; mock treatment for 3h | AtGenExpress;/share/nasc/AtGen_effec t_GA_inhibitors_on_seeds/CEL/RIKEN GODA1A2.cel |
| complete plant; mock treatment for 12h | AtGenExpress;/share/nasc/AtGen_effec t_GA_inhibitors_on_seeds/CEL/RIKEN GODA2A2.cel |
| complete plant; 10uM propiconazole (GA biosynthesis inhibitor) for 3h | AtGenExpress;/share/nasc/AtGen_effect_GA_inhibitors_on_seeds/CEL/RIKEN GODA3A2.cel |
| complete plant; 10uM propiconazole (GA biosynthesis inhibitor) for 12h | AtGenExpress;/share/nasc/AtGen_effec t_GA_inhibitors_on_seeds/CEL/RIKEN GODA4A2.cel |
| complete plant; 10uM uniconazole (GA biosynthesis inhibitor) for 3h | AtGenExpress;/share/nasc/AtGen_effec t_GA_inhibitors_on_seeds/CEL/RIKEN GODA5A2.cel |
| complete plant; 10uM uniconazole (GA biosynthesis inhibitor) for 12h | AtGenExpress;/share/nasc/AtGen_effec t_GA_inhibitors_on_seeds/CEL/RIKEN GODA6A2.cel |
| complete plant; mock treatment for 3h | AtGenExpress;/share/nasc/AtGen_Effe ct_of_proteasome_inhibitor_MG13_on_ seedlings/CEL/RIKENGODA1A9.cel |
| complete plant; 10uM MG132 (carbobenzoxyl-leucinyl-leucinyl-leucinal) for 3h | AtGenExpress;/share/nasc/AtGen_Effe ct_of_proteasome_inhibitor_MG13_on_ seedlings/CEL/RI KENGODA22A9.cel |
| seed; no treatment | AtGenExpress;/share/nasc/AtGen_exp_ profiling_early_germinating_seeds/CEL/ RIKENPRESTONOA.cel |
| seed; imbibed in water(1 hour) | AtGenExpress;/share/nasc/AtGen_exp_ profiling_early_germinating__seeds/CEL/ RIKENPRESTON1A.cel |
| seed; imbibed in water(3 hours) | AtGenExpress;/share/nasc/AtGen_exp_ profiling_early_germinating_seeds/CEL/ RIKENPRESTON2A.cel |
| seedling; 10uM ABA for 1h | AtGenExpress;/share/nasc/AtGenExpre ss_ABA_time_course_in_wildtype_see dlings/CEL/RIKENGODA13A.cel |
| seedling; mock treatment for 3h | AtGenExpress;/share/nasc/AtGenExpre ss_ABA_time_course_in_wildtype_see dlings/CEL/RIKENGODA17A.cel |
| seedling; mock treatment for 30min | AtGenExpress;/share/nasc/AtGenExpre ss_ABA_time_course_in_wildtype_see dlings/CEL/RIKENGODA1A.cel |
| seedling; 10uM ABA for 3h | AtGenExpress;/share/nasc/AtGenExpre ss_ABA_time_course_in_wildtype_see dlings/CEL/RIKENGODA21A.cel |
| seedling; 10uM ABA for 30min | AtGenExpress;/share/nasc/AtGenExpre ss_ABA_time_course_in_wildtype_see dlings/CEL/RIKENGODA5A.cel |
| seedling; mock treatment for 1 h | AtGenExpress;/share/nasc/AtGenExpre ss_ABA_ time_course_in_wildtype_see dlings/CEL/RIKENGODA9A.cel |
| seedling; 10uM ACC for 1 h | AtGenExpress;/share/nasc/AtGenExpre ss_ACC_time_course_in_wildtype_see dlings/CEL/RIKENGODA15A.cel |
| seedling; mock treatment for 3h | AtGenExpress;/share/nasc/AtGenExpre ss_ACC_time_course_in_wildtype_see dlings/CEL/RIKENGODA17AA.cel |
| seedling; mock treatment for 30min | AtGenExpress;/share/nasc/AtGenExpre ss_ACC_time_course_in_wildtype_see dlings/CEL/RIKENGODA1AA.cel |
| seedling; 10uM ACC for 3h | AtGenExpress;/share/nasc/AtGenExpre ss_ACC_time_course_in_wildtype_see dlings/CEL/RIKENGODA23A.cel |
| seedling; 10uM ACC for 30min | AtGenExpress;/share/nasc/AtGenExpre ss_ACC_time_course_in_wildtype_seedlings/CEL/RIKENGODA7A.cel |
| seedling; mock treatment for 1 h | AtGenExpress;/share/nasc/AtGenExpre ss_ACC_time_course_in_wildtype_see dlings/CEL/RIKENGODA9AA.cel |
| complete plant; 10uM 2,4,6-T (2,4,6-trihydroxybenzamide) for 3h | AtGenExpress;/share/nasc/AtGenExpre ss_Effect_of_auxin_inhibitors_on_seedl ings/CEL/RIKENGODA23A3.cel |
| complete plant; 10uM NPA (naphthylphthalamic acid) (inhibitor of auxin transport) for 3h | AtGenExpress;/share/nasc/AtGenExpre ss_Effect_of_auxin_inhibitors_on_seedl ings/CEL/RIKENGODA26A3.cel |
| complete plant; 10uM PCIB (p-chlorophenoxyisobutyric acid) (auxin inhibitor) for 3h | AtGenExpress;/share/nasc/AtGenExpre ss_Effect_of_auxin_inhibitors_on_seedl ings/CEL/RIKENGODA24A3.cel |
| complete plant; 10uM TIBA (2,3,5-triiodobenzoic acid) (inhibitor of auxin transport) for 3h | AtGenExpress;/share/nasc/AtGenExpre ss_Effect_of_auxin_inhibitors_on_seedl ings/CEL/RIKENGODA25A3.cel |
| complete plant; mock treatment for 3h | AtGenExpress;/share/nasc/AtGenExpre ss_Effect_of_auxin_inhibitors_on_seedl ings/CEL/RIKENGODA1A3.cel |
| whole plant; 100nM castasterone for 3h | AtGenExpress;/share/nasc/AtGenExpre ss_Effect_of_brassinosteroids_in_seedl ings/CEL/RIKENGODA12A6.cel |
| whole plant; 10nM brassinolide for 3h | AtGenExpress;/share/nasc/AtGenExpre ss_Effect_of_brassinosteroids_in_seedl ings/CEL/RIKENGODA13A6.cel |
| whole plant; 10uM campestanol for 3h | AtGenExpress;/share/nasc/AtGenExpre ss_Effect_of_brassinosteroids_in_seedl ings/CEURIKENGODA2A6.cel |
| whole plant; 1 uM 3-dehydro-6-deoxoteasterone for 3h | AtGenExpress;/share/nasc/AtGenExpre ss_Effect_of_brassinosteroids_in_seedl ings/CEL/RIKENGODA7A6.cel |
| whole plant; 1 uM 3-dehydroteasterone for 3h | AtGenExpress;/share/nasc/AtGenExpre ss_Effect_of_brassinosteroids_in_seedl ings/CEL/RIKENGODA8A6.cel |
| whole plant; 1 uM 6-deoxocastasterone for 3h | AtGenExpress;/share/nasc/AtGenExpre ss_Effect_of_brassinosteroids_in_seedl ings/CEL/RIKENGODA11A6.cel |
| whole plant; 1 uM 6-deoxocathasterone for 3h | AtGenExpress;/share/nasc/AtGenExpre ss_Effect_of_brassinosteroids_in_seedl ings/CEL/RIKENGODA3A6.cel |
| whole plant; 1 uM 6-deoxoteasterone for 3h | AtGenExpress;/share/nasc/AtGenExpre ss_Effect_of_brassinosteroids_in_seedl ings/CEL/RIKENGODA5A6.cel |
| whole plant; 1uM 6-deoxotyphasterol for 3h | AtGenExpress;/share/nasc/AtGenExpre ss_Effect_of_brassinosteroids_in_seedl ings/CEL/RIKENGODA9A6.cel |
| whole plant; 1 uM cathasterone for 3h | AtGenExpress;/share/nasc/AtGenExpre ss_Effect_of_brassinosteroids_in_seedl ings/CEL/RIKENGODA4A6.cel |
| whole plant; 1uM teasterone for 3h | AtGenExpress;/share/nasc/AtGenExpre ss_Effect_of_brassinosteroids_in_seedl ings/CEL/RIKENGODA6A6.cel |
| whole plant; 1uM typhasterol for 3h | AtGenExpress;/share/nasc/AtGenExpre ss_Effect_of_brassinosteroids_in_seedl ings/CEL/RIKENGODA10A6.cel |
| whole plant; mock treatment for 3h | AtGenExpress;/share/nasc/AtGenExpre ss_Effect_of_brassinosteroids_in_seedl ings/CEL/RIKENGODA1A6.cel |
| complete plant; 10uM AgNO3 (ethylene inhibitor) for 3h | AtGenExpress;/share/nasc/AtGenExpre ss_Effect_of_ethylene_inhibitors_on_se edlings/CEL/RIKENGODA19A7.cel |
| complete plant; mock treatment for 3h | AtGenExpress;/share/nasc/AtGenExpre ss_Effect_of_ethylene_inhibitors_on_se edlings/CEL/RIKENGODA1A7.cel |
| complete plant; 10uM AVG (aminoethoxyvinylglycine) (ethylene inhibitor) for 3h | AtGenExpress;/share/nasc/AtGenExpre ss_Effect_of_ethylene_inhibitors_on_se edlings/CEL/RIKENGODA20A7.cel |
| seedling; 1uM IAA for 1h | AtGenExpress;/share/nasc/AtGenExpre ss_IAA_time_course_in_wildtype_seedl ings/CEL/RIKENGODA10A.cel |
| seedling; mock treatment for 3h | AtGenExpress;/share/nasc/AtGenExpre ss_IAA_time_course_in wildtype_seedl ings/CEL/RIKENGODA17AD.cel |
| seedling; 1uM IAA for 3h | AtGenExpress;/share/nasc/AtGenExpre ss_IAA_time_course_in_wildtype_seedl ings/CEL/RIKENGODA18A.cel |
| seedling; mock treatment for 30min | AtGenExpress;/share/nasc/AtGenExpre ss_IAA_time_course_in_wildtype_seedl ings/CEL/RIKENGODA1AD.cel |
| seedling; 1 uM IAA for 30min | AtGenExpress;/share/nasc/AtGenExpre ss_IAA_time_course_in_wildtype_seedl ings/CEL/RIKENGODA2A.cel |
| seedling; mock treatment for 1 h | AtGenExpress;/share/nasc/AtGenExpre ss_IAA_time_course_In_wildtype_seedl ings/CEL/RIKENGODA9AD.cel |
| seedling; 10uM MJ for 1h | AtGenExpress;/share/nasc/AtGenExpre ss_Methyl_Jasmonate_time_course_in _wildtype/CEL/RIKENGODA14A.cel |
| seedling; mock treatment for | 3h AtGenExpress;/share/nasc/AtGenExpre ss_Methyl_Jasmonate_time_course_in _wildtype/CEL/RIKENGODA17AF.cel |
| seedling; mock treatment for 30min | AtGenExpress;/share/nasc/AtGenExpre ss_Methyl_Jasmonate_time_course_in _wildtype/CEL/RIKENGODA1AF.cel |
| seedling; 10uM MJ for | 3h AtGenExpress;/share/nasc/AtGenExpre ss_Methyl_Jasmonate_time_course_in _wildtype/CEL/RIKENGODA22A.cel |
| seedling; 10uM MJ for 30min | AtGenExpress;/share/nasc/AtGenExpre ss_Methyl_Jasmonate_time_course_in _wildtype/CEL/RIKENGODA6A.cel |
| seedling; mock treatment for 1 | h AtGenExpress;/share/nasc/AtGenExpre ss_Methyl_Jasmonate_time_course_in _wildtype/CEL/RIKENGODA9AF.cel |
| seedling; 1uM zeatin for 1h | AtGenExpress;/share/nasc/AtGenExpre ss_Zeatin_time_course_in_wildtype_se edlings/CEL/RIKENGODA11A.cel |
| seedling; 1uM zeatin for 30min | AtGenExpress;/share/nasc/AtGenExpre ss_Zeatin_time_course_in_wildtype_se edlings/CEL/RIKENGODA3A.cel |
| seedling; 1 uM zeatin for 3h | AtGenExpress;/share/nasc/AtGenExpre ss_Zeatin_time_course_in_wildtype_se edlings/CEL/RIKENGODA19A.cel |
| seedling; mock treatment for 1 h | AtGenExpress;/share/nasc/AtGenExpre ss_Zeatin_time_course_in_wildtype_se edlings/CEL/RIKENGODA9AG.cel |
| seedling; mock treatment for 30min | AtGenExpress;/share/nasc/AtGenExpress_Zeatin_time_course_in_witdtype_se edlings/CEL/RIKENGODA1AG.cel |
| seedling; mock treatment for 3h | AtGenExpress;/share/nasc/AtGenExpre ss_Zeatin_time_course_in_wildtype_se edlings/CEL/RIKENGODA17AG.cel |
| seeds; water treatment for 3 hours | AtGenExpress;/share/nasc/AtGenExpre ss_Basic_hormone_treatment_of_seed s/CEL/RIKENLI1A.cel |
| seeds; water treatment for 6 hours | AtGenExpress;/share/nasc/AtGenExpre ss_Basic_hormone_treatment_of_seed s/CEL/RIKENLI2A.cel |
| seeds; water treatment for 9 hours | AtGenExpress;/share/nasc/AtGenExpre ss_Basic_hormone_treatment_of_seed s/CEL/RIKENLI3A.cel |
| seeds; 5 uM gibberellin treatment for 3 hours | AtGenExpress;/share/nasc/AtGenExpre ss_Basic_hormone_treatment_of_seed s/CEL/RIKENLI4A.cel |
| seeds; 5 uM gibberellin treatment for 6 hours | AtGenExpress;/share/nasc/AtGenExpre ss_Basic_hormone_treatment_of_seed s/CEL/RIKENLI5A.cel |
| seeds; 5 uM gibberellin treatment for 9 hours | AtGenExpress;/share/nasc/AtGenExpre ss_Basic_hormone_treatment_of_seed s/CEL/RIKENLI6A.cel |
| Col0; complete plant; No treatment | AtGenExpress;/share/nasc/AtGenExpre ss_Cytokinin_treatment_of_seedlings/C EL/NO10.cel |
| Col0; complete plant; 20uM t-zeatin for 3h | AtGenExpress;/share/nasc/AtGenExpre ss_Cytokinin_treatment_of_seedlings/C EL/NO192.cel |
| ARR22-ox; complete plant; No treatment | AtGenExpress;/share/nasc/AtGenExpre ss_Cytokinin_treatment_of_seedlings/C EL/NO28.cel |
| ARR22-ox; complete plant; 20uM t-zeatin for 3h | AtGenExpress;/share/nasc/AtGenExpre ss_Cytokinin_treatment_of_seedlings/C EL/NO31.cel |
| seed; 24 h imbibed in 3 uM ABA | AtGenExpress;/share/nasc/AtGenExpre ss_Effect_of_ABA_during_seed_imbibit ion/CEL/RIKENNAKABAYASHI3A.cel |
| seed; 24 h imbibed in 30 uM ABA | AtGenExpress;/share/nasc/AtGenExpre ss_Effect_of_ABA_during_seed_imbibit ion/CEL/RIKENNAKABAYASHI4A.cel |
| seed; 24 h imbibed in water | AtGenExpress;/share/nasc/AtGenExpre ss_Effect_of_ABA_during_seed_imbibit ion/CEL/RIKENNAKABAYASHI2A.cel |
| seed; no treatment | AtGenExpress;/share/nasc/AtGenExpre ss_Effect_of_ABA_during_seed_imbibit ion/CEL/RIKENNAKABAYASHI1A.cel |
| Controlstress-Shoots-0h | AtGenExpress;/share/nasc/AtGenExpre ss_Stress_Treatments_Control_plants/ CEL/0011.CEL |
| Controlstress-Roots-0h | AtGenExpress;/share/nasc/AtGenExpre ss_Stress_Treatments_Control_plants/ CEL/0021.CEL |
| Controlstress-Shoots-0.5h | AtGenExpress;/share/nasc/AtGenExpre ss_Stress_Treatments_Control_plants/ CEL/0111.CEL |
| Controlstress-Roots-0.5h | AtGenExpress;/share/nasc/AtGenExpre ss_Stress_Treatments_Control_plants/CEL/0121.CEL |
| Controlstress-Shoots-1.0h | AtGenExpress;/share/nasc/AtGenExpre ss_Stress_Treatments_Control_plants/ CEL/0211.CEL |
| Controlstress-Roots-1.0h | AtGenExpress;/share/nasc/AtGenExpre ss_Stress_Treatments_Control_plants/ CEL/0221.CEL |
| Controlstress-Shoots-3.0h | AtGenExpress;/share/nasc/AtGenExpre ss_Stress_Treatments_Control_plants/ CEL/0311.CEL |
| Controlstress-Roots-3.0h | AtGenExpress;/share/nasc/AtGenExpre ss_Stress_Treatments_Control_plants/ CEL/0321.CEL |
| Controlstress-Shoots-6.0h | AtGenExpress;/share/nasc/AtGenExpre ss_Stress_Treatments_Control_plants/ CEL/0411.CEL |
| Controlstress-Roots-6.0h | AtGenExpress;/share/nasc/AtGenExpre ss_Stress_Treatments_Control_plants/ CEL/0421.CEL |
| Controlstress-Shoots-12.0h | AtGenExpress;/share/nasc/AtGenExpre ss_Stress_Treatments_Control_plants/ CEL/0511.CEL |
| Controlstress-Roots-12.0h | AtGenExpress;/share/nasc/AtGenExpre ss_Stress_Treatments_Control_plants/ CEU0521.CEL |
| Controlstress-Shoots-24.0h | AtGenExpress;/share/nasc/AtGenExpre ss_Stress_Treatments_Control_plants/ CEL/0611.CEL |
| Controlstress-Roots-24.0h | AtGenExpress;/share/nasc/AtGenExpre ss_Stress_Treatments_Control_plants/ CEL/0621.CEL |
| Controlstress-Shoots-0.25h | AtGenExpress;/share/nasc/AtGenExpre ss_Stress_Treatments_Control_plants/ CEL/0711.CEL |
| Controlstress-Roots-0.25h | AtGenExpress;/share/nasc/AtGenExpre ss_Stress_Treatments_Control_plants/ CEL/0721.CEL |
| Controlstress-Shoots-4.0h | AtGenExpress;/share/nasc/AtGenExpre ss_Stress_Treatments_Control_plants/ CEL/0811.CEL |
| Controlstress-Roots-4.0h | AtGenExpress;/share/nasc/AtGenExpre ss_Stress_Treatments_Control_plants/ CEL/0821.CEL |
| Coldstress(4?C)-Shoots-0.5h | AtGenExpress;/share/nasc/AtGenExpre ss_Stress_Treatments_Cold_stress/CE L/1111.CEL |
| Coldstress(4?C)-Roots-0.5h | AtGenExpress;/share/nasc/AtGenExpre ss_Stress_Treatments_Cold_stress/CE L/1121.CEL |
| Coldstress(4?C)-Shoots-1.0h | AtGenExpress;/share/nasc/AtGenExpre ss_Stress_Treatments_Cold_stress/CE L/1211.CEL |
| Coldstress(4?C)-Roots-1.0h | AtGenExpress;/share/nasc/AtGenExpre ss_Stress_Treatments_Cold_stress/CE L/1221.CEL |
| Coldstress(4?C)-Shoots-3.0h | AtGenExpress;/share/nasc/AtGenExpre ss_Stress_Treatments_Cold_stress/CE L/1311.CEL |
| Coldstress(4?C)-Roots-3.0h | AtGenExpress;/share/nasc/AtGenExpre ss_Stress_Treatments_Cold_stress/CE L/1321.CEL |
| Coldstress(4?C)-Shoots-6.0h | AtGenExpress;/share/nasc/AtGenExpre ss_Stress_Treatments_Cold_stress/CE L/1411.CEL |
| Coldstress(4?C)-Roots-6.0h | AtGenExpress;/share/nasc/AtGenExpre ss_Stress_Treatments_Cold_stress/CE L/1421.CEL |
| Coldstress(4?C)-Shoots-12.0h | AtGenExpress;/share/nasc/AtGenExpre ss_Stress_Treatments_Cold_stress/CE L/1511.CEL |
| Coldstress(4?C)-Roots-12.0h | AtGenExpress;/share/nasc/AtGenExpre ss_Stress_Treatments_Cold_stress/CE L/1521.CEL |
| Coldstress(4?C)-Shoots-24.0h | AtGenExpress;/share/nasc/AtGenExpre ss_Stress_Treatments_Cold_stress/CE L/1611.CEL |
| Coldstress(4?C)-Roots-24.0h | AtGenExpress;/share/nasc/AtGenExpre ss_Stress_Treatments_Cold_stress/CE L/1621.CEL |
| Osmoticstress-Shoots-0.5h | AtGenExpress;/share/nasc/AtGenExpre ss_Stress_Treatments_Osmotic_stress/ CEL/2111.CEL |
| Osmoticstress-Roots-0.5h | AtGenExpress;/share/nasc/AtGenExpre ss_Stress_Treatments_Osmotic_stress/ CEL/2121.CEL |
| Osmoticstress-Shoots-1.0h | AtGenExpress;/share/nasc/AtGenExpre ss_Stress_Treatments_Osmotic_stress/ CEL/2211.CEL |
| Osmoticstress-Roots-1.0h | AtGenExpress;/share/nasc/AtGenExpre ss_Stress_Treatments_Osmotic_stress/ CEL/2221.CEL |
| Osmoticstress-Shoots-3.0h | AtGenExpress;/share/nasc/AtGenExpre ss_Stress_Treatments_Osmotic_stress/ CEL/2311.CEL |
| Osmoticstress-Roots-3.0h | AtGenExpress;/share/nasc/AtGenExpre ss_Stress_Treatments_Osmotic_stress/ CEL/2321.CEL |
| Osmoticstress-Shoots-6.0h | AtGenExpress;/share/nasc/AtGenExpre ss_Stress_Treatments_Osmotic_stress/ CEL/2411.CEL |
| Osmoticstress-Roots-6.0h | AtGenExpress;/share/nasc/AtGenExpre ss_Stress_Treatments_Osmotic_stress/ CEU2421.CEL |
| Osmoticstress-Shoots-12.0h | AtGenExpress;/share/nasc/AtGenExpre ss_Stress_Treatments_Osmotic_stress/ CEL/2511.CEL |
| Osmoticstress-Roots-12.0h | AtGenExpress;/share/nasc/AtGenExpre ss_Stress_Treatments_Osmotic_stress/ CEL/2521.CEL |
| Osmoticstress-Shoots-24.0h | AtGenExpress;/share/nasc/AtGenExpre ss_Stress_Treatments_Osmotic_stress/ CEL/2611.CEL |
| Osmoticstress-Roots-24.0h | AtGenExpress;/share/nasc/AtGenExpre ss_Stress_Treatments_Osmotic_stress/ CEU2621.CEL |
| Saltstress-Shoots-0.5h | AtGenExpress;/share/nasc/AtGenExpress_Stress_Treatments_Salt_stress/CEL /3111.CEL |
| Saltstress-Roots-0.5h | AtGenExpress;/share/nasc/AtGenExpre ss_Stress_Treatments_Salt_stress/CEL /3121.CEL |
| Saltstress-Shoots-1.0h | AtGenExpress;/share/nasc/AtGenExpre ss_Stress_Treatments_Salt_stress/CEL /3211.CEL |
| Saltstress-Roots-1.0h | AtGenExpress;/share/nasc/AtGenExpre ss_Stress_Treatments_Salt_stress/CEL /3221.CEL |
| Saltstress-Shoots-3.0h | AtGenExpress;/share/nasc/AtGenExpre ss_Stress_Treatments_Salt_stress/CEL /3311.CEL |
| Saltstress-Roots-3.0h | AtGenExpress;/share/nasc/AtGenExpre ss_Stress_Treatments_Salt_stress/CEL /3321.CEL |
| Saltstress-Shoots-6.0h | AtGenExpress;/share/nasc/AtGenExpre ss_Stress_Treatments_Salt_stress/CEL /3411.CEL |
| Saltstress-Roots-6.0h | AtGenExpress;/share/nasc/AtGenExpre ss_Stress_Treatments_Salt_stress/CEL /3421.CEL |
| Saltstress-Shoots-12.0h | AtGenExpress;/share/nasc/AtGenExpre ss_Stress_Treatments_Salt_stress/CEL /3511.CEL |
| Saltstress-Roots-12.0h | AtGenExpress;/share/nasc/AtGenExpre ss_Stress_Treatments_Salt_stress/CEL /3521.CEL |
| Saltstress-Shoots-24.0h | AtGenExpress;/share/nasc/AtGenExpre ss_Stress_Treatments_Salt_stress/CEL /3611.CEL |
| Sattstress-Roots-24.0h | AtGenExpress;/share/nasc/AtGenExpre ss_Stress_Treatments_Salt_stress/CEL /3621.CEL |
| Droughtstress-Shoots-0.5h | AtGenExpress;/share/nasc/AtGenExpre ss_Stress_Treatments_Drought_stress/ CEL/4111.CEL |
| Droughtstress-Roots-0.5h | AtGenExpress;/share/nasc/AtGenExpre ss_Stress_Treatments_Drought_stress/ CEL/4121.CEL |
| Droughtstress-Shoots-1.0h | AtGenExpress;/share/nasc/AtGenExpre ss_Stress_Treatments_Drought_stress/ CEL/4211.CEL |
| Droughtstress-Roots-1.0h | AtGenExpress;/share/nasc/AtGenExpre ss_Stress_Treatments_Drought_stress/ CEL/4221.CEL |
| Droughtstress-Shoots-3.0h | AtGenExpress;/share/nasc/AtGenExpre ss_Stress_Treatments_Drought_stress/ CEL/4311.CEL |
| Droughtstress-Roots-3.0h | AtGenExpress;/share/nasc/AtGenExpre ss_Stress_Treatments_Drought_stress/ CEL/4321.CEL |
| Droughtstress-Shoots-6.0h | AtGenExpress;/share/nasc/AtGenExpre ss_Stress_Treatments_Drought_stress/ CEL/4411.CEL |
| Droughtstress-Roots-6.0h | AtGenExpress;/share/nasc/AtGenExpre ss_Stress_Treatments_Drought_stress/CEU4421.CEL |
| Droughtstress-Shoots-12.0h | AtGenExpress;/share/nasc/AtGenExpre ss_Stress_Treatments_Drought_stress/ CEL/4511.CEL |
| Droughtstress-Roots-12.0h | AtGenExpress;/share/nasc/AtGenExpre ss_Stress_Treatments_Drought_stress/ CEL/4521.CEL |
| Droughtstress-Shoots-24.0h | AtGenExpress;/share/nasc/AtGenExpre ss_Stress_Treatments_Drought_stress/ CEL/4611.CEL |
| Droughtstress-Roots-24.0h | AtGenExpress;/share/nasc/AtGenExpre ss_Stress_Treatments_Drought_stress/ CEU4621.CEL |
| Droughtstress-Shoots-0.25h | AtGenExpress;/share/nasc/AtGenExpre ss_Stress_Treatments_Drought_stress/ CEL/4711.CEL |
| Droughtstress-Roots-0.25h | AtGenExpress;/share/nasc/AtGenExpre ss_Stress_Treatments_Drought_stress/ CEL/4721.CEL |
| Genotoxicstress-Shoots-0.5h | AtGenExpress;/share/nasc/AtGenExpre ss_Stress_Treatments_Genotoxic_stre ss/CEL/5111.CEL |
| Genotoxicstress-Roots-0.5h | AtGenExpress;/share/nasc/AtGenExpre ss_Stress_Treatments_Genotoxic_stre ss/CEL/5121.CEL |
| Genotoxicstress-Shoots-1.0h | AtGenExpress;/share/nasc/AtGenExpre ss_Stress_Treatments_Genotoxic_stre ss/CEL/5211.CEL |
| Genotoxicstress-Roots-1.0h | AtGenExpress;/share/nasc/AtGenExpre ss_Stress_Treatments_Genotoxic_stre ss/CEL/5221.CEL |
| Genotoxicstress-Shoots-3.0h | AtGenExpress;/share/nasc/AtGenExpre ss_Stress_Treatments_Genotoxic_stre ss/CEL/5311.CEL |
| Genotoxicstress-Roots-3.0h | AtGenExpress;/share/nasc/AtGenExpre ss_Stress_Treatments_Genotoxic_stre ss/CEU5321.CEL |
| Genotoxicstress-Shoots-6.0h | AtGenExpress;/share/nasc/AtGenExpre ss_Stress_Treatments_Genotoxic_stre ss/CEL/5411.CEL |
| Genotoxicstress-Roots-6.0h | AtGenExpress;/share/nasc/AtGenExpre ss_Stress_Treatments_Genotoxic_stre ss/CEL/5421.CEL |
| Genotoxicstress-Shoots-12.0h | AtGenExpress;/share/nasc/AtGenExpre ss_Stress_Treatments_Genotoxic_stre ss/CEL/5511.CEL |
| Genotoxicstress-Roots-12.0h | AtGenExpress;/share/nasc/AtGenExpre ss_Stress_Treatments_Genotoxic_stre ss/CEL/5521.CEL |
| Genotoxicstress-Shoots-24.0h | AtGenExpress;/share/nasc/AtGenExpre ss_Stress_Treatments_Genotoxic_stre ss/CEL/5611.CEL |
| Genotoxicstress-Roots-24.0h | AtGenExpress;/share/nasc/AtGenExpre ss_Stress_Treatments_Genotoxic_stre ss/CEL/5621.CEL |
| Oxidativestress-Shoots-0.5h | AtGenExpress;/share/nasc/AtGenExpre ss_Stress_Treatments_Oxidative_stres s/CEL/6111.CEL |
| Oxidativestress-Roots-0.5h | AtGenExpress;/share/nasc/AtGenExpre ss_Stress_Treatments_Oxidative_stres s/CEL/6122.CEL |
| Oxidativestress-Shoots-1.0h | AtGenExpress;/share/nasc/AtGenExpre ss_Stress_Treatments_Oxidative_stres s/CEL/6211.CEL |
| Oxidativestress-Roots-1.0h | AtGenExpress;/share/nasc/AtGenExpre ss_Stress_Treatments_Oxidative_stres s/CEL/6223.CEL |
| Oxidativestress-Shoots-3.0h | AtGenExpress;/share/nasc/AtGenExpre ss_Stress_Treatments_Oxidative_stres s/CEL/6311.CEL |
| Oxidativestress-Roots-3.0h | AtGenExpress;/share/nasc/AtGenExpre ss_Stress_Treatments_Oxidative_stres s/CEL/6322.CEL |
| Oxidativestress-Shoots-6.0h | AtGenExpress;/share/nasc/AtGenExpre ss_Stress_Treatments_Oxidative_stres s/CEL/6411.CEL |
| Oxidativestress-Roots-6.0h | AtGenExpress;/share/nasc/AtGenExpre ss_Stress_Treatments_Oxidative_stres s/CEL/6421.CEL |
| Oxidativestress-Shoots-12.0h | AtGenExpress;/share/nasc/AtGenExpre ss_Stress_Treatments_Oxidative_stres s/CEL/6511.CEL |
| Oxidativestress-Roots-12.0h | AtGenExpress;/share/nasc/AtGenExpre ss_Stress_Treatments_Oxidative_stres s/CEL/6523.CEL |
| Oxidativestress-Shoots-24.0h | AtGenExpress;/share/nasc/AtGenExpre ss_Stress_Treatments_Oxidative_stres s/CEL/6611.CEL |
| Oxidativestress-Roots-24.0h | AtGenExpress;/share/nasc/AtGenExpre ss_Stress_Treatments_Oxidative_stres s/CEL/6621.CEL |
| UV-Bstress-Shoots-0.5h | AtGenExpress;/share/nasc/AtGenExpre ss_Stress_Treatments_UV-B_stress/CEL/7111.CEL |
| UV-Bstress-Roots-0.5h | AtGenExpress;/share/nasc/AtGenExpre ss_Stress_Treatments_UV-B_stress/CEL/7121.CEL |
| UV-Bstress-Shoots-1.0h | AtGenExpress;/share/nasc/AtGenExpre ss_Stress_Treatments_UV-B_stress/CEL/7211.CEL |
| UV-Bstress-Roots-1.0h | AtGenExpress;/share/nasc/AtGenExpre ss_Stress_Treatments_UV-B_stress/CEL/7221.CEL |
| UV-Bstress-Shoots-3.0h | AtGenExpress;/share/nasc/AtGenExpre ss_Stress_Treatments_UV-B_stress/CEL/7311.CEL |
| UV-Bstress-Roots-3.0h | AtGenExpress;/share/nasc/AtGenExpre ss_Stress_Treatments_UV-B_stress/CEL/7321.CEL |
| UV-Bstress-Shoots-6.0h | AtGenExpress;/share/nasc/AtGenExpre ss_Stress_Treatments_UV-B_stress/CEL/7411.CEL |
| UV-Bstress-Roots-6.0h | AtGenExpress;/share/nasc/AtGenExpre ss_Stress_Treatments_UV-B_stress/CEL/7421.CEL |
| UV-Bstress-Shoots-12.0h | AtGenExpress;/share/nasc/AtGenExpress_Stress_Treatments_UV-B_stress/CEL/7511.CEL |
| UV-Bstress-Roots-12.0h | AtGenExpress;/share/nasc/AtGenExpre ss_Stress_Treatments_UV-B_stress/CEL/7521.CEL |
| UV-Bstress-Shoots-24.0h | AtGenExpress;/share/nasc/AtGenExpre ss_Stress_Treatments_UV-B_stress/CEU7611.CEL |
| UV-Bstress-Roots-24.0h | AtGenExpress;/share/nasc/AtGenExpre ss_Stress_Treatments_UV-B_stress/CEL/7621.CEL |
| UV-Bstress-Shoots-0.25h | AtGenExpress;/share/nasc/AtGenExpre ss_Stress_Treatments_UV-B_stress/CEL/7711.CEL |
| UV-Bstress-Roots-0.25h | AtGenExpress;/share/nasc/AtGenExpre ss_Stress_Treatments_UV-B_stress/CEL/7721.CEL |
| Woundingstress-Shoots-0.5h | AtGenExpress;/share/nasc/AtGenExpre ss_Stress_Treatments_Wounding_stre ss/CEL/8111.CEL |
| Woundingstress-Roots-0.5h | AtGenExpress;/share/nasc/AtGenExpre ss_Stress_Treatments_Wounding_stre ss/CEL/8124.CEL |
| Woundingstress-Shoots-1.0h | AtGenExpress;/share/nasc/AtGenExpre ss_Stress_Treatments_Wounding_stre ss/CEL/8211.CEL |
| Woundingstress-Roots-1.0h | AtGenExpress;/share/nasc/AtGenExpre ss_Stress_Treatments_Wounding_stre ss/CEL/8224.CEL |
| Woundingstress-Shoots-3.0h | AtGenExpress;/share/nasc/AtGenExpre ss_Stress_Treatments_Wounding_stre ss/CEL/8313.CEL |
| Woundingstress-Roots-3.0h | AtGenExpress;/share/nasc/AtGenExpre ss_Stress_Treatments_Wounding_stre ss/CEU8324.CEL |
| Woundingstress-Shoots-6.0h | AtGenExpress;/share/nasc/AtGenExpre ss_Stress_Treatments_Wounding_stre ss/CEL/8411.CEL |
| Woundingstress-Roots-6.0h | AtGenExpress;/share/nasc/AtGenExpre ss_Stress_Treatments_Wounding_stre ss/CEL/8423.CEL |
| Woundingstress-Shoots-12.0h | AtGenExpress;/share/nasc/AtGenExpre ss_Stress_Treatments_Wounding_stre ss/CEL/8511.CEL |
| Woundingstress-Roots-12.0h | AtGenExpress;/share/nasc/AtGenExpre ss_Stress_Treatments_Wounding_stre ss/CEU8524.CEL |
| Woundingstress-Shoots-24.0h | AtGenExpress;/share/nasc/AtGenExpre ss_Stress_Treatments_Wounding_stre ss/CEL/8611.CEL |
| Woundingstress-Roots-24.0h | AtGenExpress;/share/nasc/AtGenExpre ss_Stress_Treatments_Wounding_stre ss/CEU8621.CEL |
| Woundingstress-Shoots-0.25h | AtGenExpress;/share/nasc/AtGenExpre ss_Stress_Treatments_Wounding_stre ss/CEL/8712.CEL |
| Woundingstress-Roots-0.25h | AtGenExpress;/share/nasc/AtGenExpre ss_Stress_Treatments_Wounding_stress/CEU8723.CEL |
| Heatstress-Shoots-0.5h | AtGenExpress;/share/nasc/AtGenExpre ss_Stress_Treatments_Heat_stress/CE L/9111.CEL |
| Heatstress-Roots-0.5h | AtGenExpress;/share/nasc/AtGenExpre ss_Stress_Treatments_Heat_stress/CE L/9121.CEL |
| Heatstress-Shoots-1.0h | AtGenExpress;/share/nasc/AtGenExpre ss_Stress_Treatments_Heat_stress/CE L/9211.CEL |
| Heatstress-Roots-1.0h | AtGenExpress;/share/nasc/AtGenExpre ss_Stress_Treatments_Heat_stress/CE U9221.CEL |
| Heatstress-Shoots-3.0h | AtGenExpress;/share/nasc/AtGenExpre ss_Stress_Treatments_Heat_stress/CE U9311.CEL |
| Heatstress-Roots-3.0h | AtGenExpress;/share/nasc/AtGenExpre ss_Stress_Treatments_Heat_stress/CE U9321.CEL |
| Heatstress(3h)+3hrecovery-Shoots-6.0h | AtGenExpress;/share/nasc/AtGenExpre ss_Stress_Treatments_Heat_stress/CE L/9411.CEL |
| Heatstress(3h)+3hrecovery-Roots-6.0h | AtGenExpress;/share/nasc/AtGenExpre ss_Stress_Treatments_Heat_stress/CE U9421.CEL |
| Heatstress(3h)+9hrecovery-Shoots-12.0h | AtGenExpress;/share/nasc/AtGenExpre ss_Stress_Treatments_Heat_stress/CE U9511.CEL |
| Heatstress(3h)+9hrecovery-Roots-12.0h | AtGenExpress;/share/nasc/AtGenExpre ss_Stress_Treatments_Heat_stress/CE U9521.CEL |
| Heatstress(3h)+21 hrecovery-Shoots-24.Oh | AtGenExpress;/share/nasc/AtGenExpre ss_Stress_Treatments_Heat_stress/CE U9611.CEL |
| Heatstress(3h)+21 hrecovery-Roots-24.0h | AtGenExpress;/share/nasc/AtGenExpre ss_Stress_Treatments_Heat_stress/CE U9621.CEL |
| Heatstress-Shoots-0.25h | AtGenExpress;/share/nasc/AtGenExpre ss_Stress_Treatments_Heat_stress/CE L/9711.CEL |
| Heatstress-Roots-0.25h | AtGenExpress;/share/nasc/AtGenExpre ss_Stress_Treatments_Heat_stress/CE L/9721.CEL |
| Heatstress(3h)+1 hrecovery-Shoots-4.0h | AtGenExpress;/share/nasc/AtGenExpre ss_Stress_Treatments_Heat_stress/CE L/9811.CEL |
| Heatstress(3h)+1 hrecovery-Roots-4.0h | AtGenExpress;/share/nasc/AtGenExpre ss_Stress_Treatments_Heat_stress/CE U9821.CEL |
| lateral root initiation study in xylem pole pericycle cells ;ivsme_Col-0_NPA | in-house experiment |
| lateral root initiation study in xylem pole pericycle cells ;ivsme_Col-0_2hNAA | in-house experiment |
| lateral root initiation study in xylem pole pericycle cells ;ivsme_Col-0_6hNAA | in-house experiment |
| overexpression of two prohibitins in seedlings;olake_AtPHB3_overexp | in-house experiment |
| overexpression of two prohibitins in seedlings;olake_AtPHB4_overexp | in-house experiment |
| overexpression of two prohibitins in seedlings;olake_overexp_control | in-house experiment |
| comparison of 3 mutants (2 prohibitin, 1 auxin glucosyltransferase) to wild-type in seedlings;olake_knockout AtPHB3 | in-house experiment |
| comparison of 3 mutants (2 prohibitin, 1 auxin glucosyltransferase) to wild-type in seedlings;olake_knockout_AtPHB4 | in-house experiment |
| comparison of 3 mutants (2 prohibitin, 1 auxin glucosyltransferase) to wild-type in seedlings;olake_control | in-house experiment |
| comparison of 3 mutants (2 prohibitin, 1 auxin glucosyltransferase) to wild-type in seedlings;olake_overexpression_UGTx | in-house experiment |
| WT landsberg erecta shoot apex seedlings compared to narrow leaf mutants elongata2, 3, 4, angusta4, drl1-2;defle_Ler | in-house experiment |
| WT landsberg erecta shoot apex seedlings compared to narrow leaf mutants elongata2, 3, 4, angusta4, drl1-2;defle-elo2 | in-house experiment |
| WT landsberg erecta shoot apex seedlings compared to narrow leaf mutants elongata2, 3, 4, angusta4, drl1-2;defle_drI1-2 | in-house experiment |
| WT landsberg erecta shoot apex seedlings compared to narrow leaf mutants elongata2, 3, 4, angusta4, drl1-2;defle_ang4 | in-house experiment |
| WT landsberg erecta shoot apex seedlings compared to narrow leaf mutants elongata2, 3, 4, angusta4, drl1-2;defle_elo3 | in-house experiment |
| WT shoot apex seedlings compared to narrow leaf mutants ELP3 (2) and SWP1 (1);defle_Ws | in-house experiment |
| WT shoot apex seedlings compared to narrow leaf mutants ELP3 (2) and SWP1 (1);defle_N3983 | in-house experiment |
| WT shoot apex seedlings compared to narrow leaf mutants ELP3 (2) and SWP1 (1);defle 219E08 | in-house experiment |
| WT shoot apex seedlings compared to narrow leaf mutants ELP3 (2) and SWP1 (1);defle_Swp1 | in-house experiment |
| WT landsberg erecta leaves 6 compared to broad leaf mutant rotunda 2;defle-ron2 | in-house experiment |
| WT landsberg erecta leaves 6 compared to broad leaf mutant rotunda 2;defle_Ler | in-house experiment |
| WT landsberg erecta compared to broad leaf mutant rotunda 1;defle_Ler | in-house experiment |
| WT landsberg erecta compared to broad leaf mutant rotunda 1;defle_ron1 | in-house experiment |
| WT leaves profiling at different stages of development (9, 15, 22 days) ;gebee_15 | in-house experiment |
| WT leaves profiling at different stages of development (9, 15, 22 days) ;gebee_22 | in-house experiment |
| WT leaves profiling at different stages of development (9, 15, 22 days) ;gebee_9 | in-house experiment |
| Methyl jasmonate profiling at different time points ;lapau_ATL- MeJA-0.5 | in-house experiment |
| Methyl jasmonate profiling at different time points ;lapau_ATL- MeJA-2 | in-house experiment |
| Methyl jasmonate profiling at different time points ;lapau_ATL- MeJA-6 | in-house experiment |
| Methyl jasmonate profiling at different time points ;lapau_ATL- DMSO-0.5 | in-house experiment |
| Methyl jasmonate profiling at different time points ;lapau_ATL- DMSO-2 | in-house experiment |
| Methyl jasmonate profiling at different time points ;lapau_ATL- DMSO-6 | in-house experiment |
| Methyl jasmonate profiling at different time points ;lapau_ATL- MeJA-CHX-6 | in-house experiment |
| DEL1 knockouts versus control (columbia) in seedlings; livey_Wild-type_seedling_8d | in-house experiment |
| DEL1 knockouts versus control (columbia) in seedlings;livey_DEL1_KO_seedling_8d | in-house experiment |
| E2F overexpression versus control (columbia WT) in seedlings; livey_Wild-type_seedling_8d | in-house experiment |
| E2F overexpression versus control (columbia WT) in seedlings; livey_E2Fa-DPa_OE_seedling_8d | in-house experiment |
| DEL1 overexpression versus control (columbia WT) in leaf;livey_Wild-type_leaf 15d | in-house experiment |
| DEL1 overexpression versus control (columbia WT) in leaf;livey_DEL1_OE_leaf_15d | in-house experiment |
| endocycle exp. In leaf - control;livey_Wild-type_leaf-15d | in-house experiment |
| endocycle exp. In leaf - DEL1 knockout;livey_DEL1_KO_leaf 15d | in-house experiment |
| endocycle exp. In leaf - CCS52A1 knockout;livey_CCS52A1_KO_leaf_15d | in-house experiment |
| endocycle exp. In leaf - CCS52B knockout;livey_CCSS2B_KO_leaf_15d | in-house experiment |
| endocycle exp. In leaf - CCKB1 DN;livey_CDKB1;1.DN_#9_15d | in-house experiment |
| endocycle exp. In leaf- KRP2 overexpression;livey_KRP2_OE_#7_15d | in-house experiment |
| endocycle exp. In leaf - DEL1 overexpression;livey_DEL1_OE_#2_15d | in-house experiment |
| WEE1 exp. - genotype columbia;livey_ROOT_TIP+0h | in-house experiment |
| WEE1 exp. - genotype columbia;livey_ROOT_TIP+0h_HU | in-house experiment |
| WEE1 exp. - genotype columbia;livey_ROOT_TIP+5h | in-house experiment |
| WEE1 exp. - genotype columbia;livey_ROOT-TIP+5h-HU | in-house experiment |
| WEE1 exp. - genotype columbia;livey_ROOT_TIP+1day | in-house experiment |
| WEE1 exp. - genotype columbia;livey_ROOT_TIP+1day_HU | in-house experiment |
| WEE1 exp. - genotype WEE1;livey_ROOT_TIP+0h | in-house experiment |
| WEE1 exp. - genotype WEE1;livey_ROOT_TIP+0h_HU | in-house experiment |
| WEE1 exp. - genotype WEE1;livey_ROOT_TIP+5h | in-house experiment |
| WEE1 exp. - genotype WEE1;livey_ROOT_TIP+5h_HU | in-house experiment |
| WEE1 exp. - genotype WEE1;livey_ROOT_TIP+1day | in-house experiment |
| WEE1 exp. - genotype WEE1;livey_ROOT_TIP+1day_HU | in-house experiment |
| lateral root initiation study WT versus mutant solitary root 1 (slr1) WT 0h | in-house experiment |
| lateral root initiation study WT versus mutant solitary root 1 (slr1) WT 2h | in-house experiment |
| lateral root initiation study WT versus mutant solitary root 1 (slr1) WT 6h | in-house experiment |
| lateral root initiation study WT versus mutant solitary root 1 (slr1) slr1 0h | in-house experiment |
| lateral root initiation study WT versus mutant solitary root 1 (slr1) slr1 2h | in-house experiment |
| lateral root initiation study WT versus mutant solitary root 1 (slr1) slr1 6h | in-house experiment |
| vascular tissue formation - callus formed from leaves - 5 days after treatment; 0.2mgl-1 auxin( 2,4-D NA IAA) 1.0mgl-1 | DB001_ATH1_A3-Brown-cal.CEL BA |
| vascular tissue formation - callus formed from leaves - 9 days after treatment; 0.2mgl-1 auxin( 2,4-D NA IAA) 1.0mgl-1 | DB001_ATH1_A4-Brown-cal.CEL BA |
| Arrested development mutant analysis - leaves - add3 | BP001_ATH1_A1-Pickett-ADD3-1.CEL |
| Arrested development mutant analysis - leaves - control | BP001_ATH1_A3-Pickett-a1.CEL |
| Agrobacterium induced tumor vs control; Tumour tissue induced at the base of an inflorescence stalk with Agrobacterium tumefaciens | RD001_ATH1_A1-deeke-tum.CEL |
| Agrobacterium induced tumor vs control; Inflorescence stalk injured at the base with a injection needle (control) | RD001_ATH1_A2-deeke-Inf.CEL |
| Polycomb binding protein knockout vs WT; WT | KC001_ATH1_A1-Cain-WT1.CEL |
| Polycomb binding protein knockout vs WT; CDB1 knockout | KC001_ATH1_A3-Cain-CDBI.CEL |
| axillary bud dormancy_ naphthalene-1-acetic acid wild-type | E-MEXP-148 |
| axillary bud dormancy_max4-1 mutant | E-MEXP-148 |
| axillary bud dormancy_wild type | E-MEXP-148 |
| MPK4 on ethylene regulation (leaf)_witd_type_Landsberg erecta | E-MEXP-174 |
| MPK4 on ethylene regulation (leaf)_ctr1 mutant_Columbia | E-MEXP-174 |
| MPK4 on ethylene regulation (leaf)_mpk4 mutant_Landsberg erecta | E-MEXP-174 |
| MPK4 on ethylene regulation (leaf)_ctr1 mpk4 double mutant_Landsberg/Columbia | E-MEXP-174 |
| systemic responses of MAPK to pathogens (leaf)_35S:mks1_eco_Landsberg | E-MEXP-173 |
| systemic responses of MAPK to pathogens (leaf)_mpk4 mutant_eco_Landsberg | E-MEXP-173 |
| systemic responses of MAPK to pathogens (leaf)_wild type_eco_Landsberg | E-MEXP-173 |
| secondary cell wall formation_hypocotyl_eco_Landsberg | E-MEXP-265 |
| secondary cell wall formation_leaf_eco_Landsberg | E-MEXP-265 |
| secondary cell wall formation_stem base_eco_Landsberg | E-MEXP-265 |
| secondary cell wall formation_stem lower middle_eco_Landsberg | E-MEXP-265 |
| secondary cell wall formation_stem tip_eco_Landsberg | E-MEXP-265 |
| secondary cell wall formation_stem upper middle_eco_Landsberg | E-MEXP-265 |
| comparison of mutants lacking CAF-1 subunits_fas2- 1_eco_Landsberg erecta | E-MEXP-694 |
| comparison of mutants lacking CAF-1 subunits_msi1- as_eco_Columbia | E-MEXP-694 |
| comparison of mutants lacking CAF-1 subunits_wild_type_eco_Columbia | E-MEXP-694 |
| comparison of mutants lacking CAF-1 subunits_wild_type_eco_Enkheim | E-MEXP-694 |
| comparison of mutants lacking CAF-1 subunits_wild_type_eco_Landsberg erecta | E-MEXP-694 |
| AP2/ERF TF (bolita) (leaf)_bolita mutant | E-MEXP-809 |
| AP2/ERF TF (bolita) (leaf)_wild_type | E-MEXP-809 |
| tissue comparison_stem_greenhouse Columbia-0_genome | Somerville: Tissue Type Arrays of |
| tissue comparison_leaf_greenhouse Columbia-0_genome | Somerville: Tissue Type Arrays of |
| tissue comparison_flower_growth chamber | Somerville: Tissue Type Arrays of |
| Columbia-0_genome | |
| tissue comparison_flower_greenhouse Columbia-0_genome | Somerville: Tissue Type Arrays of |
| tissue comparison_stem_growth chamber Columbia-0_genome | Somerville: Tissue Type Arrays of |
| flower_wild-type Columbia_0 days flower | Weigel: Floral transition and early development |
| flower_wild-type Columbia days flower | Weigel: Floral transition and early development |
| flower_wild-type Columbia_7 days flower | Weigel: Floral transition and early development |
| flower_wild-type Landsberg_0 days flower | Weigel: Floral transition and early development |
| flower_wild-type Landsberg_3 days flower | Weigel: Floral transition and early development |
| flower_wild-type Landsberg_5 days flower | Weigel: Floral transition and early development |
| flower_wild-type Landsberg_7 days flower | Weigel: Floral transition and early development |
| flower_mutant constans (co-2)_0 days flower | Weigel: Floral transition and early development |
| flower_mutant constans (co-2)_3 days flower | Weigel: Floral transition and early development |
| flower_mutant constans (co-2)_5 days flower | Weigel: Floral transition and early development |
| flower_mutant constans (co-2)_7 days flower | Weigel: Floral transition and early development |
| flower_mutantft (ft-2)_0 days flower | Weigel: Floral transition and early development |
| flower_mutant ft (ft-2)_3 days flower | Weigel: Floral transition and early development |
| flower_mutant ft (ft-2)_5 days flower | Weigel: Floral transition and early development |
| flower_mutant ft (ft-2)_7 days flower | Weigel: Floral transition and early development |
| flower_mutant Ify-12 Columbia-0 days flower | Weigel: Floral transition and early development |
| flower_mutant Ify-12 Columbia_3 days flower | Weigel: Floral transition and early development |
| flower_mutant Ify-12 Columbia_5 days flower | Weigel: Floral transition and early development |
| flower_mutant Ify-12 Columbia_7 days flower | Weigel: Floral transition and early development |
| laser-capture micro-dissected_embryo cotyledon (apical) | /share/nasc/Casson_laser-capture_micro-dissected_embryonic_tissues/CEL/KLO 01_ATH1_A1-Linds-cot.CEL |
| laser-capture micro-dissected_embryo root (basal) | /share/nasc/Casson_laser-capture_micro-dissected_embryonic_tissues/CEL/KL0 01_ATH1_A1-Linds-roo.CEL |
| laser-capture micro-dissected_globular embryo, apical tissue | /share/nasc/Casson/laser-capture_micro-dissected_embryonic_tissues/CEL/SC0 01_ATH1_A1.1-casso-gla.CEL |
| laser-capture micro-dissected_globular embryo, basal tissue | /share/nasc/Casson_laser-capture_micro-dissected_embryonic_tissues/CEL/SC0 01_ATH1_A2.1-casso-glb.CEL |
| laser-capture micro-dissected_heart stage embryo, cotyledon pole (apical) | /share/nasc/Casson_laser-capture_micro-dissected_embryonic_tissues/CEL/SC0 01_ATH 1_A3-1-casso-hec.CEL |
| laser-capture micro-dissected_heart stage embryo, root pole (basal) | /share/nasc/Casson laser-capture_micro-dissected_embryonic_tissues/CEL/SC0 01_ATH 1_A4-1-casso-her.CEL |
| water-Col0-1 hr | /share/nasc/De_Grauwe/CEL/DV001_A TH1_A2-degra-Cc1.CEL |
| water-etr1 (ethylene-insensitive mutant)-1hr | /share/nasc/De_Grauwe/CEUDV001_ A TH1_A7-degra-Ec1.CEL |
| GA4-Col0-1 hr | /share/nasc/De_Grauwe/CEL/DV001_A TH1_A4-degra-Cg1.CEL |
| GA4-etr1 (ethylene-insensitive mutant)-1hr | /share/nasc/De_Grauwe/CEL/D001_A TH1_A9-degra-Eg1.CEL |
| ms1ttg-young | /share/nasc/Gema_Vizcay_Barrena/CE L/ZW001_ATH1_A1-Wilson-mla.CEL |
| ms1ttg-old | /share/nasc/Gema_Vizcay_Barrena/CE L/ZW002_ATH1_A2_Wilson_Rep2.CEL |
| Ler-young | /share/nasc/Gema_Vizcay_Barrena/CE L/ZW002_ATH1_A3_Wilson_Rep2.CEL |
| Ler-old | /share/nasc/Gema_Vizcay_Barrena/CE L/ZW001_ATH1_A4-Wilson-Ler.CEL |
| Lerttg-young | /share/nasc/Gema_Vizcay_Barrena/CE L/ZW003_ATH1_C1-GVB_rep1.CEL |
| Lerttg-old | /share/nasc/Gema_Vizcay_Barrena/CE L/ZW003_ATH1_C2-GVB_rep1.CEL |
| Programmed Cell Death Control | /share/nasc/Jodi_Swidzinski/CEUJS00 1_ATH1_Control(3)new.CEL |
| Programmed Cell Death Heat | /share/nasc/Jodi_Swidzinski/CEL/JS00 1ATH1-Heat(4).CEL |
| Programmed Cell Death Senescence | /share/nasc/Jodi_Swidzinski/CEL/JS00 1_ATH1_Sen(3)new.CEL |
| Wheeler-a05-SLD-SPH1 RNAi at stage 1.05, leaf 1_ATH | /share/nasc/Mike Wheeler/CEUMW00 1_A1-Wheel-a05.CEL |
| Wheeler-a14_SLD_SPH1 RNAi at stage 1.14, leaf 1_ATH1_A1-Wheel-a14.CEL | /share/nasc/Mike Wheeler/CEL/MW00 |
| Wheeler-w05_SLD_wild type at stage 1.05, leaf 1_ATH | /share/nasc/Mike Wheeler/CEL/MW00 1_A1-Wheel-w05.CEL |
| Wheeler-w14-SLD-wild type at stage 1.14, leaf 1_ATH1_A1-Wheel-w14.CEL | /share/nasc/Mike Wheeler/CEL/MW00 |
| Ulm_1-1_4days-345nm B_response_of_Arabidopsis/CEL/Ulm_ | /share/nasc/Ulm_UV-1-1_4days-345nm_Rep1_ATH1.CEL |
| Ulm_1-4_4days-305nm B_response_of_Arabidopsis/CEL/Ulm_ | /share/nasc/Ulm_UV-1-4_4days-305m,_Rep1_ATH1.CEL |
| Ulm_1-9_4days-345nm,1hr-305nm B_response_of Arabidopsis/CEL/Ulm_ | /share/nasc/Ulm_UV-1-9_4days-345nm, 1hr-305nm_Rep3_ATH1.CEL |
| Ulm_1-10_4days-345nm,6hr-305nm B_response_of_Arabidopsis/CEL/Ulm_ | /share/nasc/Ulm_UV-1-10_4days-345nm,6hr-305nm_Rep1_ATH1.CEL |
| microgametogenesisPollen_bicellular pollen ATH1_A2-BCP1.CEL | /share/nasc/David_Honys/CEL/DH001_ |
| microgametogenesisPollen_tricellular pollen ATH1_A3-TCP1.CEL | /share/nasc/David_Honys/CEL/DH001_ |
| microgametogenesisPollen_uninucleate microspores ATH1_A1-UNM1.CEL | /share/nasc/David_Honys/CEL/DH001_ |
| Pourtau_1-1_lowN_leaf senescence | /share/nasc/Pourtau_sugar_acc_during _early_leaf_senescence/CEL/Pourtau_ 1-1_lowN_Rep1_ATH1.CEL |
| Pourtau_1-4_lowN-glu_leaf senescence_effect of glucose | /share/nasc/Pourtau_sugar_acc_during _early_leaf senescence/CEL/Pourtau_ 1-4_lowN-glu_Rep1_ATH1.CEL |
| Pourtau_1-7_highN-glu_leaf senescence_effect of nitrogen | /share/nasc/Pourtau_sugar_acc_during _early_leaf_senescence/CEL/Pourtau_ 1-7_highN-glu_Repl_ATH1.CEL |
| ozone-fumigated seedlings | /share/nasc/Short_Functional_Genomic s_of_Ozone_Stress_in_Arabidopsis/CE L/ES001_ATH 1_A1-mcain-ozo.CEL |
| air-fumigated seedlins | /share/nasc/Short_Functional_Genomic s_of_Ozone_Stress_in_Arabidopsis/CE L/ES001_ATH1_A2-mcain-con.CEL |

### References

- Altschul, S.F., Madden, T.L., Schäffer, A.A., Zhang, J., Zhang, Z., Miller, W. and Lipman, D.L. (1997), Gapped BLAST and PSI-BLAST: a new generation of protein database search programs, Nucleic Acids Res. 25, 3389-3402.
- Altschul, S.F., Wootton, J.C., Gertz, E.M., Agarwala, R., Morgulis, A., Schäffer, A.A. and Yu, Y.K. (2005). Protein database searches using compositionally adjusted substitution matrices, FEBS J. 272, 5101-5109.
- T. Bashir, N. V. Dorrello, V. Amador, D. Guardavaccaro, M. Pagano, Nature 428, 190 (Mar 11,2004).
- V. Brukhin, J. Gheyselinck, V. Gagliardini, P. Genschik, U. Grossniklaus, The Plant cell 17, 2723 (Oct, 2005).
- A. Camasses, A. Bogdanova, A. Shevchenko, W. Zachariae, Mol Cell 12, 87 (Jul, 2003).
- A. Capron, L. Okresz, P. Genschik, Trends in plant science 8, 83 (Feb, 2003).
- M. L. Churchman et al., The Plant cell 18, 3145 (Nov, 2006).
- Clough, S.J. and Bent, A.F. (1998) Floral dip: a simplified method for Agrobacterium-mediated transformation of Arabidopsis thaliana. Plant J 16, 735-743.
- J. Cui et al., Nucleic acids research 36, D999 (Jan, 2008).
- L. De Veylder, G. Segers, N. Glab, M. Van Montagu, D. Inze, Journal of experimental botany 48, 2113 (Dec, 1997).
- M. Erhardt et al., J Cell Sci 115, 2423 (Jun 1, 2002).
- J. Forment, M. A. Naranjo, M. Roldan, R. Serrano, O. Vicente, Plant J 30, 511 (Jun, 2002).
- J. Geisler-Lee et al., Plant Physiol 145, 317 (Oct, 2007).
- Y. Hase, K. H. Trung, T. Matsunaga, A. Tanaka, Plant J 46, 317 (Apr, 2006).
- M. J. Hayes et al., Nat Cell Biol 8, 607 (Jun, 2006).
- E. Huala et al., Nucleic acids research 29, 102 (Jan 1, 2001).
- W. Huanca-Mamani, M. Garcia-Aguilar, G. Leon-Martinez, U. Grossniklaus, J. P. Vielle-Calzada, Proc Natl Acad Sci U S A 102, 17231 (Nov 22, 2005).
- S. Ishida et al., Mol Cell Biol 21, 4684 (Jul, 2001).
- L. J. Jensen, T. S. Jensen, U. de Lichtenberg, S. Brunak, P. Bork, Nature 443, 594 (Oct 5, 2006).
- S. Kerrien et al., Nucleic acids research 35, D561 (Jan, 2007).
- C. S. Kwon et al., Development 133, 3223 (Aug, 2006).
- S. Maere, K. Heymans, M. Kuiper, Bioinformatics (Oxford, England) 21, 3448 (Aug 15, 2005).
- Z. Magyar et al., The Plant cell 17, 2527 (Sep, 2005).
- M. L. Mayer, S. P. Gygi, R. Aebersold, P. Hieter, Molecular cell 7, 959 (May, 2001).
- M. Menges, L. Hennig, W. Gruissem, J. A. Murray, Plant molecular biology 53, 423 (Nov, 2003).
- M. Menges, S. M. de Jager, W. Gruissem, J. A. Murray, Plant J 41, 546 (Feb, 2005).
- S. Muller et al., Curr Biol 14, 412 (Mar 9, 2004).
- A. Peres et al., J Biol Chem 282, 25588 (Aug 31, 2007).
- S. C. Popescu et al., Proc Natl Acad Sci U S A 104, 4730 (Mar 13, 2007).
- J. S. Rohila et al., Plant J 46, 1 (Apr, 2006).
- R. W. Shultz, V. M. Tatineni, L. Hanley-Bowdoin, W. F. Thompson, Plant Physiol 144, 1697 (Aug, 2007).
- G. J. Seifert, Curr Opin Plant Biol 7, 277 (Jun, 2004).
- P. Shannon et al., Genome Res 13, 2498 (Nov, 2003).
- N. Takahashi et al., The EMBO journal 27, 1840 (Jul 9, 2008).
- N. Tsesmetzis et al., The Plant cell 20, 1426 (Jun, 2008).
- K. Vandepoele et al., The Plant cell 14, 903 (Apr, 2002).
- K. Vandepoele et al., Plant Physiol 139, 316 (Sep, 2005).
- K. Vandepoele, T. Casneuf, Y. Van de Peer, Genome Biol 7, R103 (2006).
- J. Van Leene et al., Mol Cell Proteomics 6, 1226 (Jul, 2007).
- J. Van Leene, E. Witters, D. Inze, G. De Jaeger, Trends in plant science 13, 517 (Sep 2, 2008).
- I. Vastrik et al., Genome biology 8, R39 (2007).
- H. C. Vodermaier, C. Gieffers, S. Maurer-Stroh, F. Eisenhaber, J. M. Peters, Curr Biol 13, 1459 (Sep 2, 2003).
- J. Wuarin, V. Buck, P. Nurse, J. B. Millar, Cell 111, 419 (Nov 1, 2002).

## Claims

1. The use of At3g17020 (SEQ ID N° 2) or a variant thereof with at least 50% identities at protein level, to promote plant growth.

2. The use of a cell cycle related protein according to claim1, whereby said use is overexpression.

3. The use according to claim 1 or 2, whereby plant promotion of plant growth is measured as plant biomass increase.

4. The use, according to any of the claims 1-3, whereby said plant is a crop plant.

5. The use according to claim 4, whereby said crop plant is a cereal.

6. The use according to claim 5, whereby said cereal is selected from the group consisting of rice, maize, wheat, barley, millet, rye, sorghum and oats.


SEQUENCE LISTING

<110> VIB VZW
      UNIVERSITEIT GENT
      UNIVERSITEIT ANTWERPEN

<120> METHOD FOR SCREENING GENES INVOLVED IN PLANT CELL CYCLE

<130> GDJ/INT/V305

<150> EP 08171187.1
<151> 2008-12-10

<160> 5

<170> PatentIn version 3.5

<210> 1
<211> 432
<212> PRT
<213> Arabidopsis thaliana

<400> 1

Met Ala Met Tyr Val Ile Tyr Glu Ser Ser Ser Gly Tyr Gly Leu Phe
1               5                   10                  15

Glu Val His Gly Leu Asp Glu Ile Gly Gln Asn Thr Glu Ala Val Arg
            20                  25                  30

Thr Ser Val Ser Asp Leu Ser Arg Phe Gly Arg Val Val Gln Leu Thr
        35                  40                  45

Ala Phe His Pro Phe Glu Ser Ala Leu Asp Ala Leu Asn Gln Val Asn
    50                  55                  60

Ala Val Ser Glu Gly Val Met Thr Asp Glu Leu Arg Ser Phe Leu Glu
65                  70                  75                  80

Leu Asn Leu Pro Lys Val Lys Glu Gly Lys Lys Pro Lys Phe Ser Leu
                85                  90                  95

Gly Leu Ala Glu Pro Lys Leu Gly Ser His Ile Phe Glu Ala Thr Lys
            100                 105                 110

Ile Pro Cys Gln Ser Asn Glu Phe Val Leu Glu Leu Leu Arg Val Val
        115                 120                 125

Arg Gln His Phe Asp Arg Phe Ile Lys Asp Leu Lys Pro Gly Asp Leu
    130                 135                 140

Glu Lys Ser Gln Leu Gly Leu Ala His Ser Tyr Ser Arg Ala Lys Val

145                 150                 155                 160

Lys Phe Asn Val Asn Arg Val Asp Asn Met Val Ile Gln Ala Ile Phe
                165                 170                 175

Met Leu Asp Thr Leu Asp Lys Asp Ile Asn Ser Phe Ala Met Arg Val
            180                 185                 190

Arg Glu Trp Tyr Ser Trp His Phe Pro Glu Leu Val Lys Ile Val Asn
        195                 200                 205

Asp Asn Tyr Leu Tyr Ala Arg Val Ser Lys Met Ile Asp Asp Lys Ser
    210                 215                 220

Lys Leu Thr Glu Asp His Ile Pro Met Leu Thr Glu Val Leu Gly Asp
225                 230                 235                 240

Glu Asp Lys Ala Lys Glu Val Ile Glu Ala Gly Lys Ala Ser Met Gly
                245                 250                 255

Ser Asp Leu Ser Pro Leu Asp Leu Ile Asn Val Gln Thr Phe Ala Gln
            260                 265                 270

Lys Val Met Asp Leu Ala Asp Tyr Arg Lys Lys Leu Tyr Asp Tyr Leu
        275                 280                 285

Val Thr Lys Met Ser Asp Ile Ala Pro Asn Leu Ala Ala Leu Ile Gly
    290                 295                 300

Glu Met Val Gly Ala Arg Leu Ile Ser His Ala Gly Ser Leu Thr Asn
305                 310                 315                 320

Leu Ala Lys Cys Pro Ser Ser Thr Leu Gln Ile Leu Gly Ala Glu Lys
                325                 330                 335

Ala Leu Phe Arg Ala Leu Lys Thr Arg Gly Asn Thr Pro Lys Tyr Gly
            340                 345                 350

Leu Ile Phe His Ser Ser Phe Ile Gly Arg Ala Ser Ala Lys Asn Lys
        355                 360                 365

Gly Arg Ile Ala Arg Tyr Leu Ala Asn Lys Cys Ser Ile Ala Ser Arg
    370                 375                 380

Ile Asp Cys Phe Ala Asp Gly Ala Thr Thr Ala Phe Gly Glu Lys Leu
385                 390                 395                 400

Arg Glu Gln Val Glu Glu Arg Leu Glu Phe Tyr Asp Lys Gly Val Ala
                405                 410                 415

Pro Arg Lys Asn Val Asp Val Met Lys Glu Val Ile Glu Asn Leu Lys
            420                 425                 430

<210>  2
<211>  163
<212>  PRT
<213>  Arabidopsis thaliana

<400>  2

Met Ala Glu Ser Gly Gly Arg Arg Ile Gly Val Ala Val Asp Phe Ser
1               5                   10                  15

Asp Cys Ser Lys Lys Ala Leu Ser Trp Ala Ile Asp Asn Val Val Arg
            20                  25                  30

Asp Gly Asp His Leu Ile Leu Ile Thr Ile Ala His Asp Met Asn Tyr
        35                  40                  45

Glu Glu Gly Glu Met Gln Leu Trp Glu Thr Val Gly Ser Pro Phe Ile
    50                  55                  60

Pro Met Ser Glu Phe Ser Asp Ala Ala Val Met Lys Lys Tyr Ala Leu
65                  70                  75                  80

Lys Pro Asp Ala Glu Thr Leu Asp Ile Val Asn Thr Ala Ala Arg Lys
                85                  90                  95

Lys Thr Ile Thr Val Val Met Lys Ile Tyr Trp Gly Asp Pro Arg Glu
            100                 105                 110

Lys Ile Cys Ala Ala Ala Glu Gln Ile Pro Leu Ser Ser Leu Val Met
        115                 120                 125

Gly Asn Arg Gly Leu Gly Gly Leu Lys Arg Met Ile Met Gly Ser Val
    130                 135                 140

Ser Asn His Val Val Asn Asn Val Ala Cys Pro Val Thr Val Val Lys
145                 150                 155                 160

Ala His Ile

<210>  3
<211>  387
<212>  PRT
<213>  Arabidopsis thaliana

<400>  3

Met Glu Ala Leu Phe Thr Ser Thr His Thr Pro Asn Leu Gln Thr Lys
1               5                   10                  15

Pro Leu Leu Lys Ser Pro Leu Pro Thr Ser Ser Gln Ser Ser Cys Trp
            20                  25                  30

Phe Cys Asn Ser Leu Pro Lys Thr Gln Phe Pro Lys Leu Arg Leu Ser
        35                  40                  45

Asn Gly Ser Ser His Gly Leu Arg Ile Gln Ala Leu Leu Arg Asn Glu
    50                  55                  60

Thr Pro Ser Glu Gly Glu Asp Asn Asn Gly Phe Gly Phe Phe Pro Gly
65                  70                  75                  80

Asp Ile Phe Ser Leu Ser Gln Glu Lys Leu Gly Ser Asn Ser Asn Gly
                85                  90                  95

Glu Thr Ser His Ser Val Ile Asp Val Glu Ala Ser Leu Ala His Pro
            100                 105                 110

Gln Gly Gly Gly Gly Asn Arg Ala Gly Leu Phe Arg Thr Pro Ile Ser
        115                 120                 125

Gly Gly Val Gln Asn Ala Thr Ser Ala His Ala Leu Pro Arg Pro Ala
    130                 135                 140

Leu Ala Val Arg Asn Leu Leu Glu Gln Ala Arg Phe Ala His Leu Cys
145                 150                 155                 160

Thr Val Met Ser Lys Met His His Arg Arg Glu Gly Tyr Pro Phe Gly
                165                 170                 175

Ser Leu Val Asp Phe Ala Pro Asp Arg Met Gly His Pro Ile Phe Leu
            180                 185                 190

Phe Ser Pro Leu Ala Ile His Thr Arg Asn Leu Leu Asn Glu Pro Arg
        195                 200                 205

Cys Ser Leu Val Val Gln Ile Pro Gly Trp Ser Gly Leu Ser Asn Ala
    210                 215                 220

Arg Val Thr Leu Phe Gly Asp Val Tyr Pro Leu Ser Glu Asp Glu Gln
225                 230                 235                 240

Glu Trp Ala His Lys Gln Tyr Ile Ala Lys His Pro His Gly Pro Ser
                245                 250                 255

Glu Gln Trp Gly Asn Phe His Tyr Phe Arg Met Gln Asn Ile Ser Asp
            260                 265                 270

Ile Tyr Phe Ile Gly Gly Phe Gly Thr Val Ala Trp Val Asp Val Lys
        275                 280                 285

Glu Tyr Glu Gly Leu Gln Pro Asp Lys Ile Ala Val Asp Gly Gly Glu
    290                 295                 300

Arg Asn Leu Lys Glu Leu Asn Ala Ile Phe Ser Lys Pro Leu Arg Glu
305                 310                 315                 320

Leu Leu Ser Thr Glu Ser Glu Val Asp Asp Ala Ala Leu Ile Ser Ile
                325                 330                 335

Asp Ser Lys Gly Ile Asp Val Arg Val Arg Gln Gly Ala Gln Phe Asn
            340                 345                 350

Ile Gln Arg Leu Ala Phe Glu Glu Gly His Gly Val Glu Thr Leu Glu
        355                 360                 365

Glu Ala Lys Ser Ala Leu Trp Lys Val Leu Glu Lys Val Lys Leu Asn
    370                 375                 380

Leu Gln Lys
385

<210>  4
<211>  186
<212>  PRT
<213>  Arabidopsis thaliana

<400>  4

Met Arg Ser Leu Tyr Pro Pro Arg Pro Thr Asn Lys Asn Ile Leu Lys
1               5                   10                  15

Asn Gly Gly Phe Glu Glu Gly Pro Leu Val Leu Pro Gly Ser Thr Thr
            20                  25                  30

Gly Val Leu Ile Pro Pro Phe Ile Glu Asp Asp His Ser Pro Leu Pro
        35                  40                  45

Gly Trp Met Val Glu Ser Leu Lys Ala Val Lys Tyr Val Asp Val Glu
    50                  55                  60

His Phe Ser Val Pro Gln Gly Arg Arg Ala Ile Glu Leu Val Ala Gly
65                  70                  75                  80

Lys Glu Ser Ala Ile Ala Gln Val Val Arg Thr Val Ile Gly Lys Thr
                85                  90                  95

Tyr Val Leu Ser Phe Ala Val Gly Asp Ala Asn Asn Ala Cys Lys Gly
            100                 105                 110

Ser Met Val Val Glu Ala Phe Ala Gly Lys Asp Thr Leu Lys Val Pro
        115                 120                 125

Tyr Glu Ser Lys Gly Thr Gly Gly Phe Lys Arg Ala Ser Ile Arg Phe
    130                 135                 140

Val Ala Val Ser Thr Arg Ser Arg Ile Met Phe Tyr Ser Thr Phe Tyr
145                 150                 155                 160

Ala Met Arg Ser Asp Asp Phe Ser Ser Leu Cys Gly Pro Val Ile Asp
                165                 170                 175

Asp Val Lys Leu Ile Ser Val Arg Lys Pro
            180                 185

<210>  5
<211>  237
<212>  PRT
<213>  Arabidopsis thaliana

<400>  5

Ile Ala Arg Asp Ser Val Leu Val Phe Arg Phe Ala Asp Val Gly Lys
1               5                   10                  15

Leu Pro Pro Pro Val Leu Gln Phe Val Glu Val Ser Phe Gly Tyr Thr
            20                  25                  30

Pro Asp Tyr Leu Ile Tyr Lys Asn Ile Asp Phe Gly Val Asp Leu Asp
        35                  40                  45

Ser Arg Val Ala Leu Val Gly Pro Asn Gly Ala Gly Lys Ser Thr Leu
    50                  55                  60

Leu Lys Leu Met Thr Gly Glu Leu His Pro Thr Glu Gly Met Val Arg
65                  70                  75                  80

Arg His Asn His Leu Lys Ile Ala Gln Tyr His Gln His Leu Ala Glu
                85                  90                  95

Lys Leu Asp Leu Glu Leu Pro Ala Leu Leu Tyr Met Met Arg Glu Phe
            100                 105                 110

Pro Gly Thr Glu Glu Glu Lys Met Arg Ala Ala Ile Gly Arg Phe Gly
        115                 120                 125

Leu Thr Gly Lys Ala Gln Val Met Pro Met Lys Asn Leu Ser Asp Gly
    130                 135                 140

Gln Arg Ser Arg Val Ile Phe Ala Trp Leu Ala Tyr Lys Gln Pro Asn
145                 150                 155                 160

Met Leu Leu Leu Asp Glu Pro Thr Asn His Leu Asp Ile Glu Thr Ile
                165                 170                 175

Asp Ser Leu Ala Glu Ala Leu Asn Glu Trp Asp Gly Gly Leu Val Leu
            180                 185                 190

Val Ser His Asp Phe Arg Leu Ile Asn Gln Val Ala His Glu Ile Trp
        195                 200                 205

Val Cys Glu Lys Gln Cys Ile Thr Lys Trp Asn Gly Asp Ile Met Asp
    210                 215                 220

Phe Lys Arg His Leu Lys Ala Lys Ala Gly Leu Glu Asp
225                 230                 235